# EUROPEAN PATENT SPECIFICATION

(11) **EP 1 259 520 B1**
(45) Date of publication and mention of the grant of the patent: **24.05.2006**
(21) Application number: 00986551.0
(22) Date of filing: 18.12.2000
(51) Int. Cl.: C07F 9/6561

(54) **NOVEL PURINES**
NEUE PURINE
NOUVELLES PURINES

(30) Priority: 17.12.1999 US 172510 P; 17.12.1999 US 172161 P; 16.10.2000 US 240788 P; 18.12.2000 US 740267; 18.12.2000 US 740619
(43) Date of publication of application: 27.11.2002
(73) Proprietor: ARIAD PHARMACEUTICALS, INC., Cambridge, MA 02139-4234 (US)
(72) Inventor: WEIGELE, Manfred, Cambridge, MA 02141 (US); SHAKESPEARE, William, Southborough, MA 01772 (US); SAWYER, Tomi, K., Southborough, MA 01772 (US); SUNDARAMOORTHI, Rajeswari, Watertown, MA 02472 (US); BOHACEK, Regine, Boston, MA 02116 (US); WANG, Yihan, Newton , MA 02460 (US); METCALF, Chester, A., III, Needham, MA 02492 (US); DALGARNO, David, C., Brookline, MA 02446 (US)
(74) Representative: Vossius & Partner
(86) International application number: PCT/US2000/034417
(87) International publication number: WO 2001/044260

(56) References cited:
- EP-A- 0 398 231
- EP-A- 0 478 292
- EP-A- 0 531 597
- EP-A- 0 832 896
- WO-A-98/11121
- WO-A-98/15563
- WO-A-98/39344
- US-A- 4 008 363
- US-A- 5 837 871
- US-A- 5 849 905
- CHARUBALA, RAMAMURTHY ET AL: "Nucleotides. XIII. Phosphorylations of adenosine and 2'-deoxyadenosine by phosphorochloridates" HETEROCYCLES (1981), 15(2), 761-76 , XP000985265
- FALETTO M.B. ET AL: 'UNIQUE INTRACELLULAR ACTIVATION OF THE POTENT ANTI-HUMAN IMMUNODEFICIENCY VIRUS AGENT 1592U89', ANTIMICROBIAL AGENTS AND CHEMOTHERAPY, AMERICAN SOCIETY FOR MICROBIOLOGY, WASHINGTON, DC, US; 1997, pp. 1099-1107 XP000886350
- ALEXANDER P. ET AL: 'SYNTHESIS OF ACYCLIC NUCLEOTIDE ANALOGUES DERIVED FROM N3-SUBSTITUTED ISOGUANINE' COLLECTION OF CZECHOSLOVAK CHEMICAL COMMUNICATIONS vol. 65, 2000, ACADEMIC PRESS, LONDON, GB, pages 1713 - 1725, XP009040608

## Description

### Priority Information

The present application claims priority under 35 U.S.C. § 119(e) to U.S. Provisional Patent Application number 60/172,510, filed December 17, 1999, entitled "Bone Targeting Agents", U.S. Provisional Patent Application number 60/172,161, filed December 17, 1999, entitled "Proton Pump Inhibitors"; and U.S. Provisional Patent Application number 60/240,788, filed October 16, 2000 entitled "Bone Targeting Agents".

The application further claims priority to U.S. National Patent Application number 09/740,267, entitled "Novel Heterocycles", and U.S. National Patent Application number 09/741,619, entitled "Proton Pump Inhibitors".

### Background of the Invention

The need to treat debilitating bone disorders, such as osteoporosis, has led to extensive research on the mechanism and regulation of continuous bone formation and resorption. In particular, an appropriate balance of osteoblasts, which function to form bone tissue, and osteoclasts, which function to resorb bone tissue, is required to maintain the structural integrity and proper functioning of the skeleton in spite of continuous metabolism. Any changes in this balance of metabolism, such as an increased bone resorption (either absolute, or an increase via decreased bone formation relative to bone resorption) can lead bone diseases or disorders. One of the most common diseases resulting from this imbalance is osteoporosis, which is characterized by a decrease in bone mass and deterioration in skeletal micro-architecture leading to an increased fragility and susceptibility to fractures. Other diseases which result from alterations in bone resorption include, but are not limited to, Paget's Disease, primary and secondary hyperparathyroidism, humoral hypercalcemia of malignancy, various cancers where resorption is increased, and rheumatoid arthritis.

Because of the serious disorders that may result from a metabolic imbalance, researchers have been interested in studying bone metabolism, and the mechanism by which bone resorption and formation occurs, to ultimately develop a strategy for inhibiting resorption, and/or for improving bone mass and/or bone micro-architecture by stimulating osteoblast activity. However, the action of both osteoclasts and osteoblasts is controlled by a number of complex factors, and thus developing selective therapeutics has proven to be a difficult task.

One approach that has been taken for the development of novel therapeutics for bone disorders is inhibition of the osteoclast proton pump. Baron and coworkers have previously demonstrated that this proton pump is a vacuolar H⁺-ATPase (see, Blair et al., *Science* **1989***, 245,* 855-857; Finbow et al., *Biochem. J.* **1997**, *324*, 697-712; Forgac, M. *Soc. Gen. Physiol. Ser.* **1996**, *51,* 121-132). It has been shown that osteoclasts, to effect bone resorption, ultimately lower the pH in the sealed microcompartment which underlies their site of attachment to the bone surface (see, Baron et al., *J. Cell. Biol.* **1985,** *101*, 2210-2222), thus resulting in the acidic envionment required to dissolve the bone mineral and to allow degradation of the bone matrix by proteases. The osteoclast uses a proton pump (an ATP-dependent transport of protons) to achieve this acidification and thus any therapeutic inhibition of the osteoclast proton pump should lead to a decrease in bone loss or turnover. As a result, many novel therapeutics developed to reduce bone resorption have focused on the inhibition of the proton pump to prevent osteoclast activity and excessive bone resorption. For a discussion of the vacuolar H⁺-ATPase and inhibitors of vacuolar H⁺-ATPase see Farina et al., *Exp. Opin. Ther. Patents* **1999**, *9*, 157-168 and David, P. and Baron, R. "The Vacuolar H⁺-ATPase: A Potential Target for Drug Development in Bone Diseases" *Exp. Opin. Invest. Drugs* **1995**, *4*, 725-740.

In addition to the inhibition of the proton pump, studies have also been directed towards the control of signal transduction to ultimately affect osteoclast or osteoblast function. For example, studies have provided evidence that Src protein kinases play a cruical role in osteoclastic function, and it has been shown in different cell types that phosphorylation by Src, and related kinases, of proteins proposed to participate or regulate the cytoskeletal architecture is one important requirement for their proper function (see, for example, Missbach et al., "A Novel Inhibitor of the Tyrosine Kinase Src Suppresses Phosphorylation of Its Major Cellular Substrates and Reduces Bone Resorption In Vitro and in Rodent Models In Vivo." *Bone* **1999**, *24*, 437-449). Because the cytoskeleton plays an important role in osteoclast motility, attachment, and formation of the sealing zone, it is likely that these cytoskeletal proteins may influence osteoclast function. Thus, agents which inhibit or promote interactions with Src or related kinases, are likely to affect cyctoskeletal proteins and ultimately affect osteoclast function. Several compounds have been reported as inhibitors of tyrosine Src kinase and thus are useful in the inhibition of osteoclast-mediated bone resorption (see, for example, Missbach et al., *Bone* **1999**, *24*, 437-449; Connolly et al., *Bioorg. & Med. Chem. Lett.* **1997**, *7*, 2415-2420; Trump-Kallmeyer et al., *J. Med. Chem.* **1998**, *41*, 1752-1763; Klutchko et al., *J. Med. Chem.* **1998**, *41*, 3276-3292; Legraverend et al, *Bioorg. & Med. Chem.* **1999**, *7*, 1281-1293; Chang et al., *Chem. & Biol.* **1999**, *6*, 361-375; Lev et al. *Nature* **1995,** *376,* 737-784; Palmer et al., *J. Med. Chem.* **1997***, 40,* 1519-1529.

Collect. Czech. Chem. Commun. 65, 11, 2000, 1713 - 1725 discloses a number of purine derivatives. Those derivatives differ from compounds of this invention, inter alia, by containing -NH2 at purine ring position 6 instead of an -NHRB group as indicated in Figure 1, where RB cannot be H.

As described above, many of the existing therapeutics that have been developed for the treatment of bone disorders such as osteoporosis are thought to act by inhibiting osteoclast activity. For example, estrogens, bisphosphonates, calcitonin, flavonoids, and selective estrogen receptor modulators are believed to act by the inhibition of osteoclast activity. Additionally, more recently, novel therapeutics have been developed to promote a fast increase in bone mineral content by promoting osteoblast activity. Such examples include peptides from the parathyroid hormone family, strontium ranelate, and growth hormone and insulin-like growth response (see, for example, Reginster et al. "Promising New Agents in Osteoporosis," *Drugs R & D* **1999**, *3*, 195-201). Unfortunately, a significant problem of many of these therapetic agents, however, is that they are not specific enough for bone tissue and thus may lead to unwanted adverse side effects.

Clearly, as evidenced by the number of different approaches to the available therapeutic agents, bone metabolism is controlled by a variety of factors. A common theme, however, is the desire to develop selective inhibitors or promoters of osteoclas or osteoblast activity, respectively. Although progress has been made towards developing therapeutic agents for osteoporosis and other bone disorders, there remains a need to develop potent and selective agents having minimal side effects.
Furthermore, there remains a need to develop compounds that can regulate signal transduction pathways (e.g., inhibit or promote interactions between protein kinases and their receptors). Such compounds may then be used to inhibit or promote complex biological processes in order to treat and/or prevent diseases associated with signaling.

### Summary of the Invention

Novel purines and uses of these compounds for the treatment of bone related disorders and cancer are disclosed. For example, the invention provides compounds, and compositions containing those compound, having the general formula: 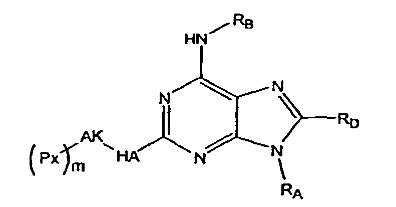 wherein R_{A} is hydrogen, an aliphatic, heteroaliphatic, aryl, heteroaryl, alkylaryl, or alkylheteroaryl moiety; R_{B} is an aliphatic, heteroaliphatic, aryl, heteroaryl, alkylaryl, or alkylheteroaryl moiety; and R_{D} is hydrogen, halogen, an aliphatic, heteroaliphatic, aryl, heteroaryl, alkylaryl, or alkylheteroaryl moiety, or -ZR_{E}, wherein Z is -O-, -S-, or NR_{F}, wherein R_{E} is hydrogen, or an aliphatic, heteroaliphatic, aryl, heteroaryl, alkylaryl, or alkylheteroaryl moiety, and R_{F} is an aliphatic, heteroaliphatic, aryl, heteroaryl, alkylaryl, or alkylheteroaryl moiety,
wherein in each of the foregoing groups each aliphatic, heteroaliphatic, alkylaryl, or alkylheteroaryl moiety may be branched or unbranched, cyclic or acyclic and substituted or unsubstituted, and each aryl and heteroaryl moiety may be substituted or unsubstituted;
wherein AK is a linear or branched, cyclic or acyclic, substituted or unsubstituted aliphatic or heteoraliphatic moiety; and wherein HA is absent, -O-, -S- or -NH-;
wherein Pₓ is a phosphorus containing moiety having the structure -P(X)YR_{G}YR_{H}, wherein X is independently an alkyl moiety, =O or =S; R_{G} and R_{H}, for each occurrence, are independently hydrogen, or substituted or unsubstituted aliphatic, heteroaliphatic, aryl, heteroaryl, alkylaryl, or alkylheteroaryl, and each occurrence of Y is independently a covalent bond, -O-, -S- or N(R_{J})₂, wherein R_{J}, for each occurrence, is independently hydrogen, aliphatic, heteroaliphatic, aryl, heteroaryl, alkylaryl, or alkylheteroaryl;
or is a phosphorus moiety having any one of structures i-viii: 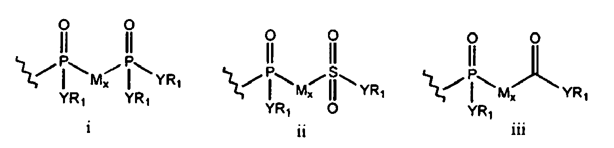 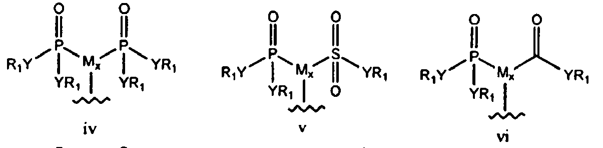 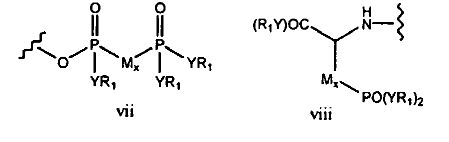 wherein each occurrence of M is independently CH₂, CHV, CHOH, or CV₂; each occurrence of Y is independently a covalent bond, -O-, -S- or N(R_{J})₂, wherein R_{J}, for each occurrence, is independently hydrogen, aliphatic, heteroaliphatic, aryl, heteroaryl, alkylaryl, or alkylheteroaryl; wherein V is a halogen; each occurrence of x is independently 1-6, and in certain embodiments is 1 or 2; and each occurrence of R₁ is independently hydrogen, aliphatic, heteroaliphatic, aryl, heteroaryl, alkylaryl, alkylheteroaryl, a prodrug or pharmaceutically acceptable derivative; and
wherein m is 1-3.

The invention further provides methods of making and using the described compounds, for example, in the inhibition of kinase activity, particularly the inhibition of src kinase activity; the treatment of bone-related disorders, including the inhibition of osteoclast activity; the inhibition of tumor cell growth; and the treatment of cancer.

### Definitions

As discussed above, the present invention provides a novel class of compounds useful for the treatment of bone related disorders, cancer, and regulation of signal transduction pathways generally. For example, certain compounds of the invention are useful as kinase inhibitors, and in one embodiment are useful as Src kinase inhibitors and thus are useful in preventing or treating a proliferative disease, cancer and osteoporosis to name a few.

Compounds of this invention include those specifically set forth above and described herein, and are illustrated in part by the various classes, subgenera and species disclosed elsewhere herein.

It will be appreciated by one of ordinary skill in the art that asymmetric centers may exist in the compounds of the present invention. Thus, inventive compounds and pharmaceutical compositions thereof may be in the form of an individual enantiomer, diastereomer or geometric isomer, or may be in the form of a mixture of stereoisomers. In certain embodiments, the compounds of the invention are enantiopure compounds. In certain other embodiments, a mixtures of stereoisomers or diastereomers are provided.

Additionally, the present invention provides pharmaceutically acceptable derivatives of the inventive compounds, and methods of treating a subject using these compounds, pharmaceutical compositions thereof, or either of these in combination with one or more additional therapeutic agents. The phrase, "pharmaceutically acceptable derivative", as used herein, denotes any pharmaceutically acceptable salt, ester, or salt of such ester, of such compound, or any other adduct or derivative which, upon administration to a patient, is capable of providing (directly or indirectly) a compound as otherwise described herein, or a metabolite or residue thereof. Pharmaceutically acceptable derivatives thus include among others pro-drugs. A prodrug is a derivative of a compound, usually with significantly reduced pharmacological activity, which contains an additional moiety which is susceptible to removal *in vivo* yielding the parent molecule as the pharmacologically active species. An example of a pro-drug is an ester which is cleaved in vivo to yield a compound of interest. Pro-drugs of a variety of compounds, and materials and methods for derivatizing the parent compounds to create the pro-drugs, are known and may be adapted to the present invention. Certain exemplary pharmaceutical compositions and pharmaceutically acceptable derivatives will be discussed in more detail herein below.

Certain compounds of the present invention, and definitions of specific functional groups are also described in more detail below. For purposes of this invention, the chemical elements are identified in accordance with the Periodic Table of the Elements, CAS version, Handbook of Chemistry and Physics, 75^{th} Ed., inside cover, and specific functional groups are generally defined as described therein. Additionally, general principles of organic chemistry, as well as specific functional moieties and reactivity, are described in "Organic Chemistry", Thomas Sorrell, University Science Books, Sausalito: 1999, the entire contents of which are incorporated herein by reference. Furthermore, it will be appreciated by one of ordinary skill in the art that the synthetic methods, as described herein, utilize a variety of protecting groups. By the term "protecting group", has used herein, it is meant that a particular functional moiety, e.g., O, S, or N, is temporarily blocked so that a reaction can be carried out selectively at another reactive site in a multifunctional compound. In preferred embodiments, a protecting group reacts selectively in good yield to give a protected substrate that is stable to the projected reactions; the protecting group must be selectively removed in good yield by readily available, preferably nontoxic reagents that do not attack the other funcational groups; the protecting group forms an easily separable derivative (more preferably without the generation of new stereogenic centers); and the protecting group has a minimum of additional functionality to avoid further sites of reaction. As detailed herein, oxygen, sulfur, nitrogen and carbon protecting groups may be utilized. Exemplary protecting groups are detailed herein, however, it will be appreciated that the present invention is not intended to be limited to these protecting groups; rather, a variety of additional equivalent protecting groups can be readily identified using the above criteria and utilized in the method of the present invention. Additionally, a variety of protecting groups are described in "Protective Groups in Organic Synthesis" Third Ed. Greene, T.W. and Wuts, P.G., Eds., John Wiley & Sons, New York: 1999, the entire contents of which are hereby incorporated by reference.

It will be appreciated that the compounds, as described herein, may be substituted with any number of substituents or functional moieties. In general, the term "substituted" whether preceded by the term "optionally" or not, and substituents contained in formulas of this invention, refer to the replacement of hydrogen radicals in a given structure with the radical of a specified substituent. When more than one position in any given structure may be substituted with more than one substituent selected from a specified group, the substituent may be either the same or different at every position. As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. For purposes of this invention, heteroatoms such as nitrogen may have hydrogen substituents and/or any permissible substituents of organic compounds described herein which satisfy the valencies of the heteroatoms. Furthermore, this invention is not intended to be limited in any manner by the permissible substituents of organic compounds. Combinations of substituents and variables envisioned by this invention are preferably those that result in the formation of stable compounds useful in the treatment, for example of bone related disorders, cancer and/or disorders related to call signalling. The term "stable", as used herein, preferably refers to compounds which possess stability sufficient to allow manufacture and which maintain the integrity of the compound for a sufficient period of time to be useful for the purposes detailed herein.

The term "aliphatic", as used herein, includes both saturated and unsaturated, straight chain (i.e., unbranched), branched, cyclic, or polycyclic aliphatic hydrocarbons, which are optionally substituted with one or more functional groups. As will be appreciated by one of ordinary skill in the art, "aliphatic" is intended herein to include, but is not limited to, alkyl, alkenyl, alkynyl, cycloalkyl, cycloalkenyl, and cycloalkynyl moieties. Thus, as used herein, the term "alkyl" includes both straight, branched and cyclic alkyl groups. An analogous convention applies to other generic terms such as "alkenyl", "alkynyl" and the like. Furthermore, as used herein, the terms "alkyl", "alkenyl", "alkynyl" and the like encompass both substituted and unsubstituted groups.

Unless otherwise specified, alkyl and other aliphatic groups preferably contain 1-6, or 1-3, contiguous aliphatic carbon atoms. Illustrative aliphatic groups thus include, but are not limited to, for example, methyl, ethyl, n-propyl, isopropyl, cyclopropyl, -CH₂-cyclopropyl, allyl, n-butyl, sec-butyl, isobutyl, tert-butyl, cyclobutyl, -CH₂-cyclobutyl, n-pentyl, sec-pentyl, isopentyl, tert-pentyl, cyclopentyl, -CH₂-cyclopentyl, n-hexyl, sec-hexyl, cyclohexyl, -CH₂-cyclohexyl moieties and the like, which again, may bear one or more substituents. Alkenyl groups include, but are not limited to, for example, ethenyl, propenyl, butenyl, 1-methyl-2-buten-1-yl, and the like. Representative alkynyl groups include, but are not limited to, ethynyl, 2-propynyl (propargyl), 1-propynyl and the like.

The term "alkoxy", or "thioalkyl" as used herein refers to an alkyl group, as previously defined, attached to the parent molecular moiety through an oxygen atom or through a sulfur atom. Examples of alkoxy, include but are not limited to, methoxy, ethoxy, propoxy, isopropoxy, n-butoxy, tert-butoxy, neopentoxy and n-hexoxy. Examples of thioalkyl include, but are not limited to methylthio, ethylthio, propylthio, isopropylthio, n-butylthio, and the like.

The term "alkylamino" refers to a group having the structure -NHR' wherein R' is alkyl, as defined herein. Examples of alkylamino include, but are not limited to, methylamino, ethylamino, iso-propylamino and the like. In certain embodiments, C₁₋C₃ alkylamino groups are utilized in the present invention.

Some examples of substituents of the above-described aliphatic (and other) moieties of compounds of the invention include, but are not limited to: F, Cl, Br, I, OH, NO₂, CN, C(O)-alkyl, C(O)-aryl, C(O)-heteroaryl, CO₂-alkyl, CO₂-aryl, CO₂₋heteroaryl, CONH₂, CONH-alkyl, CONH-aryl, CONH-heteroaryl, OC(O)-alkyl, OC(O)-aryl, OC(O)-heteroaryl, OCO₂-alkyl, OCO₂-aryl, OCO₂-heteroaryl, OCONH₂, OCONH-alkyl, OCONH-aryl, OCONH-heteroaryl, NHC(O)-alkyl, NHC(O)-aryl, NHC(O)-heteroaryl, NHCO₂-alkyl, NHCO₂-aryl, NHCONH-heteroaryl, SO₂-alkyl, SO₂-aryl, C₃-C₆-cycloalkyl, CF₃, CH₂CF₃, CHCl₂, CH₂OH, CH₂CH₂OH, CH₂NH₂, CH₂SO₂CH₃, aryl, heteroaryl, benzyl, benzyloxy, aryloxy, heteroaryloxy, alkoxy, methoxymethoxy, methoxyethoxy, amino, benzylamino, arylamino, heteroarylamino, -alkyl-amino, thio, aryl-thio, heteroarylthio, benzyl-thio, alkyl-thio, or methylthiomethyl. Additional examples of generally applicable substituents are illustrated by the specific embodiments shown in the Examples which are described herein.

In general, the terms "aryl" and "heteroaryl", as used herein, refer to stable mono- or polycyclic, heterocyclic, polycyclic, and polyheterocyclic unsaturated moieties having preferably 3-14 carbon atoms, each of which may be substituted or unsubstituted. Substituents include, but are not limited to, any of the previously mentioned substitutents, i.e., the substituents recited for aliphatic moieties, or for other moieties as disclosed herein, resulting in the formation of a stable compound. In certain embodiments of the present invention, "aryl" refers to a mono- or bicyclic carbocyclic ring system having one or two aromatic rings including, but not limited to, phenyl, naphthyl, tetrahydronaphthyl, indanyl, indenyl and the like. In certain embodiments of the present invention, the term "heteroaryl", as used herein, refers to a cyclic aromatic radical having from five to ten ring atoms of which one ring atom is selected from S, O and N; zero, one or two ring atoms are additional heteroatoms independently selected from S, O and N; and the remaining ring atoms are carbon, the radical being joined to the rest of the molecule via any of the ring atoms, such as, for example, pyridyl, pyrazinyl, pyrimidinyl, pyrrolyl, pyrazolyl, imidazolyl, thiazolyl, oxazolyl, isooxazolyl, thiadiazolyl,oxadiazolyl, thiophenyl, furanyl, quinolinyl, isoquinolinyl, and the like.

It will be appreciated that aryl and heteroaryl groups (including bicyclic aryl groups) can be unsubstituted or substituted, wherein substitution includes replacement of one, two or three of the hydrogen atoms thereon independently with any one or more of the following moieties including, but not limited to: F, Cl, Br, I, OH, NO₂, CN, C(O)-alkyl, C(O)-aryl, C(O)-heteroaryl, CO₂-alkyl, CO₂-aryl, CO₂-heteroaryl, CONH₂, CONH-alkyl, CONH-aryl, CONH-heteroaryl, OC(O)-alkyl, OC(O)-aryl, OC(O)-heteroaryl, OCO₂-alkyl, OCO₂-aryl, OCO₂-heteroaryl, OCONH₂, OCONH-alkyl, OCONH-aryl, OCONH-heteroaryl, NHC(O)-alkyl, NHC(O)-aryl, NHC(O)-heteroaryl, NHCO₂-alkyl, NHCO₂-aryl, NHCONH-heteroaryl, SO₂-alkyl, SO₂-aryl, C₃-C₆-cycloalkyl, CF₃, CH₂CF₃, CHCl₂, CH₂OH, CH₂CH₂OH, CH₂NH₂, CH₂SO₂CH₃, aryl, heteroaryl, benzyl, benzyloxy, aryloxy, heteroaryloxy, alkoxy, methoxymethoxy, methoxyethoxy, amino, benzylamino, arylamino, heteroarylamino, alkyl-amino, thio, aryl-thio, heteroarylthio, benzyl-thio, alkyl-thio, or methylthiomethyl. Additional examples of generally applicable substitutents are illustrated by the specific embodiments shown in the Examples which are described herein.

The term "cycloalkyl", as used herein, refers specifically to groups having three to seven, preferably three to ten carbon atoms. Suitable cycloalkyls include, but are not limited to cyclopropyl, cyclobutyl, cyclopentyl, cyclohexyl, cycloheptyl and the like, which, as in the case of other aliphatic, heteroaliphatic or hetercyclic moieties, may optionally be substituted. F, Cl, Br, I, OH, NO₂, CN, C(O)-alkyl, C(O)-aryl, C(O)-heteroaryl, CO₂-alkyl, CO₂-aryl, CO₂-heteroaryl, CONH₂, CONH-alkyl, CONH-aryl, CONH-heteroaryl, OC(O)-alkyl, OC(O)-aryl, OC(O)-heteroaryl, OCO₂₋alkyl, OCO₂-aryl, OCO₂-heteroaryl, OCONH₂, OCONH-alkyl, OCONH-aryl, OCONH-heteroaryl, NHC(O)-alkyl, NHC(O)-aryl, NHC(O)-heteroaryl, NHCO₂₋alkyl, NHCO₂-aryl, NHCONH-heteroaryl, SO₂-alkyl SO₂-aryl, C₃-C₆-cycloalkyl, CF₃, CH₂CF₃, CHCl₂, CH₂OH, CH₂CH₂OH, CH₂NH₂, CH₂SO₂CH₃, aryl, heteroaryl, benzyl, benzyloxy, aryloxy, heteroaryloxy, alkoxy, methoxymethoxy, methoxyethoxy, amino, benzylamino, arylamino, heteroarylamino, alkyl-amino, thio, aryl-thio, heteroarylthio, benzyl-thio, alkyl-thio, or methylthiomethyl. Additional examples of generally applicable substitutents are illustrated by the specific embodiments shown in the Examples which are described herein.

The term "heteroaliphatic", as used herein, refers to aliphatic moieties which contain one or more oxygen, sulfur, nitrogen, phosphorous or silicon atoms, e.g., in place of carbon atoms. Heteroaliphatic moieties may be branched, unbranched or cyclic and include saturated and unsaturated heterocycles such as morpholino, pyrrolidinyl, etc. In certain embodiments, heteroaliphatic moieties are substituted by independent replacement of one or more of the hydrogen atoms thereon with one or more moieties including, but not limited to: F, Cl, Br, I, OH, NO₂, CN, C(O)-alkyl, C(O)-aryl, C(O)-heteroaryl, CO₂-alkyl, CO₂-aryl, CO₂-heteroaryl, CONH₂, CONH-alkyl, CONH-aryl, CONH-heteroaryl, OC(O)-alkyl, OC(O)-aryl, OC(O)-heteroaryl, OCO₂-alkyl, OCO₂-aryl, OCO₂-heteroaryl, OCONH₂, OCONH-alkyl, OCONH-aryl, OCONH-heteroaryl, NHC(O)-alkyl, NHC(O)-aryl, NHC(O)-heteroaryl, NHCO₂₋alkyl, NHCO₂-aryl, NHCONH-heteroaryl, SO₂-alkyl, SO₂-aryl, C₃-C₆-cycloalkyl, CF₃, CH₂CF₃, CHCl₂, CH₂OH, CH₂CH₂OH, CH₂NH₂, CH₂SO₂CH₃, aryl, heteroaryl, benzyl, benzyloxy, aryloxy, heteroaryloxy, alkoxy, methoxymethoxy, methoxyethoxy, amino, benzylamino, arylamino, heteroarylamino, alkyl-amino, thio, aryl-thio, heteroarylthio, benzyl-thio, alkyl-thio, or methylthiomethyl. Additional examples of generally applicable substitutents are illustrated by the specific embodiments shown in the Examples which are described herein.

The terms "halo" and "halogen" as used herein refer to an atom selected from fluorine, chlorine, bromine and iodine.

The term "haloalkyl" denotes an alkyl group, as defined above, having one, two, or three halogen atoms attached thereto and is exemplified by such groups as chloromethyl, bromoethyl, trifluoromethyl, and the like.

The term "heterocycloalkyl" or "heterocycle", as used herein, refers to a non-aromatic 5-, 6- or 7- membered ring or a bi- or tri-cyclic group comprising fused six-membered rings having between one and three heteroatoms independently selected from oxygen, sulfur and nitrogen, wherein (i) each 5-membered ring has 0 to 1 double bonds and each 6-membered ring has 0 to 2 double bonds, (ii) the nitrogen and sulfur heteroatoms may be optionally be oxidized, (iii) the nitrogen heteroatom may optionally be quatemized, and (iv) any of the above heterocyclic rings may be fused to a benzene ring. Representative heterocycles include, but are not limited to, pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, and tetrahydrofuryl. In certain embodiments, a "substituted heterocycloalkyl or heterocycle" group is utilized and as used herein, refers to a heterocycloalkyl or heterocycle group, as defined above, substituted by the independent replacement of one, two or three of the hydrogen atoms thereon with but are not limited to: F, Cl, Br, I, OH, NO₂, CN, C(O)-alkyl, C(O)-aryl, C(O)-heteroaryl, CO₂-alkyl, CO₂-aryl, CO₂₋heteroaryl, CONH₂, CONH-alkyl, CONH-aryl, CONH-heteroaryl, OC(O)-alkyl, OC(O)-aryl, OC(O)-heteroaryl, OCO₂-alkyl, OCO₂-aryl, OCO₂-heteroaryl, OCONH₂, OCONH-alkyl, OCONH-aryl, OCONH-heteroaryl, NHC(O)-alkyl, NHC(O)-aryl, NHC(O)-heteroaryl, NHCO₂-alkyl, NHCO₂-aryl, NHCONH-heteroaryl, SO₂-alkyl, SO₂-aryl, C₃-C₆-cycloalkyl, CF₃, CH₂CF₃, CHCl₂, CH₂OH, CH₂CH₂OH, CH₂NH₂, CH₂SO₂CH₃, aryl, heteroaryl, benzyl, benzyloxy, aryloxy, heteroaryloxy, alkoxy, methoxymethoxy, methoxyethoxy, amino, benzylamino, arylamino, heteroarylamino, alkyl-amino, thio, aryl-thio, heteroarylthio, benzyl-thio, alkyl-thio, or methylthiomethyl. Additional examples of generally applicable substitutents are illustrated by the specific embodiments shown in the Examples which are described herein.

"Phosphorus containing moiety" As used herein, the phrase, "phosphorus containing moiety" pertains to phosphorous moities having the structure -P(X)YR_{G}YR_{H}, wherein X is independently an alkyl moiety, =O, =S; R_{G} and R_{H}, for each occurrence, are independently hydrogen, or substituted or unsubstituted aliphatic, heteroaliphatic, aryl, heteroaryl, alkylaryl, or alkylheteroaryl, and each occurrence of Y is independently a covalent bond, -O-, -S- or N(R_{J})₂, wherein R_{J}, for each occurrence, is independently hydrogen, aliphatic, heteroaliphatic, aryl, heteroaryl, alkylaryl, or alkylheteroaryl;
or phosphorus moieties having any one of structures i-viii: 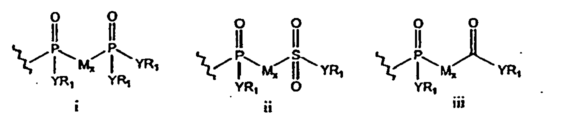 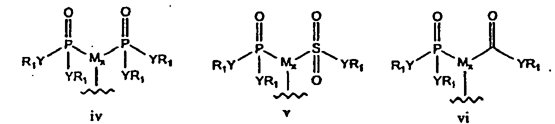 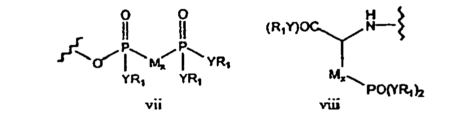 wherein each occurrence of M is independently CH₂, CHV, CHOH, or CV₂; each occurrence of Y is independently a covalent bond, -O-, -S- or N(R_{J})₂, wherein R_{J}, for each occurrence, is independently hydrogen, aliphatic, heteroaliphatic, aryl, heteroaryl, alkylaryl, or alkylheteroaryl; V is a halogen; each occurrence of x is independently 1-6, and in certain emobidment is 1-2; and each occurrence of R₁ is independently hydrogen, aliphatic, heteroaliphatic, aryl, heteroaryl, alkylaryl, alkylheteroaryl, a prodrug or pharmaceutically acceptable derivative wherein m is 1-3. It will be appreciated that the compounds of the invention can be substituted with one or more of any of the above-described moieties or any combination thereof. Furthermore, in one subset of compounds, purines are substituted with an aryl or heteroaryl moiety substituted with one or more phosphorus moieties as described above. In certain embodiments, the substituted aryl or heteroaryl moieties are selected from the following: 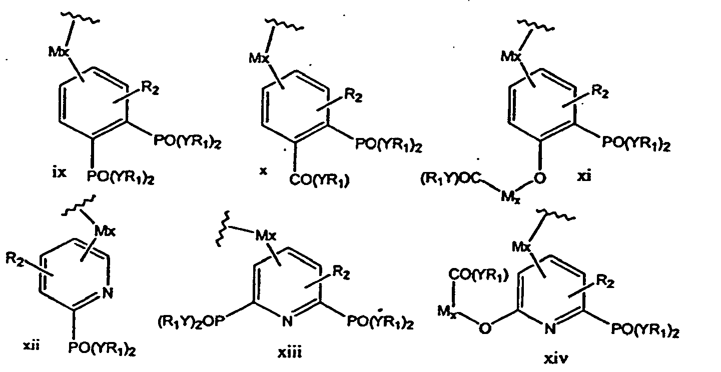 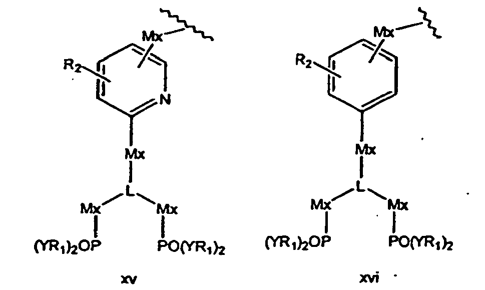 wherein each occurrence of M is independently CV₂, -NV-, -O- or -S-, wherein each occurrence of V is independently hydrogen, OH, halogen, or aliphatic; each occurrence of Y is independently a covalent bond, -O-, -S- or N(R_{J})₂, wherein R_{J}, for each occurrence, is independently hydrogen, aliphatic, heteroaliphatic, aryl, heteroaryl, alkylaryl, or alkylheteroaryl; each occurrence of x is independently 0-6; and in certain embodiment is 1-2; wherein L is CW or N, wherein W is hydrogen, aliphatic, heteroaliphatic aryl, alkylaryl, heteroaryl, alkylheteroaryl or hydroxyl; and each occurrence of R₁ is independently hydrogen, aliphatic, heteroaliphatic, aryl, heteroaryl, alkylaryl, alkylheteroaryl, a prodrug or pharmaceutically acceptable derivative; and
wherein each occurrence of R₂ independently represents from 0-3 substituents independently selected from the group consisting of halogen, lower alkyl, lower alkenyl, aryl, heteroaryl, carbonyl, thiocarbonyl, ketone, aldehyde, amino, acylamino, amido, amidino, cyano, nitro, azido, sulfonyl, sulfoxido, sulfate, sulfonate, sulfamoyl, sulfonamido, phosphoryl, phosphorothioate, phosphonate, phosphinate, -(CH₂)ₚalkyl, -(CH₂)ₚalkenyl, -(CH₂)ₚalkynyl, -(CH₂)ₚaryl, -(CH₂)ₚaralkyl, -(CH₂)ₚOH, -(CH₂)ₚO-lower alkyl, -(CH₂)ₚO-lower alkenyl, -O(CH₂)ₚR, -(CH₂)ₚSH, -(CH₂)ₚS-lower alkyl, -(CH₂)ₚS-lower alkenyl, -S(CH₂)ₚR, -(CH₂)ₚN(R)₂, -(CH₂)ₚNR-lower alkyl, -(CH₂)ₚNR-lower alkenyl, -NR(CH₂)ₚR, and protected forms of the above, wherein R represents, independently for each occurrence, hydrogen, or substituted or unsubstituted aryl, heterocycle, heteroaryl, alkylaryl, alkenyl, or alkyl, and wherein each occurrence of p independently represents an integer from 0-10.

In certain embodiments, the phosphorus moiety is defined by its structure, as described above. In certain other embodiments, a phosphorus containing moiety is a bone targeting moiety and, preferably, selectively targets bone, and thus is useful in the treatment of bone disorders.

### Detailed Description of Certain Preferred Embodiments of the Invention

As discussed in more detail herein, there remains a need for the development of novel therapeutics useful for the treatment of bone related disorders, cancer, and signalling disorders generally. There is also a particular need for the development of selective therapeutic agents (e.g. those that can selectively target bone, or can selectively inhibit or promote cellular signaling pathways.

In recognition of the need to develop more selective and potent therapeutic agents, the present invention provides novel purines having the general formula **(I)** (as well as tautomers thereof): 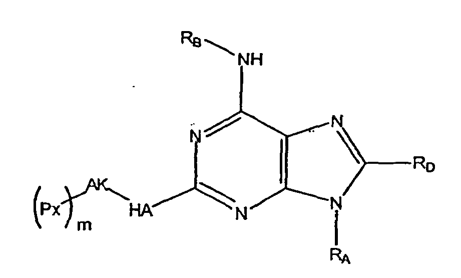 wherein R_{A} is hydrogen, an aliphatic, heteroaliphatic, aryl, heteroaryl, alkylaryl, or alkylheteroaryl moiety; R_{B} is an aliphatic, heteroaliphatic, aryl, heteroaryl, alkylaryl, or alkylheteroaryl moiety; and R_{D} is hydrogen, halogen, an aliphatic, heteroaliphatic, aryl, heteroaryl, alkylaryl, or alkylheteroaryl moiety, or -ZR_{E}, wherein Z is -O-, -S-, or NR_{F}, wherein R_{E} is hydrogen, or an aliphatic, heteroaliphatic, aryl, heteroaryl, alkylaryl, or alkylheteroaryl moiety, and R_{F} is an aliphatic, heteroaliphatic, aryl, heteroaryl, alkylaryl, or alkylheteroaryl moiety,
wherein in each of the foregoing groups each aliphatic, heteroaliphatic, alkylaryl, or alkylheteroaryl moiety may be branched or unbranched, cyclic or acyclic and substituted or unsubstituted, and each aryl and heteroaryl moiety may be substituted or unsubstituted;
wherein AK is a linear or branched, cyclic or acyclic, substituted or unsubstituted aliphatic or heteoraliphatic moiety. and wherein HA is absent, -O-, -S- or NH-;
wherein Pₓ is a phosphorus containing moiety having the structure -P(X)YR_{G}YR_{H}, wherein X is independently an alkyl moiety, =O or =S; R_{G} and R_{H}, for each occurrence, are independently hydrogen, or substituted or unsubstituted aliphatic, heteroaliphatic, aryl, heteroaryl, alkylaryl, or alkylheteroaryl, and each occurrence of Y is independently a covalent bond, -O-, -S- or N(R_{J})₂, wherein R_{J}, for each occurrence, is independently hydrogen, aliphatic, heteroaliphatic, aryl, heteroaryl, alkylaryl, or alkylheteroaryl;
or is a phosphorus moiety having any one of structures i-viii: 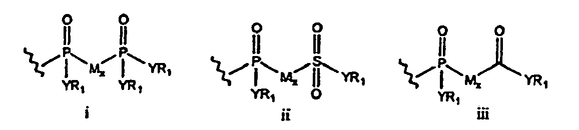 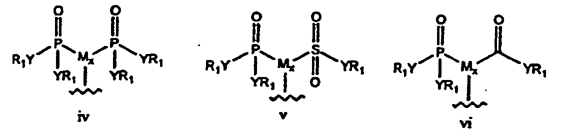 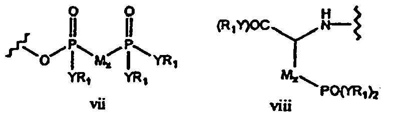 wherein each occurrence of M is independently CH₂, CHV, CHOH, or CV₂; each occurrence of Y is independently a covalent bond, -O-, -S- or N(R_{J})₂, wherein R_{J}, for each occurrence, is independently hydrogen, aliphatic, heteroaliphatic, aryl, heteroaryl, alkylaryl, or alkylheteroaryl; wherein V is a halogen; each occurrence of x is independently 1-6, and in certain embodiments is 1 or 2; and each occurrence of R₁ is independently hydrogen, aliphatic, heteroaliphatic, aryl, heteroaryl, alkylaryl, alkylheteroaryl, a prodrug or pharmaceutically acceptable derivative; and wherein m is 1-3.

Thus, it will be appreciated that the moiety AK can be attached directly to the purinyl ring (when HA is absent) or can be attached through a moiety HA: -O-, -S- or -NH-. In certain embodiments, HA is absent. In other embodiments, HA is -NH-. In still other embodiments, HA is -O- or -S-.

In certain embodiments, Px is i-vii, m is 1 and one or more occurrences of Y is O. In certain other embodiments, one or more occurrences of Y is a covalent bond. In yet other embodiments, Px is -P(X)YRG YR_{H}, one or more occurrences of Y is O, X is O, R_{G} and R_{H} are each hydrogen or aliphatic, and m is 2 or 3. In still other embodiments, one or more occurrences of R₁ is hydrogen. In still other embodiments, in particular for those compounds for use in the treatment of cancer as described herein occurrences of R_{G}, R_{H}, R₁ are each independently alkyl, heteroalkyl, aryl, heteroaryl, alkylaryl, or alkylheteroaryl.

For example, in certain embodiments, R_{D} is hydrogen.

In still other embodiments, R_{A} is an aryl, heteroaryl, alkylaryl, or alkylheteroaryl moiety optionally substituted with one or more hydroxyl moieties.

In yet other embodiments, R_{A} is a substituted or unsubstituted branched or unbranched, cyclic or acyclic aliphatic or heteroaliphatic moiety optionally substituted by one of more hydroxyl moieties.

As described herein, other compounds of special interest include those compounds wherein preferred substitutions of R_{A}, R_{B} and R_{D} include, but are not limited to the following moieties:

In certain embodiments, R_{D} is hydrogen.

In still other embodiments, R_{B} is an aryl, heteroaryl, alkylaryl, or alkylheteroaryl optionally substituted with one or more halogen groups. In compounds of special interest, the halogen is chlorine.

In still other compounds of special interst, R_{B} is phenyl.

In yet other embodiments R_{A} is an aryl, heteroaryl, alkylaryl, or alkylheteroaryl moiety optionally substituted with one or more hydroxyl moieties.

In still other embodiments, R_{A} is a substituted or unsubstituted branched or unbranched, cyclic or acyclic aliphatic or heteroaliphatic moiety optionally substituted by one of more hydroxyl moieties.

In further embodiments, R_{A} is a branched or unbranched, cyclic or acyclic aliphatic or heteroaliphatic moiety.

In still other compounds of interest R_{A} is CH(CH₃)₂, Me, cyclopentyl or cyclohexyl.

It will be appreciated that the inventive compounds as described above and herein can be prepared using a variety of synthetic techniques known in the art, and as described herein. In certain embodiments, combinatorial methods, either solution phase or solid phase are utilized, as described generally below and in more detail in the Exemplification section.

### Solid Phase Synthesis and Combinatorial Libraries of Purine Compounds

It will be appreciated that, in addition to preparing the inventive compounds using traditional solution phase techniques, the present invention contemplates the preparation of compounds and libraries of compounds using solid phase techniques. Thus, the desired components may be modified so that they may be attached to the solid support. The use of a solid support bound component enables the use of more rapid split and pool techniques to generate larger libraries (e.g., greater than 10,000 members) more easily. It will be appreciated that solid phase parallel synthesis techniques also can be utilized, such as those described in U.S. Patents 5,712,171 and 5,736,412.

A solid support, for the purposes of this invention, is defined as an insoluble material to which compounds are attached during a synthesis sequence. The use of a solid support is advantageous for the synthesis of libraries because the isolation of support-bound reaction products can be accomplished simply by washing away reagents from the support-bound material and therefore the reaction can be driven to completion by the use of excess reagents. Additionally, the use of a solid support also enables the use of specific encoding techniques to "track" the identity of the inventive compounds in the library. A solid support can be any material which is an insoluble matrix and can have a rigid or semi-rigid surface. Exemplary solid supports include, but are not limited to, pellets, disks, capillaries, hollow fibers, needles, pins, solid fibers, cellulose beads, pore-glass beads, silica gels, polystyrene beads optionally cross-linked with divinylbenzene, grafted co-poly beads, poly-acrylamide beads, latex beads, dimethylacrylamide beads optionally crosslinked with N-N'-bis-acryloylethylenediamine, and glass particles coated with a hydrophobic polymer. One of ordinary skill in the art will realize that the choice of particular solid support will be limited by the compatability of the support with the reaction chemistry being utilized. An exemplary solid support is a Tentagel amino resin, a composite of 1) a polystyrene bead crosslinked with divinylbenzene and 2) PEG (polyethylene glycol), is employed for use in the present invention. Tentagel is a particularly useful solid support because it provides a versatile support for use in on-bead or off-bead assays, and it also undergoes excellent swelling in solvents ranging from toluene to water.

Specific compounds may be attached directly to the solid support or may be attached to the solid support through a linking reagent. Direct attachment to the solid support may be useful if it is desired not to detach the library member from the solid support. For example, for direct on-bead analysis of biological/pharmacological activitiy or analysis of the compound structure, a stronger interaction between the library member and the solid support may be desirable. Alternatively, the use of a linking reagent may be useful if more facile cleavage of the inventive library members from the solid support is desired.

Furthermore, any linking reagent used in the present invention may comprise a single linking molecule, or alternatively may comprise a linking molecule and one or more spacer molecules. A spacer molecule is particularly useful when the particular reaction conditions require that the linking molecule be separated from the library member, or if additional distance between the solid support/linking unit and the library member is desired. In one particularly preferred embodiment, photocleavable linkers are employed to attach the solid phase resin to the component. Photocleavable linkers are advantageous because of the ability to use these linkers in in vivo screening strategies. Once the compound is released from the solid support via photocleavage, the compound is able to enter the cell. Exemplary photocleavable linkers include, but are not limited to ortho-Nitrobenzyl photolinkers and dithiane protected benzoin photolinkers. One of ordinary skill in the art will realize that the method of the present invention is not limited to the use of photocleavable linkers; rather other linkers may be employed, preferably those that are capable of delivering the desired compounds in vivo.

Thus, the synthesis of libraries of purine compounds can be performed using established combinatorial methods for solution phase, solid phase, or a combination of solution phase and solid phase synthesis techniques. The synthesis of combinatorial libraries is well known in the art and has been reviewed (see, e.g., "Combinatorial Chemistry", Chemical and Engineering News, Feb. 24, 1997, p. 43; Thompson, L.A., Ellman, J.A., *Chem. Rev.* **1996,** *96*, 555).

One of ordinary skill in the art will realize that the choice of method will depend upon the specific number of compounds to be synthesized, the specific reaction chemistry, and the availability of specific instrumentation, such as robotic instrumentation for the preparation and analysis of the inventive libraries. In particularly preferred embodiments, the reactions to be performed on the inventive scaffolds to generate the libraries are selected for their ability to proceed in high yield, and in a stereoselective fashion, if applicable.

In one embodiment of the present invention, libraries are generated using a solution phase technique. Traditional advantages of solution phase techniques for the synthesis of combinatorial libraries include the availability of a much wider range of organic reactions, and the relative ease with which products can be characterized. In a preferred embodiment, for the generation of a solution phase combinatorial library, a parallel synthesis technique is utilized, in which all of the products are assembled separately in their own reaction vessels. In a particularly preferred parallel synthesis procedure, a microtitre plate containing n rows and m columns of tiny wells which are capable of holding a few milliliters of the solvent in which the reaction will occur, is utilized. It is possible to then use n variants of reactant A, and m variants of reactant B, to obtain n x m variants, in n x m wells. One of ordinary skill in the art will realize that this particular procedure is most useful when smaller libraries are desired, and the specific wells can provide a ready means to identify the library members in a particular well.

In another embodiment of the present invention, a solid phase synthesis technique is utilized, in which the desired scaffold structures are attached to the solid phase directly or though a linking unit, as discussed above. Advantages of solid phase techniques include the ability to more easily conduct multi-step reactions and the ability to drive reactions to completion because excess reagents can be utilized and the unreacted reagent washed away. Perhaps one of the most significant advantages of solid phase synthesis is the ability to use a technique called "split and pool", in addition to the parallel synthesis technique, develped by Furka. (Furka et al., *Abstr. 14th Int. Congr. Biochem.,* Prague, Czechoslovakia, **1988,** *5*, **47**; Furka et al., *Int. J. Pept. Protein Res.* **1991,** *37*, 487; Sebestyen et al., *Bioorg. Med. Chem. Lett.,* **1993,** *3*, 413) In this technique, a mixture of related compounds can be made in the same reaction vessel, thus substantially reducing the number of containers required for the synthesis of very large libraries, such as those containing as many as or more than one million library members. As an example, the solid support scaffolds can be divided into n vessels, where n represents the number species of reagent A to be reacted with the scaffold structures. After reaction, the contents from n vessels are combined and then split into m vessels, where m represents the number of species of reagent B to be reacted with the scaffold structures. This procedure is repeated until the desired number of reagents is reacted with the scaffold structures to yield the inventive library.

The use of solid phase techniques in the present invention may also include the use of a specific encoding technique. Specific encoding techniques have been reviewed by Czamik. (Czarnik, A.W., *Current Opinion in Chemical Biology,* **1997,** *1*, 60) As used in the present invention, an encoding technique involves the use of a particular "identifiying agent" attached to the solid support, which enables the determination of the structure of a specific library member without reference to its spatial coordinates. One of ordinary skill in the art will also realize that if smaller solid phase libraries are generated in specific reaction wells, such as 96 well plates, or on plastic pins, the reaction history of these library members may also be identified by their spatial coordinates in the particular plate, and thus are spatially encoded. It is most preferred, however for large combinatorial libraries, to use an alternative encoding technique to record the specific reaction history.

Examples of alternative encoding techniques that can be utilized in the present invention include, but are not limited to, spatial encoding techniques, graphical encoding techniques, including the "tea bag" method, chemical encoding methods, and spectrophotometric encoding methods. Spatial encoding refers to recording a reaction's history based on its location. Graphical encoding techniques involve the coding of each synthesis platform to permit the generation of a relational database. Examples of preferred spectrophotometic encoding methods include the use of mass spectroscopy, fluorescence emission, and nuclear magnetic resonance spectroscopy. In a preferred embodiment, chemical encoding methods are utilized, which uses the structure of the reaction product to code for its identity. Decoding using this method can be performed on the solid phase or off of the solid phase. One of ordinary skill in the art will realize that the particular encoding method to be used in the present invention must be selected based upon the number of library members desired, and the reaction chemistry employed.

Subsequent characterization of the library members, or individual compounds, can be performed using standard analytical techniques, such as mass spectrometry, Nuclear Magnetic Resonance Spectroscopy, and gas chromatrograpy.

Once specific libraries of compounds have been prepared, specific assay techniques, such as those described herein, may be utilized to test the activity of the inventive purine compounds (e.g., in one embodiment, to function as Src kinase inhibitors). In certain preferred embodiments, high throughput assay techniques are utilized.

### Pharmaceutical Compositions

As discussed above the present invention provides novel compounds which are useful in the treatment of bone disorders and in cancer, as evidenced by their anti-resorption activity and their cytotoxicity. Accordingly, in another aspect of the present invention, pharmaceutical compositions are provided, wherein these compositions comprise any one of the compounds as described herein, and optionally comprise a pharmaceutically acceptable carrier. In certain preferred embodiments, these compositions optionally further comprise one or more additional therapeutic agents. In certain other embodiments, the additional therapeutic agent is an anticancer agent, or an approved agent for the treatment of a bone disorder, as discussed in more detail herein.

Thus, according to another aspect of the present invention, pharmaceutical compositions are provided comprising compounds as described and a pharmaceutically acceptable carrier.

It will also be appreciated that certain of the compounds of present invention can exist in free form for treatment, or where appropriate, as a pharmaceutically acceptable derivative thereof. According to the present invention, a pharmaceutically acceptable derivative includes, but is not limited to, pharmaceutically acceptable salts, esters, salts of such esters, or any other adduct or derivative which upon administration to a patient in need is capable of providing, directly or indirectly, a compound as otherwise described herein, or a metabolite or residue thereof, e.g., a prodrug.

As used herein, the term "pharmaceutically acceptable salt" refers to those salts which are, within the scope of sound medical judgement, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, *et al.* describe pharmaceutically acceptable salts in detail in *J*. *Pharmaceutical Sciences, 66:* 1-19 (**1977**). The salts can be prepared in situ during the final isolation and purification of the compounds of the invention, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hernisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

Additionally, as used herein, the term "pharmaceutically acceptable ester" refers to esters which hydrolyze in vivo and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters includes formates, acetates, propionates, butyrates, acrylates and ethylsuccinates.

Furthermore, the term "pharmaceutically acceptable prodrugs" as used herein refers to those prodrugs of the compounds of the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transformed in vivo to yield the parent compound of the above formula, for example by hydrolysis in blood. A thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987.

As described above, the pharmaceutical compositions of the present invention additionally comprise a pharmaceutically acceptable carrier, which, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences, Fifteenth Edition, E. W. Martin (Mack Publishing Co., Easton, Pa., 1975) discloses various carriers used in formulating pharmaceutical compositions and known techniques for the preparation thereof. Except insofar as any conventional carrier medium is incompatible with the anti-viral compounds of the invention, such as by producing any undesirable biological effect or otherwise interacting in a deleterious manner with any other component(s) of the pharmaceutical composition, its use is contemplated to be within the scope of this invention. Some examples of materials which can serve as pharmaceutically acceptable carriers include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

### Uses of Compounds of the Invention

As discussed herein, the compounds of the present invention have been shown to inhibit tyrosine kinase activity, and in one embodiment have been shown to inhibit Src tyrosine kinase activity (see exemplification herein). In certain other embodiments, the inventive compounds are have been shown to inhibit osteoclast activity. As such, the compounds of the invention are useful in the treatment of bone disorders and cancer.

Thus, in one aspect, the present invention provides the use of the compounds for the preparation of a pharmaceutical composition for treating cancer for the inhibition of cellular (preferably tumor) growth and for the killing of tumor cells.

Compounds which are useful for the treatment of certain cancers, and, in certain embodiments, leukemia, include compounds as described herein and those compounds as shown above, in which Y and R₁ are as defined herein, and in certain compounds of special interest Y is O; R_{D} is hydrogen, R_{A} is an aliphatic or alkylaryl substituted with one or more hydroxyl moieties. It is generally preferred that the inventive compounds exhibit activity of less than 500 µM or less than 100 µM, and it is particularly preferred that the inventive compounds exhibit activity of less than 1 nM.

Additionally, compounds as described herein are useful for the treatment of bone disorders, and thus, in another aspect, the use of the compounds for the preparation of a pharmaceutical composition for the treatment of bone disorders is provided.

In certain embodiments of the present invention a "therapeutically effective amount" of the inventive compound or pharmaceutical composition is that amount effective for killing or inhibiting the growth of tumor cells, or is an amount that is effective for promoting or inhibiting osteoclast activity, which activity is believed to be involved in the effect of bone disorders, although the present invention is not intended to be bound by any particular theory.

The compounds and compositions, according to the present invention, may be administered using any amount and any route of administration effective for killing or inhibiting the growth of tumor cells, or for treating bone disorders. Thus, the expression "effective amount" as used herein, refers to a sufficient amount of agent to kill or inhibit the growth of tumor cells or to treat and/or prevent bone disorders. The exact amount required will vary from subject to subject, depending on the species, age, and general condition of the subject, the severity of the infection, the particular anticancer agent, its mode of administration, and the like. The anticancer compounds of the invention are preferably formulated in dosage unit form for ease of administration and uniformity of dosage. The expression "dosage unit form" as used herein refers to a physically discrete unit of anticancer agent appropriate for the patient to be treated. It will be understood, however, that the total daily usage of the compounds and compositions of the present invention will be decided by the attending physician within the scope of sound medical judgment. The specific therapeutically effective dose level for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

Furthermore, after formulation with an appropriate pharmaceutically acceptable carrier in a desired dosage, the pharmaceutical compositions of this invention can be administered to humans and other animals orally, rectally, parenterally, intracisternally, intravaginally, intraperitoneally, topically (as by powders, ointments, or drops), bucally, as an oral or nasal spray, or the like, depending on the severity of the infection being treated. In certain embodiments, the compounds of the invention may be administered orally or parenterally at dosage levels of about 0.01 mg/kg to about 50 mg/kg and preferably from about 1 mg/kg to about 25 mg/kg, of subject body weight per day, one or more times a day, to obtain the desired therapeutic effect.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

Compositions for rectal or vaginal administration are preferably suppositories which can be prepared by mixing the compounds of this invention with suitable nonirritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar--agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes. Solid compositions of a similar type may also be employed as fillers in soft and hard-filled gelatin capsules using such excipients as lactose or milk sugar as well as high molecular weight polethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, and eye drops are also contemplated as being within the scope of this invention. Additionally, the present invention contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

As discussed above, in one aspect, the compounds of the present invention are useful as anticancer agents, and thus may be useful in the treatment of cancer, by effecting tumor cell death or inhibiting the growth of tumor cells. In general, the inventive anticancer agents are useful in the treatment of cancers and other proliferative disorders, including, but not limited to breast cancer, cervical cancer, colon and rectal cancer, leukemia, lung cancer, melanoma, multiple myeloma, non-Hodgkin's lymphoma, ovarian cancer, pancreatic cancer, prostate cancer, and gastric cancer, to name a few. In certain embodiments, the inventive anticancer agents are active against leukemia cells and melanoma cells, and thus are useful for the treatment of leukemias (e.g., myeloid, lymphocytic, myelocytic and lymphoblastic leukemias) and malignant melanomas. In still other embodiments, the inventive anticancer agents are active against solid tumors and also kill and/or inhibit the growth of multidrug resistant cells (MDR cells).

It will also be appreciated that the compounds and pharmaceutical compositions of the present invention can be employed in combination therapies, that is, the compounds and pharmaceutical compositions can be administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, an inventive compound may be administered concurrently with another anticancer agent), or they may achieve different effects (e.g., control of any adverse effects).

For example, other therapies or anticancer agents that may be used in combination with the inventive anticancer agents of the present invention include surgery, radiotherapy (in but a few examples, γ-radiation, neutron beam radiotherapy, electron beam radiotherapy, proton therapy, brachytherapy, and systemic radioactive isotopes, to name a few), endocrine therapy, biologic response modifiers (interferons, interleukins, and tumor necrosis factor (TNF) to name a few), hyperthermia and cryotherapy, agents to attenuate any adverse effects (e.g., antiemetics), and other approved chemotherapeutic drugs, including, but not limited to, alkylating drugs (mechlorethamine, chlorambucil, Cyclophosphamide, Melphalan, Ifosfamide), antimetabolites (Methotrexate), purine antagonists and pyrimidine antagonists (6-Mercaptopurine, 5-Fluorouracil, Cytarabile, Gemcitabine), spindle poisons (Vinblastine, Vincristine, Vinorelbine, Paclitaxel), podophyllotoxins (Etoposide, Irinotecan, Topotecan), antibiotics (Doxorubicin, Bleomycin, Mitomycin), nitrosoureas (Carmustine, Lomustine), inorganic ions (Cisplatin, Carboplatin), enzymes (Asparaginase), and hormones (Tamoxifen, Leuprolide, Flutamide, and Megestrol), to name a few. For a more comprehensive discussion of updated cancer therapies see, http://www.nci.nih.gov/, a list of the FDA approved oncology drugs at http://www.fda.gov/cder/cancer/druglistframe.htm, and The Merck Manual, Seventeenth Ed. 1999, the entire contents of which are hereby incorporated by reference.

As discussed above, in another aspect, the compounds of the present invention are useful in the selective treatment or prevention of bone disorders, and may effect treatment via inhibition of osteoclast activity, promotion of osteoblast activity, or promotion or inhibition of other cellular events necessary for healthy bone metabolism. In certain preferred embodiments, these compounds are useful for the treatment or prevention of diseases and conditions associated with bone metabolic disorders such as osteoclast overactivity. In still other preferred embodiments, the compounds of the present invention are targeted Src kinase inhibitors and thus inhibit bone resorption by osteoclasts.

The present invention therefore provides the use in the treatment, prophylaxis, and/or prevention of bone and other related disorders which method comprises the administration of an effective non-toxic amount of an inventive compound, or a pharmaceutically composition thereof. As mentioned above, although the inventive compounds effect treatment via several mechanisms, (i.e. inhibition of osteoclast activity, promotion of osteoblast activity, or regulation of other cellular events necessary for healthy bone metabolism), in certain preferred embodiments, these compounds are selective inhibitors of osteoclast activity.

In a further aspect, the present invention provides an inhibitor of mammalian osteoclasts, for example any one of the compounds of the present invention or a pharmaceutical composition thereof. In still another aspect, the present invention provides compounds or pharmaceutical compositions that are selective Src kinase inhibitors. In particular, the method of present invention comprises providing any one of the compounds of the present invention or a pharmaceutically composition thereof, for use in the treatment of and/or prophylaxis of osteoporosis and related osteopenic diseases.

It will be appreciated that, in addition to the treatment or prevention of osteoporosis, particularly osteoporosis associated with the peri and post menopausal conditions, the present invention also contemplates the treatment and prophylaxis or prevention of Paget's disease, hypercalcemia associated with bone neoplasms and other types of osteoporotic diseases and related disorders, including but not limited to involutional osteoporosis, Type I or postmenopausal osteoporosis, Type II or senile osteoporosis, juvenile osteoporosis, idiopathic osteoporosis, endocrine abnormality, hyperthyroidism, hypogonadism, ovarian agensis or Turner's syndrome, hyperadrenocorticism or Cushing's syndrome, hyperparathyroidism, bone marrow abnormalities, multiple myeloma and related disorders, systemic mastocytosis, disseminated carcinoma, Gaucher's disease, connective tissue abnormalities, osteogenesis imperfecta, homocystinuria, Ehlers-Danlos syndrome, Marfan's syndrome, Menke's syndrome, immobilization or weightlessness, Sudeck's atrophy, chronic obstructive pulmonary disease, chronic heparin administration, and chronic ingestion of anticonvulsant drugs

### Treatment Kits

In other embodiments, the present invention relates to a kit for conveniently and effectively carrying out the methods in accordance with the present invention. In general, the pharmaceutical pack or kit comprises one or more containers filled with one or more of the ingredients of the pharmaceutical compositions of the invention. Such kits are especially suited for the delivery of solid oral forms such as tablets or capsules. Such a kit preferably includes a number of unit dosages, and may also include a card having the dosages oriented in the order of their intended use. If desired, a memory aid can be provided, for example in the form of numbers, letters, or other markings or with a calendar insert, designating the days in the treatment schedule in which the dosages can be administered. Alternatively, placebo dosages, or calcium dietary supplements, either in a form similar to or distinct from the substituted purine dosages, can be included to provide a kit in which a dosage is taken every day. Optionally associated with such container(s) can be a notice in the form prescribed by a governmental agency regulating the manufacture, use or sale of pharmaceutical products, which notice reflects approval by the agency of manufacture, use or sale for human administration.

### Equivalents

The representative examples which follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. It should further be appreciated that the contents of those cited references are incorporated herein by reference to help illustrate the state of the art.

The following examples contain important additional information, exemplification and guidance which can be adapted to the practice of this invention in its various embodiments and the equivalents thereof.

### Exemplification

The described phosphorus-containing moieties can be synthesized according to the schemes outlined below:

### Example 1

### [(3-Amino-propyl)-ethoxy-phosphinoylmethyl]-phosphonic acid diethyl ester

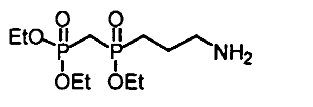

### [(3-Benzyloxy-propyl)-ethoxy-phosphinoylmethyl]-phosphonic acid diethyl ester:

To an oven-dried flask was added 10.25 g (44.7 mmol) of (3-Bromo-propoxymethyl)-benzene and 7.67 mL (44.7mmol) of triethyl phosphite. The flask was fitted with a short-path distillation head, for removal of bromoethane, and the mixture heated at 150 °C for 4 h. The reaction was cooled to ambient temperature, and then diluted with 120 mL of absolute ethanol and 1.8 N KOH (120mL, 216 mol). The distillation head was replaced with a reflux condenser and the solution heated at reflux for 5 h. The reaction was cooled then concentrated *in vacuo.* The basic aqueous layer was extracted with EtOAc (2x) and then acidified to pH 3 with conc. HCl. The aqueous layer was extracted with EtOAc (3x) and the combined extracts were dried over MgSO₄ and concentrated. The resulting crude product (8.24 g) was used as is in the next reaction. ³¹P NMR (300 MHz, DMSO-*d*₆) δ 34.113.

To a solution of the crude phosphonate (8.24 g, 32.5 mmol) in 100 mL CH₂Cl₂, under an atmosphere of N₂, was added 10.8 mL (113.8 mmol) of oxalyl chloride. DMF (several drops) was slowly added to initiate the reaction. After gas evolution had ceased, the reaction was stirred for 30 min at ambient temperature. Upon concentration *in vacuo,* the residue was titurated several times with hexane, then dissolved in 167 mL of anhydrous THF. In a separate flask, a cooled (-78 °C, under N₂) solution of diethyl methylphosphonate (10.25 mL, 69.9 mmol) in 337 mL of anhydrous THF was added 2.5 M n-butyl lithium (27.95 mL, 69.9 mmol) dropwise. The reaction mixture was stirred for 30 min at -78 °C, at which time the *in situ* generated acid chloride was added dropwise. The solution was stirred for an additional 2.5 h at -78 °C, quenched with 5 mL glacial acetic acid, and then warmed to ambient temperature. Water was added to the reaction mixture and the THF was removed *in vacuo.* The aqueous layer was extracted with EtOAc (3x) and the combined organics washed with saturated NaHCO₃, brine, then dried over MgSO₄ and concentrated. The crude product was purified by silica gel chromatography (eluted with 50:1 CH₂Cl₂/MeOH) affording 6.15 g of a yellow oil. ³¹P NMR (300 MHz, DMSO-*d*₆) δ 51.479, 26.291.

### [(3-Amino-propyl)-ethoxy-phosphinoy/methyl]-phosphonic acid diethyl ester:

To a solution of [(3-Benzyloxy-propyl)-ethoxy-phosphinoylmethyl]-phosphonic acid diethyl ester (5.7 g, 14.5 mmol) in 100 mL of EtOH was added 1.2 g of palladium on carbon. The mixture was flushed with H₂ and stirred at ambient temperature (H₂ balloon) for 1 h. The reaction mixture was filtered through Celite and the solvent evaporated to provide 3.5 g of a pale yellow oil. ³¹P NMR (300 MHz, DMSO-*d*₆) d 52.219, 26.317.

To a cooled (0 °C, under N₂) solution of the crude alcohol (3.5 g, 14.5 mmol) in 53 mL of CH₂Cl₂ was added 2.4 mL (17.4 mmol) of triethylamine followed by 1.25 mL (16 mmol) of methanesulfonyl chloride. The reaction mixture was warmed to ambient temperature and stirred for 1 h. The reaction mixture was then quenched with water and the layers separated. The organic layer was washed with water and brine, dried over Na₂SO₄, and concentrated. The crude orange-yellow oil (5.5 g) was used as is in the next reaction. ³¹P NMR (300 MHz, DMSO-*d*₆) δ 51.135, 26.614.

To a solution of the crude mesylate (5.5 g, 14.4 mmol) in 17 mL DMF was added 4.7 g (72.4 mmol) of sodium azide. The resulting slurry was heated at 55 °C and stirred overnight. The reaction mixture was diluted with EtOAc and washed with water (2x). The combined organics were then dried over Na₂SO₄ and concentrated. The crude azide (2.61 g) was used as is in the next reaction. ³¹P NMR (300 MHz, DMSO-*d*₆) d 51.230,26.183.

To a solution of the crude azide (2.61 g, 8 mmol) in 100 mL of EtOH was added 0.8 g of palladium on carbon. The mixture was flushed with H₂ and stirred at ambient temperature (H₂ balloon) for 16 h. The reaction mixture was filtered through Celite and the solvent evaporated to provide 2.3 g of a yellow oil: ¹H NMR (300 MHz, *DMSO-d*₆) δ 4.03 (m, 6H), 2.84-2.52 (m, 4H), 1.91-1.80 (m, 2H), 1.65-1.61 (m, 2H), 1.23 (m,9H). ³¹P NMR (300 MHz, *DMSO-d*₆) δ 51.757, 26.344.

### Example 2

### [(4-Amino-phenyl)-ethoxy-phosphinoylmethyl]-phosphonic acid diethyl ester

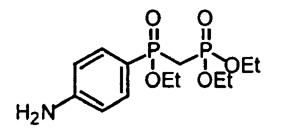

### [(4-Nitro-phenyl)-ethoxy-phosphinoylmethyl]-phosphonic acid diethyl ester:

A mixture of diethyl (ethoxyphosphinyl)methylphosphonate (2.35 g, 9.62 mmol), Et₃N (3.8 mL, 27.5 mmol), 1-iodo-4-nitrobenzene (2.28 g, 9.17 mmol) and Pd(PPh₃)₄ (265 mg, 0.229 mmol) in CH₃CN (14 mL) under N₂ was stirred at 80 °C for 2.5 h. After cooling to rt, the reaction mixture was poured into 50 mL of 1 *N* aq HCl and extracted with CH₂Cl₂. The extract was washed with H₂O (50 mL) and brine (50 mL). The aqueous washes were reextracted once with CH₂Cl₂, and the combined extracts were dried over Na₂SO₄ and concentrated. The crude material was purified by flash chromatography on silica gel. Elution with 30:1 CHCl₃-MeOH followed by 20:1 CHCl₃-MeOH and finally 15:1 CHCl₃-MeOH afforded 3.28 g of the desired (arylphosphinylmethyl)phosphonate.

### [(4-Amino-phenyl)-ethoxy-phosphinoylmethyl]-phosphonic acid diethyl ester:

A mixture of [(4-nitro-phenyl)-ethoxy-phosphinoylmethyl]-phosphonic acid diethyl ester (940 mg, 2.57 mmol) and SnCl₂•2H₂O (2.9 g, 12.9 mmol) in EtOH (~10 mL) was stirred at 70 °C for 44 min and then concentrated at ambient temperature. The residue was taken up in CH₂Cl₂ and washed with half saturated aq NaHCO₃ (40 mL), H₂O (40 mL) and brine (40 mL). The aqueous washes were reextracted once with CH₂Cl₂, and the combined extracts were dried over K₂CO₃ and concentrated. The crude material was purified by flash chromatography on silica gel. Elution with 20:1 CHCl₃-MeOH followed by 15:1 CHCl₃-MeOH afforded 657 mg of product.

### Example 3

### Synthesis of Phenyl Dialkyl Phosphine Oxides

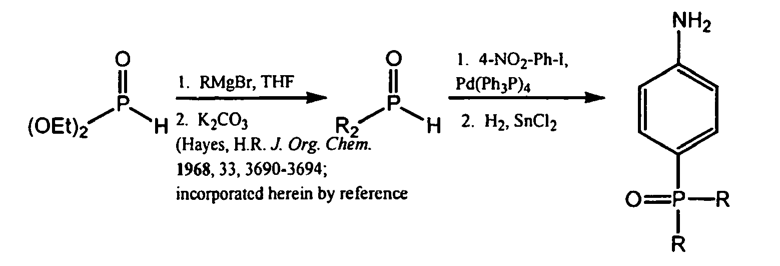

Each occurrence of R in the above scheme is independently a substituted or unsubstituted aliphatic, heteroaliphatic, aryl, heteroaryl, alkylaryl, or alkylheteroaryl group.

### Example 4

In one embodiment, compounds as disclosed and described herein can be synthesized by solution-phase methods according to the scheme outlined below:

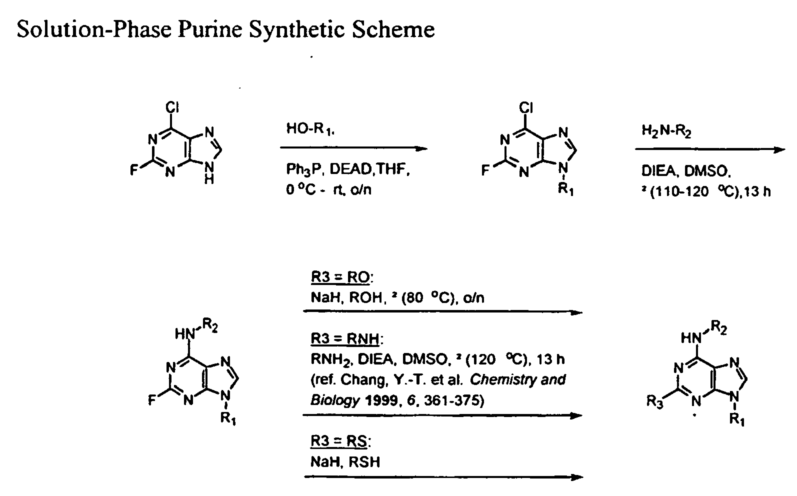

### Example 5

### ({2-[6-(3-Chloro-phenylamino)-9-isopropyl-9H-purin-2-ylamino]-ethoxy}-hydroxy-phosphorylmethyl)-phosphonic acid

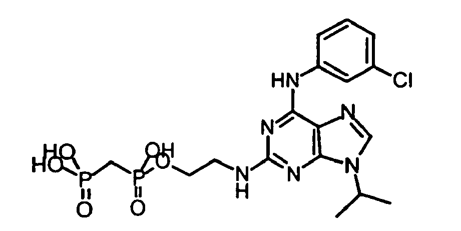

### 6-Chloro-2-fluoro-9H-purine:

A 0.3 M aqueous solution of NaNO₂ (200 mL, 60 mmol) was added dropwise to a cooled (~15 °C), vigorously stirred suspension of 2-amino-6-chloro-9*H*-purine (6.0 g, 35.4 mmol) in 120 mL HBF₄ (48 w% in H₂O, 0.92 mol) over 75 min. The pale yellow reaction was stirred at r. t. for 20 min and then recooled to -15 °C and neutralized to PH = 6.0 with aqueous NaOH (50 w% in H₂O). The water was removed *in vacuo* and the resulting orange solid chromatographed on silica gel (90 : 10 CH₂Cl₂: MeOH, Rf 0.50). The final product was obtained as white solid (3.0 g, 49.1 %).

### 6-Chloro-2-fluoro-9-isopropyl-9H-purine:

6-Chloro-2-fluoro-9*H*-purine (517.7 mg, 3 mmol), 2-propanol (198.3 mg, 3.3 mmol), PPh₃ (866 mg, 3.3 mmol) was mixed under N₂ in a 50 mL round-bottom flask at 0 °C. DEAD (575 mg, 3.3 mmol) was added via syringe dropwise to the mixture. The temperature was raised to r. t. and the mixture was stirred overnight. Sovent was removed *in vacuo* and the resulting residue was chromatographed on silica gel (CH₂Cl₂/EtOAc, 4:1, Rf 0.62). The product was obtained as a white solid (411 mg, 64 %).

### (3-Chlorophenyl)-(2-fluoro-9-isopropyl-9H-purin-6-yl)amine:

6-Chloro-2-fluoro-9-isopropyl-9*H* purine (214 mg, 1 mmol) was mixed with 3-chloroaniline (127.6 mg, 1 mmol) in 12 mL n-BuOH. DIEA (357.6 mg, 2.8 mmol) was added and the solution was heated at 90 °C overnight. Solvent was removed *in vacuo* and the residue was chromatographed on silica gel (CH₂Cl₂/EtOAc 2:2, Rf 0.44) to get the product as a white solid (148 mg, 48 %).

### 2-(6-(3-chlorophenylamino-9-isopropyl-9H-purin-2-ylamino)ethanol:

(3-Chlorophenyl)-(2-fluoro-9-isopropyl-9*H*-purin-6-yl)amine (92 mg, 0.3 mmol) and ethanolamine (92 mg, 1.5 mmol) was mixed in 5 mL nBuOH/DMSO (4/1 v/v) and heated at 120 °C overnight. Solvent was removed *in vacuo.* The residue was chromatographed on silica gel (EtOAc, Rf 0.45) to get the product as a greenish solid (24 mg, 90 %).

### ({2-[6-(3-Chloro-phenylammo)9-isopropyl-9H-purin-2-ylamino]-ethoxy}-hydroxy-phosphorylmethyl)-phosphonic acid:

2-(6-(3-chlorophenylamino-9-isopropyl-9H-purin-2-ylamino)ethanol (180 mg, 0.52 mmol) was dissolved in 3 mL trimethyl phosphate at 0 °C. Methylenebis(phosphonic dichloride) (514 mg, 2.1 mmol) was added in one portion and the reaction was stirred at 0 °C for 16 hrs. The solution was neutralized with 5 N ammonia to PH 6.0. The resulting mixture was purified by RP HPLC. Lyophilization left a white solid (147 mg, 56 %).

### Example 6

### ({2-[6-(3-Chloro-phenylamino)-9-isopropyl-9H-purin-2-ylamino]-3-methyl-butoxy}-hydroxy-phosphorylmethyl)-phosphonic acid

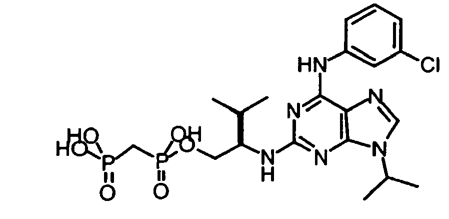

The title compound was synthesized in a manner similar to that described in Example 5. ES-MS: *m*/*z* 546 (M-H).

### Example 7

### ({3-[6-(3-Chloro-phenylamino)-9-isopropyl-9H-purin-2-ylamino]-propoxy}-hydroxy-phosphorylmethyl)-phosphonic acid

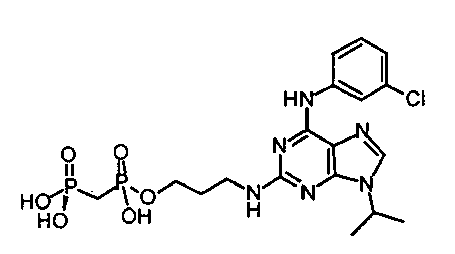

The title compound was synthesized in a manner similar to that described in Example 5. ES-MS: *m*/*z* 518 (M-H).

### Example 8

### ({4-[6-(3-Chloro-phenylamino)-9-isopropyl-9H-purin-2-ylamino]-butoxy}-hydroxy-phosphorylmethyl)-phosphonic acid

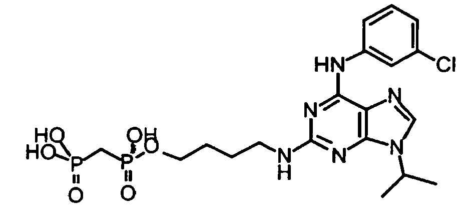

The title compound was synthesized in a manner similar to that described in Example 5. ES-MS: *m*/*z* 532 (M-H).

### Example 9

### ({2-[6-(3-Chloro-phenylamino)-9-isopropyl-9H-purin-2-ylamino]-3-methyl-butoxy}-hydroxy-phosphorylmethyl)-phosphonic acid

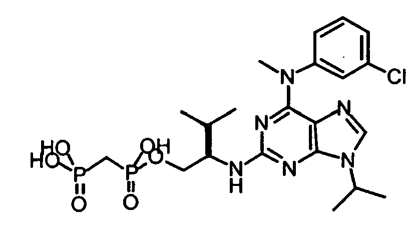

The title compound was synthesized in a manner similar to that described in Example 5. ES-MS: *m*/*z* 560 (M-H).

### Example 10

### ({2-[6-(3-Chloro-phenylamino)-9-methyl-9H-purin-2-ylamino]-3-methyl-butoxy}-hydroxy-phosphorylmethyl)-phosphonic acid

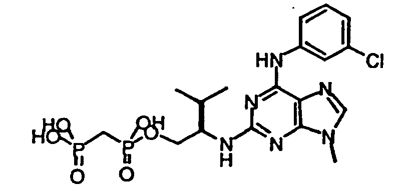

The title compound was synthesized in a manner similar to that described in Example 5. ES-MS: *m*/*z* 518 (M-H).

### Reference Example 11

### [(4-{2-Ethoxy-9-[2-(3-hydroxy-phenyl)-ethyl]-9H-purin-6-ylamino}-phenyl)-hydroxy-phosphinoylmethyl]-phosphonic acid

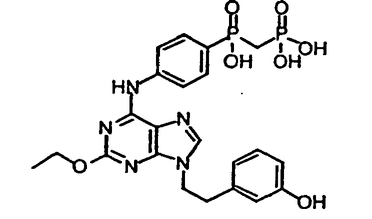

### 2-[3-(tert-Butyl-dimethyl-silanyloxy) phenyl]-ethanol:

To a solution of 3-hydroxyphenethyl alcohol (6.0 g, 43.4 mmol) in 275 mL of CH₂Cl₂ was added 6.55 g (43.4 mmol) of TBDMS-Cl (*tert*-butyldimethylsilyl chloride), cooled to 0 °C, then added 7.0 mL (86.8 mmol) of pyridine. The reaction mixture was stirred at ambient temperature overnight. Upon concentration, the crude mixture was purified by silica gel flash chromatography (eluted with hexane then 5% EtOAc/hexane) to provide 8.9 g of a clear oil: ¹H NMR (300 MHz, DMSO-*d*₆) δ 9.17 (s, 1H), 7.04 (m, 1H), 6.60 (m, 3H), 3.73 (t, J = 6.9 Hz, 2H), 2.65 (t, J = 6.9. Hz, 2H), 0.83 (s, 9H), -0.03 (s, 6H).

### 9-{2-[3-(tert-Butyl-dimethyl-silanyloxy)-phenyl]-ethyl}-6-chloro-2-fluoro-9H-purine:

To a flask containing 1.26 g (7.3 mmol) of 6-chloro-2-fluoro-9*H*-purine and 3.82 g (14.6 mmol) of triphenylphosphine, under an atmosphere of N₂, was added a solution of 2-[3-(*tert*-Butyl-dimethyl-silanyloxy)-phenyl]-ethanol (3.7 g, 14.6 mmol) in 40 mL of THF. To the cooled (0 °C) reaction mixture was added a solution of diethyl azodicarboxylate (2.3 mL, 14.6 mmol) in 40.0 mL of THF and the resulting solution allowed to stir to ambient temperature overnight. Upon quenching with H₂O (~1 .0 mL), the solvent was removed and the crude mixture was purified by silica gel flash chromatography (eluted with 10% EtOAc/hexane then 15% EtOAc/hexane) to provide 1.71 g of a white solid: ¹H NMR (300 MHz, DMSO-*d*₆) δ 8.52 (s, 1H), 7.11 (t, J = 7.8 Hz, 1H), 6.76 (d, J = 7.6 Hz, 1H), 6.65-6.62 (m, 1H), 6.49 (m, 1H), 4.48 (t, J = 6.8 Hz, 2H), 3.12 (t, J = 6.8 Hz, 2H), 0.88 (s, 9H), 0.06 (s, 6H); ¹⁹F NMR (DMSO-*d*₆) δ -48.23.

### [(4-Amino-phenyl)-hydroxy-phosphinoylmethyl]-phosphonic acid:

A solution of [(4-Amino-phenyl)-ethoxy-phosphinoylmethyl]-phosphonic acid diethyl ester (2.78 g, 8.29 mmol) in 250 mL of conc. HCl was heated at reflux (apparatus equipped with a NaOH trap) for 5 h. Concentrated solution via distillation to provide a sticky yellow-white solid, which was used without purification in the next step: *m*/*z* 252 (M+H).

### [(4-{2-Fluoro-9-[2-(3-hydroxy-phenyl)-ethyl]-9H-purin-6-ylamino}-phenyl)-hydroxy-phosphinoylmethyl]-phosphonic acid:

A scaled pressure flask, flushed with N₂, containing a mixture of crude [(4-Amino-phenyl)-hydroxy-phosphinoylmethyl]-phosphonic acid (8.29 mmol), 9-{2-[3-(*tert-*Butyl-dimethyl-silanyloxy)-phenyl]-ethyl}-6-chloro-2-fluoro-9*H*-purine (3.37 g, 8.29 mmol), and N,N-diisopropylethylamine (7.2 mL, 41.5 mmol) in 45.0 mL DMSO was heated at 110-120 °C for 13 h. Upon removing excess N,N-diisopropylethylamine (N₂ flow, slight heat) the slightly brown solution was cooled to ambient temperature and added ~500 mL of H₂O to produce a milky, off-white solution. Further precipitation occurred upon addition of TFA (final pH = 1-2), at which time the mixture was heated (100 °C hot plate) and stirred vigorously for ~20 min, filtered while hot, then washed with acidified H₂O (TFA, pH = 1-2; 4x20 mL), EtOH (2x20 mL), and Et₂O (4x20 mL). Isolation of a second crop from the concentrated filtrate in the same manner as described above, followed by removal of excess solvent (in vacuo) provided 2.47 g of an off-white solid: *m*/*z* 506 (M-H).

### [(4-{2-Ethoxy-9-[2-(3-hydroxy-phenyl)-ethyl]-9H -purin-6-ylamino}-phenyl)-hydroxy-phosphinoylmethyl]-phosphonic acid:

To a cooled flask (0 °C) under an atmosphere of N₂ containing 0.096 g (4.02 mmol) of sodium hydride was added 2.0 mL of absolute ethanol. After the effervescence had ceased, the solution was transferred via pipette to a cooled (0 °C) pressure flask, under an atmosphere of N₂, containing a mixture of [(4-{2-Fluoro-9-[2-(3-hydroxy-phenyl)-ethyl]-9H-purin-6-ylamino}-phenyl)-hydroxy-phosphinoylmethyl]-phosphonic acid (0.199 g, 0.39 mmol) in 2.0 mL of absolute ethanol. Upon sealing the reaction vessel, the resulting mixture was stirred at ambient temperature for ~10 min, then heated at 80 °C overnight Ethanol was removed via N₂ flow (slight heat) and the resulting thick suspension was dissolved in 25 mL of H₂O (adjusted to pH ~8 with 10% NaOH), filtered (0.2 µm, PTFE filter), and purified by RP-HPLC (CH₃CN/H₂O). Lyophilization provided a white solid (0.105 g): *m*/*z* 532 (M-H).

### Reference Example 12

### [Hydroxy-(4-{9-[2-(3-hydroxy-phenyl)-ethyl]-2-methoxy-9H-purin-6-ylamino}-phenyl)-phosphinoylmethyl]-phosphonic acid

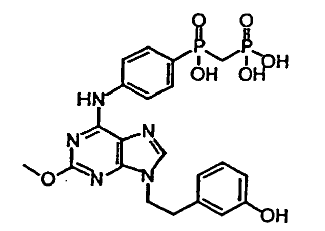

The title compound was synthesized in a manner similar to that described for Example 11. The product was obtained as a white solid: *m*/*z* 518 (M-H)

### Reference Example 13

### [Hydroxy-(4-{9-[2-(3-hydroxy-phenyl)-ethyl]-2-isopropoxy-9H-purin-6-ylamino}-phenyl)-phosphinoylmethyl]-phosohonic acid

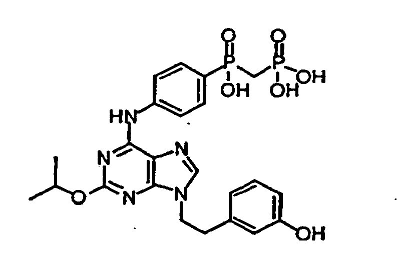

The title compound was synthesized in a manner similar to that described for Example 11. The product was obtained as a white solid: *m*/*z* 546 (M-H)

### Reference Example 14

### (Hydroxy-{4-[9-methyl-2-(piperidin-4-ylmethoxy)-9H-purin-6-ylamino]-phenyl}-phosphinoylmethyl)-phosphonic acid

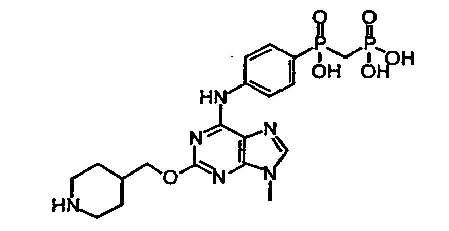

The title compound was synthesized in a manner similar to that described for Example 11. The product was obtained as a white solid: 495 *m*/*z* (M-H)

### Reference Example 15

### {[4-(9-Ethyl-2-isopropoxy-9H-purin-6-ylamino)-phenyl]-hydroxy-phosphinoylmethyl}-phospbonic acid

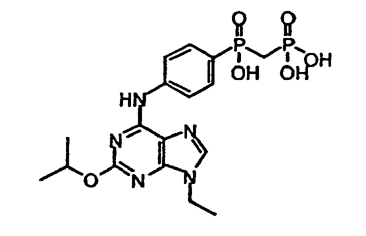

The title compound was synthesized in a manner similar to that described for Example 11. The product was obtained as a white solid: 454 *m*/*z* (M-H)

### Reference Example 16

### ({4-[9-Ethyl-2-(piperidin-4-ylmethoxy)-9H-purin-6-ylamnino]-phenyl}-hydroxy-phosphinoylmethyl)-phosphonic acid

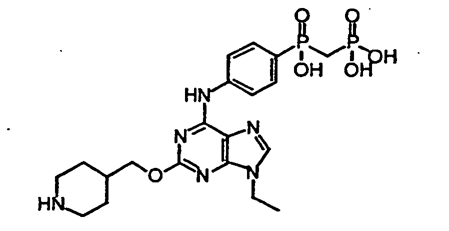

The title compound was synthesized in a manner similar to that described for Example 11. The product was obtained as a white solid: 509 *m*/*z* (M-H)

### Reference Example 17

### [(4-{2-(2-Dimethylamino-propylamino)-9-[2-(3-hydroxy-phenyl)-ethyl]-9H-purin-6-ylamino}-phenyl)-hydroxy-phosphinoylmethyl]-phosphonic acid

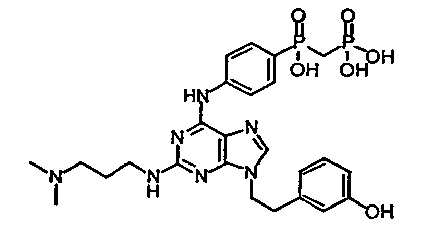

### [(4-{2-(2-Dimethylamino-propylamino)-9-[2-(3-hydroxy-phenyl)-ethyl]-9H-purin-6-ylamino}-phenyl)-hydroxy-phosphinoylmethyl]-phosphonic acid:

A reaction vessel, flushed with N₂, containing a biphasic solution of [(4-{2-Fluoro-9-[2-(3-hydroxy-phenyl)-ethyl]-9H-purin-6-ylamino}-phenyl)-hydroxy-phosphinoyhnethyl]-phosphonic acid (0.2 g, 0.39 mmol), 3-(dimethylamino)propylamine (0.121 g, 1.18 mmol), and N,N-diisopropylethylamine (0.69 mL, 3.94 mmol) in 2.0 mL DMSO was heated at 120 °C for 13 h. Upon removing excess N,N-diisopropylethylamine, under reduced pressure, the slightly brown solution was added ~10 mL of H₂O to produce a milky, off-white solution. Further precipitation occurred upon addition of TFA (final pH = 1-2), at which time the mixture was stirred vigorously for ~20 min, filtered, then washed with acidified H₂O (TFA, pH = 1-2; 4x20 mL), EtOH (2x20 mL), and Et₂O (4x20 mL). The resulting solid was dissolved in ~30 mL of H₂O (adjusted to pH ~9 with 10% NaOH), filtered (0.2 µm, PTFE filter), and purified by RP-HPLC (CH₃CN/H₂O). Lyophilization provided an off-white solid, isolated as its TFA salt (0.082 g): *m*/*z* 588 (M-H).

### Reference Example 18

### ({4-[2-(trans-4-Amino-cyclohexylamino)-9-isopropyl-9H-purin-6-ylamino]-phenyl}-hydroxy-phosphinoylmethyl)-phosphonic acid

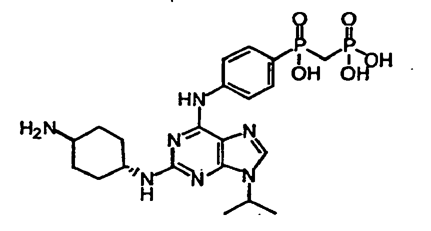

The title compound was synthesized in a manner similar to that described for Example 17. The product was obtained as a white solid: 522 *m*/*z* (M-H)

### Reference Example 19

### [(4-{2-Ethylamino-9-[2-(3-hydroxy-phenyl)-ethyl]-9H-purin-6-ylamino}-phenyl)-hydroxy-phosphinoylmethyl]-phosphonic acid

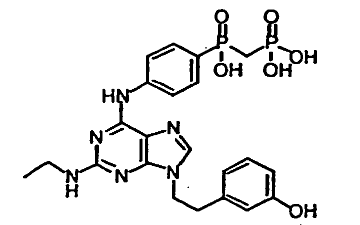

The title compound was synthesized in a manner similar to that described for Example 17. The product was obtained as a white solid: 531 *m*/*z* (M-H)

### Reference Example 20

### [Hydroxy-(4-{9-[2-(3-hydroxy-phenyl)-ethyl]-2-[(tetrahydro-furan-S-2-ylmethyl)-amino]-9H-purin-6-ylamino}-phenyl)-phosphinoylmethyl]-phosphonic acid

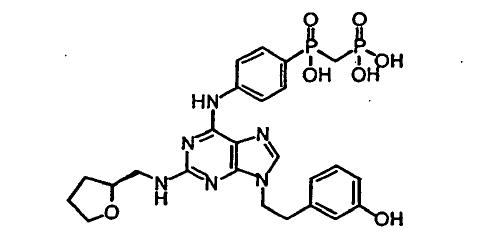

The title compound was synthesized in a manner similar to that described for Example 17. The product was obtained as a white solid: 587 *m*/*z* (M-H)

### Reference Example 21

### [Hydroxy-(4-{9-[2-(3-hydroxy-phenyl)-ethyl]-2-[(tetrahydro-furan-R-2-ylmethyl)-amino]-9H-purin-6-ylamino}-phenyl)-phosphinoylmethyl]-phosphonic acid

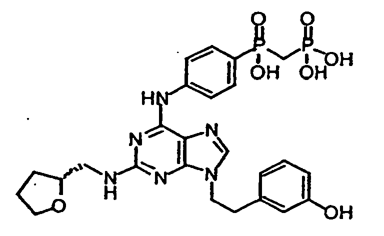

The title compound was synthesized in a manner similar to that described for Example 17. The product was obtained as a white solid: 587 *m*/*z* (M-H)

### Reference Example 22

### ({4-[2-(4-Amino-cyclohexylamino)-9-ethyl-9H-purin-6-ylamino]-phenyl}-hydroxy-phosphinoylmethyl)-phosphonic acid

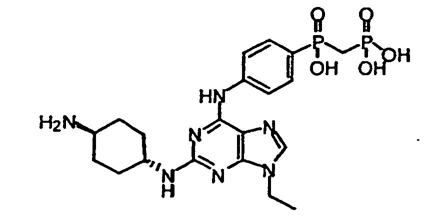

The title compound was synthesized in a manner similar to that described for Example 17. The product was obtained as a white solid: 508 *m*/*z* (M-H)

### Reference Example 23

### [(4-{9-Ethyl-2-[2-(1H-imidazol-4-yl)-ethylamino]-9H-purin-6-ylamino}-phenyl)-hydroxy-phosphinoylmethyl]-phosphonic acid

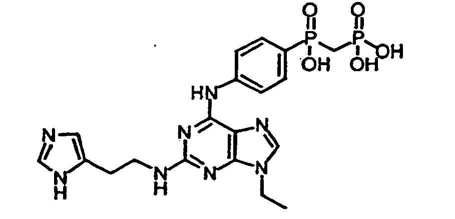

The title compound was synthesized in a manner similar to that described for Example 17. The product was obtained as a white solid: 505 *m*/*z* (M-H)

### Reference Example 24

### [(4-{9-Ethyl-2-[(piperidin-4-ylmethyl)-aminol-9H-purin-6-ylamino}-phenyl)-hydroxy-phosphinoylmethyl]-phosphonic acid

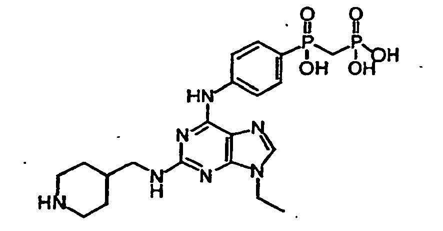

The title compound was synthesized in a manner similar to that described for Example 17. The product was obtained as a white solid: 508 *m*/*z* (M-H)

### Reference Example 25

In another embodiment, compounds as disclosed and described herein can be synthesized by solid-phase parallel synthesis, for example on a Quest 210 synthesizer (Argonaut Technologies); according to the Scheme outlined below:

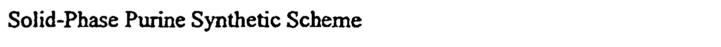

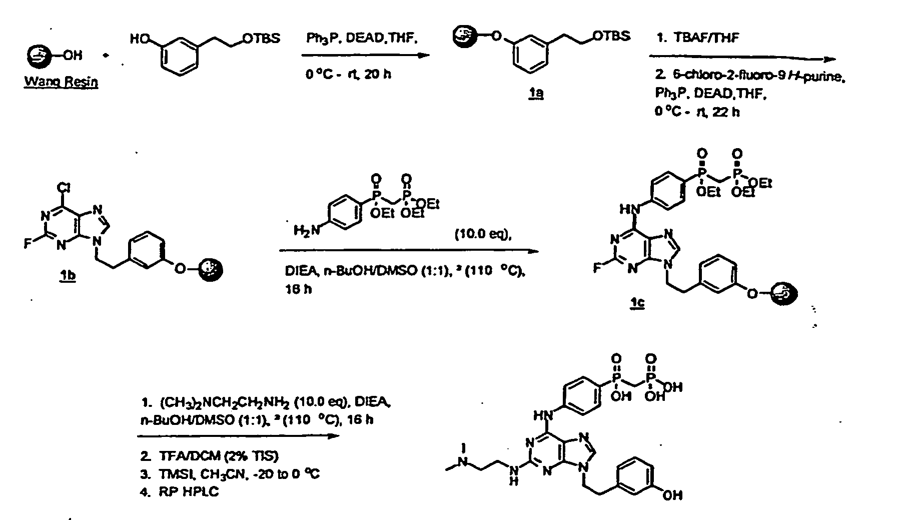

### Reference Example 26

### [(4-{2-(2-Dimethylamino-ethylamino]-9-[2-(3-hydroxy-phenyl)ethyl]-9H-purin-6-ylamino}-phenyl)-hydroxy-phosphinoylmethyl]-phosphonic acid

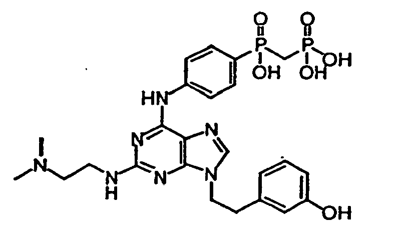

### Preparation of Ether Resin (1a):

To a Teflon® RV (reaction vessel) containing 0.3 g (0.96 mmol/g, 0.29 mmol) of Wang resin was added a solution of 3-[2-(*tert*-Butyl-dimethyl-silanyloxy)-ethyl]-phenol (0.73 g, 2.9 mmol) and triphenylphosphine (0.38 g, 1.44 mmol) in 1.4 mL of THF. The RV was cooled to 0 °C (Julabo chiller) and then added, under an atmosphere of N₂, 2.0 mL (1.44 mmol) of a 0.72 M solution of DEAD (diethyl azodicarboxylate) in THF. The resin mixture was warmed, while agitating, to ambient temperature over 2 h and then agitated for an additional 20 h, upon which the RV was drained and the resin washed successively with THF (5×5.0 mL), DMA (5×5.0 mL), CH₂Cl₂ (5×5.0 mL), Et₂O (2×5.0 mL), CH₂Cl₂ (1×5.0 mL), Et₂O (1×5.0 mL), and CH₂Cl₂ (2×5.0 mL). Excess solvent was removed via N₂ flow overnight to provide the ether resin **1a.** The following analytical data was obtained upon cleavage of **1a** (3-5 mg) with 30% TFA/CH₂Cl₂ (~5 min): 83% HPLC purity; HPLC RT (retention time, min) matches commercially available 3-hydroxyphenethyl alcohol (TBS group removed in TFA cleavage).

### Preparation of Purine Resin (1b):

To the ether resin **1a** (0.29 mmol) was added 6.6 mL (6.57 mmol) of a 1.0 M solution of TBAF (tetrabutylammonium fluoride) in THF. The resin mixture was agitated for 2 h, upon which the RV was drained and the resin washed successively with THF (5×5.0 mL), DMA (5×5.0 mL), CH₂Cl₂ (5×5.0 mL), Et₂O (2×5.0 mL), CH₂Cl₂ (1×5.0 mL), Et₂O (1×5.0 mL), and CH₂Cl₂ (2×5.0 mL). Excess solvent was removed via N₂ flow overnight to provide the deprotected resin. A resin aliquot (3-5 mg) was cleaved with 30% TFA/CH₂Cl₂ (~5 min) to verify resin bound compound integrity: 80% HPLC purity; HPLC RT (retention time, min) matches commercially available 3-hydroxyphenethyl alcohol.

To the dried resin (0.29 mmol) was added a homogeneous suspension of 6-chloro-2-fluoro-9*H* purine (0.50 g, 2.9 mmol) and triphenylphosphine (0.38 g, 1.44 mmol) in 1.75 mL of THF. The RV was cooled to 0 °C (Julabo chiller) and then added, under an atmosphere of N₂, 2.0 mL (1.44 mmol) of a 0.72 M solution of DEAD (diethyl azodicarboxylate) in THF. The resin mixture was warmed, while agitating, to ambient temperature over 1.5 h and then agitated for an additional 22 h, upon which the RV was drained and the resin washed successively with THF (5×5.0 mL), DMA (5×5.0 mL), CH₂Cl₂ (5×5.0 mL), Et₂O (2×5.0 mL), CH₂Cl₂ (1×5.0 mL), Et₂O (1×5.0 mL), and CH₂Cl₂ (2×5.0 mL). Excess solvent was removed via N₂ flow overnight to provide the purine resin **1b.** The following analytical data was obtained upon cleavage of **1b** (3-5 mg) with 30% TFA/CH₂Cl₂ (~5 min): 65% HPLC purity, ~5:1 major/minor peaks (no apparent 3-hydroxyphenethyl alcohol HPLC peak).

### Preparation of Purine Resin (1c):

To the purine resin **1b** (0.29 mmol) was added a solution of [(4-Amino-phenyl)-ethoxy-phosphinoylmethyl]-phosphonic acid diethyl ester (0.97 g, 2.88 mmol) and N,N-diisopropylethylamine (0.25 mL, 1.44 mmol) in 3.0 mL of 1:1 n-butanol/DMSO. The sealed RV was heated at 110 °C for 16 h, upon which the RV was cooled to ambient temperature, drained, and the resin washed successively with DMA (5×5.0 mL), CH₂Cl₂ (5×5.0 mL), Et₂O (2×5.0 mL), CH₂Cl₂ (1×5.0 mL), Et₂O (1×5.0 mL), and CH₂Cl₂ (2×5.0 mL). Excess solvent was removed via N₂ flow overnight to provide the aminated purine resin **1c**. The following analytical data was obtained upon cleavage of **1c** (3-5 mg) with 30% TFA/CH₂Cl₂ (~5 min): *m*/*z* 592 (M+H).

### [(4-{2-(2-Dimethylamino-ethylamino)-9-[2-(3-hydroxy-phenyl)-ethyl]-9H-purin-6-ylamino}-phenyl)-hydroxy-phosphinoylmethyl]-phosphonic acid:

To the aminated purine resin **1c** (0.29 mmol) was added a solution of N,N-dimethylethylenediamine (0.25 g, 2.88 mmol) and N,N-diisopropylethylamine (0.25 mL, 1.44 mmol) in 3.0 mL of 1:1 n-butanol/DMSO. The sealed RV was heated at 110 °C for 16 h, upon which the heat was turned off, the RV drained immediately, and the resin washed (while still hot) successively with DMA (5×5.0 mL), CH₂Cl₂ (5×5.0 mL, at ambient temperature), Et₂O (2×5.0 mL), CH₂Cl₂ (1×5.0 mL), Et₂O (1×5.0 mL), and CH₂Cl₂ (2×5.0 mL). Excess solvent was removed via N₂ flow overnight to provide the bis-aminated purine resin.
To the bis-aminated purine resin (0.29 mmol) was added 5.6 mL of 30% TFA/CH₂Cl₂ (2 % triisopropyl silane). The resin mixture was agitated for 1 h, upon which the filtrate was collected and the resin washed with CH₂Cl₂ (3x5.0 mL). The combined filtrates were concentrated (Savant speed-vac), added 3-4 mL CH₂Cl₂, then reconcentrated to provide a dark yellow oil.
The oil was dissolved in 6.6 mL of CH₃CN, cooled to 0 °C, then added 1.0 mL (7.2 mmol) of TMSI (iodotrimethylsilane). The resulting yellow solution (some precipitate) was stored at -20 °C for 2 h (periodic swirling), then 0 °C for 1 h, upon which 0.4 mL (2.81 mmol) of TMSI was added and reaction continued at 0 °C for 3 h. The excess TMSI was quenched at 0 °C with ~4 mL of 20% aqueous NaHSO₃, the pH adjusted to 11-12 with 10% NaOH, and the CH₃CN removed by rotary evaporation. The pH was re-adjusted to 10-11 with TFA, upon which the solution was filtered (0.2 µm, PTFE filter) and purified by RP-HPLC (CH₃CN/H₂O). Lyophilization provided a white solid, isolated as its TFA salt (0.035 g): ¹H NMR (300 MHz, DMSO-*d*₆) δ 9.76 (s, 1H), 9.33 (br s, 1H), 8.07-7.65 (m, 5H), 7.08-6.59 (m, 5H), 4.27 (m, 2H), 3.66 (m, 2H), 3.32 (m, 2H), 3.04 (m, 2H), 2.85 (s, 6H), 2.37 (m, 2H); *m*/*z* 576 (M+H).

### Reference Example 27

### [(4-{2-(trans-4-Amino-cyclohexylamino)-9-[2-(3-hydroxy-phenyl)-ethyl]-9H-purin-6-ylamino}-phenyl)-hydroxy-phosphinoylmethyl]-phosphonic acid

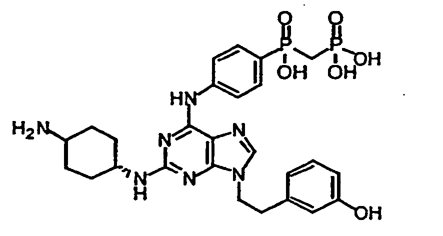

The title compound was synthesized in a manner similar to that described for Example 26. The white solid was isolated as its TFA salt: *m*/*z* 602 (M+H)

### Reference Example 28

### [Hydroxy-(4-{9-[2-(3-hydroxy-phenyl)-ethyl]-2-[2-(3H-imidazol-4-yl)-ethylamino]-9H-purin-6-ylamino}-phenyl)-phosphinoylmethyl]-phosphonic acid

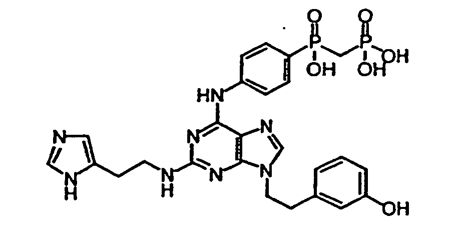

The title compound was synthesized in a manner similar to that described for Example 26. The white solid was isolated as its TFA salt: *m*/*z* 599 (M+H)

### Reference Example 29

### [Hydroxy-(4-{2-(2-hydroxy-ethylamino)-9-[2-(3-hydroxy-phenyl)-ethyl]-9H-purin-6-ylamino}-phenyl)-phosphinoylmethyl]-phosphonic acid

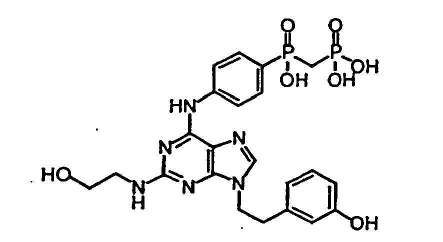

The title compound was synthesized in a manner similar to that described for Example 26. The product was obtained as a white solid: *m*/*z* 549 (M+H)

### Reference Example 30

### (5-{2-(trans-4-Amino-cyclohexylamino)-9-[2-(3-hydroxy-phenyl)-ethyl]-9H-purin-6-ylamino}-2-phosphono-phenyl)-phosphonic acid

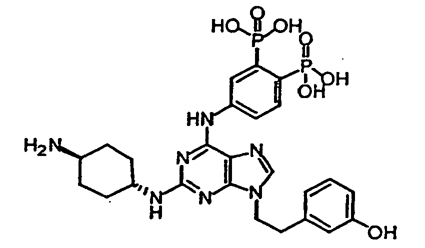

The title compound was synthesized in a manner similar to that described for Example 26. The white solid was isolated as its TFA salt: *m*/*z* 604 (M+H)

### Reference Example 31

### (5-{9-[2-(3-hydroxy-phenyl)-ethyl]-2-[2-(3H-imidazol-4-yl)-ethylamino]-9H-purin-6-ylamino}-2-phosphono-phenyl)-phosphonic acid

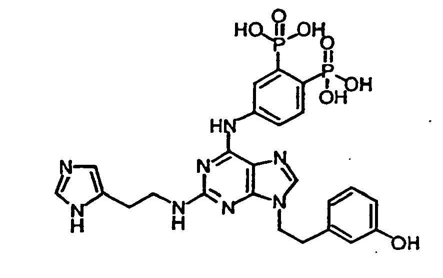

The title compound was synthesized in a manner similar to that described for Example 26. The white solid was isolated as its TFA salt: *m*/*z* 601 (M+H)

### Reference Example 32

### (5-{2-(2-Dimethylamino-ethylamino)-9-[2-(3-hydroxy-phenyl)-ethyl]-9H -purin-6-ylamino}-2-phosphono-phenyl)-phosphonic acid

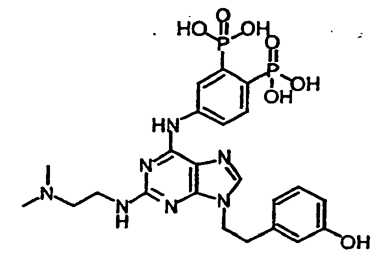

The title compound was synthesized in a manner similar to that described for Example 26. The white solid was isolated as its TFA salt: *m*/*z* 578 (M+H)

### Example 33

### [Hydroxy-(3-{9-[2-(4-hydroxy-phenyl)-ethyl]-6-phenylamino-9H-purin-2-ylamino}-propyl)-phosphinoylmethyl]-phosphonic acid

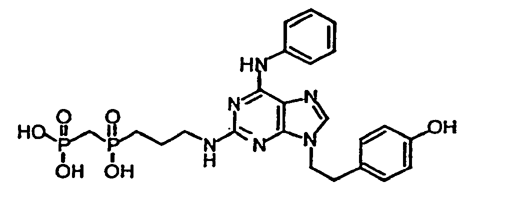

The title compound was synthesized in a manner similar to that described for Example 26. The product was obtained as a white solid: *m*/*z* 547 (M+H)

### Example 34

### [Hydroxy-(3-{9-[2-(3-hydroxy-phenyl)-ethyl]-6-phenylamino-9H-purin-2-ylamino}-propyl)-phosphinoylmethyl]-phosphonic acid

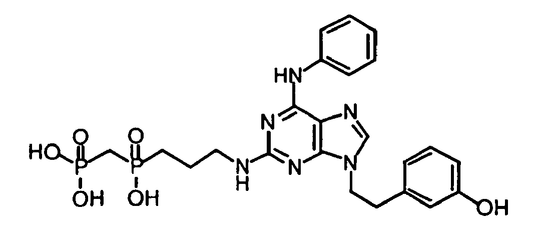

The title compound was synthesized in a manner similar to that described for Example 26. The product was obtained as a white solid: *m*/*z* 547 (M+H)

### Example 35

### (Hydroxy-{3-[9-(3-hydroxy-benzyl)-6-phenylamino-9H-purin-2-ylamino]-propyl}-phosphinoylmethyl)-phosphonic acid

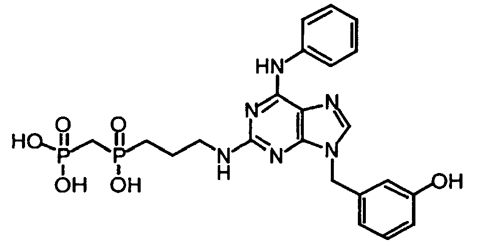

The title compound was synthesized in a manner similar to that described for Example 26. The product was obtained as a white solid: *m*/*z* 533 (M+H)

### Example 36

### ({3-[6-(3-Chloro-phenylamino)-9-(3-hydroxy-benzyl)-9H-purin-2-ylamino]-propyl}-hydroxy-phosphinoylmethyl)-phosphonic acid

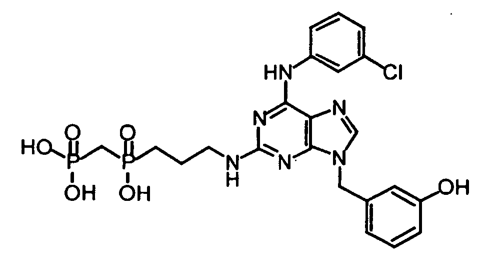

The title compound was synthesized in a manner similar to that described for Example 26. The product was obtained as a white solid: *m*/*z* 567 (M+H)

### Example 37

### [(3-{6-(3-Chloro-phenylamino)-9-[2-(4-hydroxy-phenyl)-ethyl]-9H-purin-2-ylamino}-propyl)-hydroxy-phosphinoylmethyl]-phosphonic acid

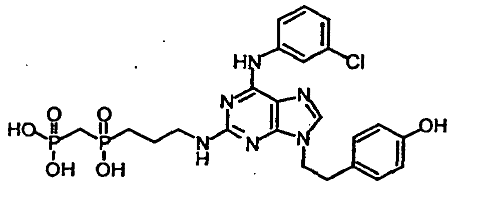

The title compound was synthesized in a manner similar to that described for Example 26. The product was obtained as a white solid: *m*/*z* 581 (M+H)

### Example 38

### [(3-{6-(3-Chloro-phenylamino)-9-[2-(3-hydroxy-phenyl)-ethyl]-9H-purin-2-ylamino}-propyl)-hydroxy-phosphinoylmethyl]-phosphonic acid

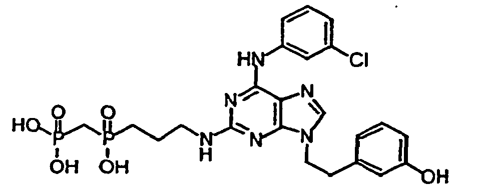

The title compound was synthesized in a manner similar to that described for Example 26. The product was obtained as a white solid: *m*/*z* 581 (M+H)

### Reference Example 39

In another embodiment, compounds as disclosed and herein described can be synthesized according to the Scheme outlined below:

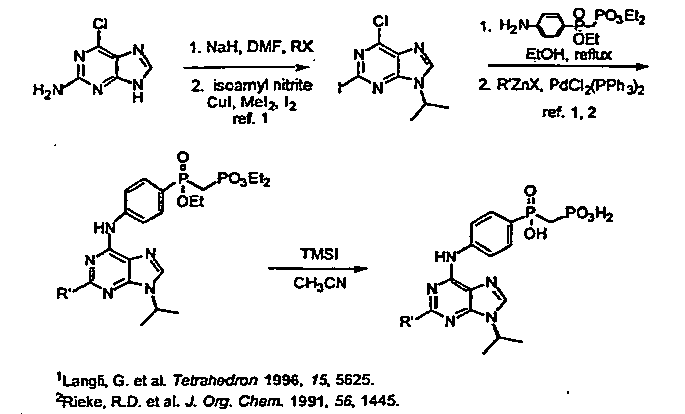

### Reference Example 40

### {[4-(2-Cyclopentyl-9-isopropyl-9H-purin-6-ylamino)-phenyl]-hydroxy-phosphinoylmethyl}-phosphonic acid

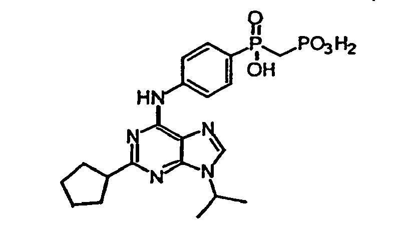

### 6-Chloro-9-isopropyl-9H-purin-2-ylamine:

To 6-chloro-9*H*-purin-2-ylamine (20 g, 0.12 moles) in DMF (200 mL) at -11 °C was added NaH (5.7 g, 0.14 moles, 60%) portionwise over 1 h. The mixture as stirred for 1 h, then 2-iodopropane (14.2 mL, 0.14 moles) was added dropwise. The solution was warmed to rt, stirred for 18 h, quenched with sat'd NH₄Cl, and extracted with EtOAc. The combined extracts, were washed with water, sat'd NaCl, then dried over MgSO₄ and filtered. Concentration yielded an oil which was purified by silica gel chromatography (60% EtOAc/Hexane) to a white solid (13.4 g, 54%): MS [M + H]⁺ 212; m.p. 135-136 °C.

### 6-Chloro-2-iodo-9-isopropyl -9H purine:

6-Chloro-9-isopropyl-9H purin-2-ylamine (2.7 g, 12.8 mmol), CuI (1.2 g, 13.4 mmol), I₂ (3.3 g, 12.8 mmol), isoamyl nitrite (10.6 mL), and CH₂I₂ (5.3 g, 39.3 mmol) in THF (60 mL) were heated at reflux for 75 min. The mixture was cooled to rt, filtered through Celite, and adsorbed onto silica gel. Purification by chromatography (60% EtOAc/hexane) yielded an off-white solid (13.4 g, 54%): MS [M + H]⁺ 323; m.p.104 °C.

### {Ethoxy-[4-(2-iodo-9-isopropyl-9H-purin-6-ylamino)-phenyl]-phosphinoylmethyl}-phosphonic acid diethyl ester:

6-Chloro-2-iodo-9-isopropyl-9*H*-purine (1.7 g, 5.2 mmol), [(4-amino-phenyl)-ethoxy-phosphinoylmethyl]-phosphonic acid diethyl ester (2.6 g, 7.7 mmol), and DIPEA (2.7 mL, 15.5 mmol) were dissolved in EtOH (27 mL) and heated in a sealed tube at 105 °C for 48 h. After cooling, the EtOH was evaporated. The residue was poured into water and extracted with EtOAc. The combined extracts, were washed with water, sat'd NaCl, then dried over MgSO₄ and filtered. Concentration yielded an oil which was purified by silica gel chromatography (11% MeOH/EtOAc) to a glassy solid (1.8 g, 53%): MS [M+H]⁺ 621.

### {[4-(2-Cyclopentyl-9-isopropyl-9H-purin-6-ylamino)-phenyl]-ethoxy-phosphinoylmethyl}-phosphonic acid diethyl ester:

To {ethoxy-[4-(2-iodo-9-isopropyl-9*H*-purin-6-ylamino)-phenyl]-phosphinoylmethyl}-phosphonic acid diethyl ester (0.30 g, 0.48 mmol) in DMF (3. 9 mL) was added PdCl₂(PPh₃)₄ (17 mg, 0.024 mmol) followed by cyclopentylzinc bromide (0.5 M THF, 3.9 mL, 1.9 mmol). The mixture was stirred at rt for 1h, poured into water and extracted with EtOAc. The combined extracts, were washed with water, sat'd NaCl, then dried over MgSO₄ and filtered. The material was used without purification in the next reaction.

### {[4-(2-Cyclopentyl-9-isopropyl-9H-purin-6-ylamino)-phenyl]-hydroxy-phosphinoylmethyl}-phosphonic acid:

To {[4-(2-cyclopentyl-9-isopropyl-9*H*-purin-6-ylamino)-phenyl]-ethoxy-phosphinoylmethyl}-phosphonic acid diethyl ester (0.34 g, 0.6 mmol) in CH₃CN (3 mL) at 0 °C was added TMSI (0.98 mL, 72 mmol). The mixture was stirred for 1 h, quenched with water and Na₂S₂O₅. The pH of the solution was adjusted to 7.5 with the dropwise addition of 2N NaOH and purified by RP HPLC (CH₃CN/H₂O). Lyophylization yielded a white solid: (0.14 g, 59%, two steps): MS [M + H]⁺ 480.

### Reference Example 41

In yet another embodiment, compounds as disclosed and described herein can be synthesized according to the scheme outlined below:

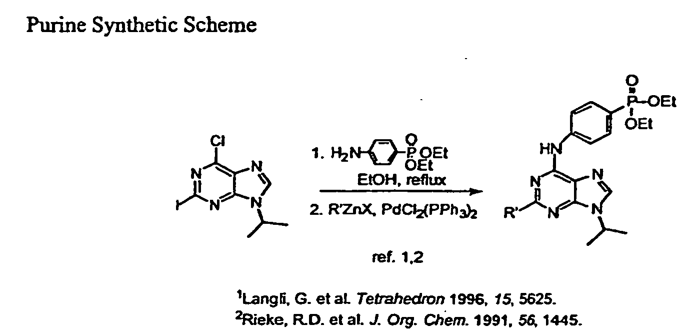

### Reference Example 42

### [4-(2-Cyclopentyl-9-isoprooyl-9H-purin-6-ylamino)-phenyl]-phosphonic acid diethyl ester

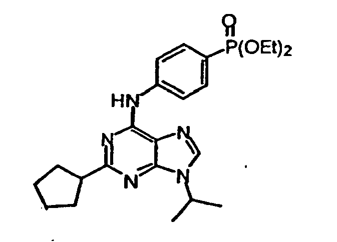

### (4-Amino-phenyl)-phosphonic acid diethyl ester:

(4-Nitro-phenyl)-phosphonic acid diethyl ester (7.6 g, 29.1 mmol) and SnCl₂ (29.6 g, 0.13 mmol) were heated at reflux for 1h. The mixture was poured in CH₂Cl₂ (500 mL) and adjusted to pH 8 with sat'd Na₂CO₃. The resulting mixture was filtered through Celite (CH₂Cl₂ wash) and the layers separated. The aqueous layer was extracted with CH₂Cl₂ and the combined extracts, were washed with water, sat'd NaCl, then dried over MgSO₄ and filtered. Concentration yielded a light yellow solid (5.9 g, 88%): MS [M + H]⁺ 230; m.p. 117-118 °C.

### [4-(2-Iodo-9-isopropyl-9H-purin-6-ylamino)-phenyl]-phosphonic acid diethyl ester:

Identical to that from Example 40 except for the substrate. Glassy solid: (0.95 g, 60%): MS [M + H]⁺ 516.

### [4-(2-Cyclopentyl-9-isopropyl-9H-purin-6-ylamino)-phenyl]-phosphonic acid diethyl ester:

Identical to that from Example 40 except for the substrate. Purified by RP HPLC (CH₃CN/H₂O). Lyophylization yielded a white solid: (45 mg, 63%): MS [M + H]⁺ 458.

The additional described phosphorus-containing moieties can be synthesized according to the schemes outlined below:

### Example 43

### [4-Aminomethyl-2-(diethoxy-phosphoryl)-phenyl]-phosphonic acid diethyl ester

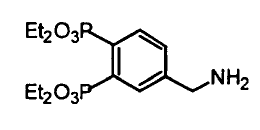

### (3,4-Dihydroxy-benzyl)-carbamic acid tert-butyl ester:

4-Aminomethyl-benzene-1,2-diol hydrobromide (5.6 g, 25.2 mmol) was dissolved in CH₃CN/H₂O 1:1 (100 mL). NaHCO₃ (4.3 g, 50.4 mmol) was added followed by Boc₂O (5.5 g, 25.2 mmol). The mixture was stirred for 18 h, concentrated, and extracted with EtOAc. The combined extracts were washed with water, dried over magnesium sulfate, and concentrated to a tan solid which was used without purification in the next reaction.

### Trifluoro-methanesulfonic acid 5-(tert-butoxycarbonylamino-methyl)-2-trifluoromethanesulfonyloxy-phenyl ester:

(3,4-Dihydroxy-benzyl)-carbamic acid tert-butyl ester (5.5 g, 23.0 mmol), N-phenyltrifluoromethanesulfonimide (26.9 g, 75 mmol), and Et₃N (14.9 mL, 107 mmol) were dissolved in CH₂Cl₂ (80 mL) and stirred for 24 h. The mixture was dumped into water and the layers seperated. The aqueous layer was extracted with methylene chloride. The combined extracts were washed with water, dried over magnesium sulfate, concentrated, and purified by silica gel chromatography (hexane/EtOAc 3:1) to yield the product as a brown oil (9.0 g, 78%).

### [4-(tert-Butoxycarbonylamino-methyl)-2-(diethoxy-phosphoryl)-phenyl]-phosphonic acid diethyl ester:

Trifluoro-methanesulfonic acid 5-(*tert-*butoxycarbonylamino-methyl)-2-trifluoromethanesulfonyloxy-phenyl ester (5 g, 10.o mmol), diethyl phosphite (2.8 mL, 20.3 mmol), N-methylmorpholine (2.7 mL, 25.1 mmol) and tetrakis(triphenylphosphine)-palladium(0) (1.2 g) were dissolved in anhydrous acetonitrile (100 mL) and heated in a sealed tube at 90 °C for 48 h. After cooling, the mixture was diluted with EtOAc (200 mL) and washed with water, 1 N HCl and brine. The organic layer was dried over magnesium sulfate, concentrated, and purified by silica gel chromatography (5% MeOH/CHCl₃) to yield the product as a colorless oil (2.0 g, 42%). ¹H NMR (300 Mhz, CDCl₃) δ 1.35 (m, 12 H), 3.79 (bs, 2 H), 3.96 (m, 8H), 7.45 (m, 1H), 7.91 (m, 2H).

### [4-Aminomethyl-2-(diethoxy-phosphoryl)-phenyl]-phosphonic acid diethyl ester:

[4-(*tert*-Butoxycarbonylamino-methyl)-2-(diethoxy-phosphoryl)-phenyl]-phosphonic acid diethyl ester (2.0 g, 4.2 mmol) was dissolved in TFA/CH₂Cl₂ (25%, 20 mL) and stirred for 3 h. The mixture was evaporated under a stream of N₂, dissolved in EtOAc and washed with sat'd NaHCO₃. The organic layer was dried over magnesium sulfate, and concentrated to a brown oil (0.9 g, 2.3 mmol) which was used without purification in the next reaction.

### Example 44:

### 1-(Bis-diethylphosphonomethylene)-4-aminobenzene

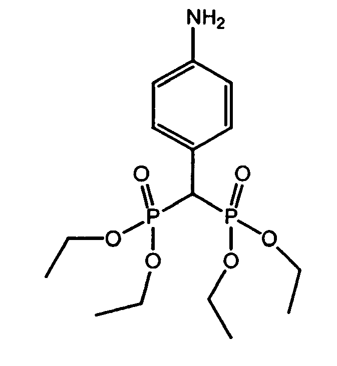

### Diethyl (4-(N-trifluoroacetyl amino benzyl) phosphonate

To diethyl(4-aminobenzyl)phosphonate (10 g) in anhydrous dichloromethane (100 mL) was added pyridine (4.0 mL)followed by trifluoroacetic anhydride (7.0 mL) and stirred at rt overnight (~18 hrs). Reaction mixture was washed carefully with a saturated solution of aqueous sodium bicarbonate (10 mL), brine (10 mL) and the dichloromethane was dried (Na₂SO₄) to afford product (93%). MS: 338 (M-1).

### Diethyl (4-(N-trifuoroacetylamino)-α-bromobenzyl) phosphonate

To the diethyl(4-(N-trifluoroacetyl amino benzyl) phosphonate (9.75 g, 41.13 mmols) in anhydrous carbon tetrachloride (100 mL) was added NBS (1.6 g , 41.13 mmols) and heated to reflux under intense visible light with stirring under which time a white precipitate formed. The reaction was cooled to rt and then filtered. The filtrate was half concentrated then left in the freezer overnight to crystallize. The crystalline product was seperated by filtration (7.2 g, 60%). MS : 416, 418 (M-1 Br⁷⁹Br⁸⁰).

### 1-(Bis-diethylphosphonomethylene-4-(N-trifluoroacetylamino)benzene

To diethyl(4-(N-trifuoroacetylamino)-α-bromobenzyl) phosphonate (7.2 g, 17.22 mmols) in anhydrous THF (50 mL) was added triethylphosphite (0.82 mL, 17mmol) and stirred under reflux for 4 h. Reaction mixture was cooled to rt and concentrated. Residue was taken up in boiling ethyl ether and cooled to rt. Solid was filtered off to afford 6.0 g product (73%). Pale pink solid. MS: 476 (M+H), 475 (M-H).

### 1-(Bis-diethylphosphonomethylene)-4-aminobenzene

The above 1-(bis-diethylphosphonomethylene-4-(N-trifluoroacetylamino)benzene (1.2 g, 2.52 mmols) in NaOH solution (0.1 N, 10 mL) was heated to 60 °C for 5 h. The reaction mixture was cooled to rt, and extracted with methylene chloride. Combined organic layers were dried over sodium sulfate and concentrated to afford the product (0.85 g, 97%). MS: 412 (M+23).

### Example 45:

### G. Bis-3,4-(diethylphophonyl)-β-phenylethyl amine 5

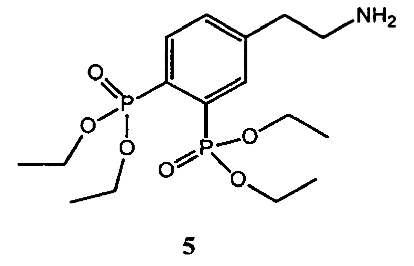

### N-t-butoxycarbonyl-3-hydroxytyramine

To a solution of 3-hydroxytyramine hydrochloride (5.0 g, 26.36 mmol) in dixane/water (50/30 mL) at 0 °C was added sodium bicarbonate (6.64g, 79.08 mmol) and stirred for 10 min. To this was added Boc anhydride (7.48 g, 34.275 mmol) and stirred at rt for 18 h. After removing dioxane in vacuo, the slurry was taken up in water (~60 mL) and extracted in ethyl acetate (25 mL X 3). The organics were washed with 1N HCl (10 mL X 2) followed by brine (10mL); dried (sodium sulfate) and concentrated which when cooled in the refregerator crystallized the next day (3.87 g, 57%). MS: 252 (M-H).

### N-t-butoxycarbonyl-bis-3,4-O-triflyl-β-phenylethyl amine

To a solution of N-Boc-3-hydroxytyramine (3.87 g, 15.28 mmol) in anhydrous dichloromethane (70 mL) was added triethyl amine (61.12 mmol) followed by N-phenyl triflamide (16.37 g, 45.84 mmol) and stirred at rt for 24 h. Reaction mixture was diluted with dichloromethane (100 mL) and washed successively with 1N HCl (3X 10 mL) and brine (10 mL) and dried (sodium sulfate). After concentration of dichloromethane extract the brown oil was chromatographed on silicagel using hexane /ethyl acetate (10-100%) to give product as a viscous oil (6.32 g, 80%). MS: 516 (M-H).

### N-t-butoxycarbonyl-bis-3,4-(diethylphophonyl)-β-pherrylethyl amine

To the N-t-butoxycarbonyl-bis-3,4-0-triflyl-β-phenytethyl amine (6.32 g, 12.21 mmol) in acetonitrile in an atmosphere of argon was carefully added diethyl phosphite (3.46 mL, 26.87 mmol), N-methylmorpholine (3.09 mL, 30.54 mmol), tetrakistriphenylphosphine palladium(0) (1.41 g, 1.221 mmol) and after flushing the solution with argon for 10 min. it was stoppered and heated to 90 °C for 2 days. Acetonitrile was concentrated, and the residue was diluted with ethyl acetate. The organic layer was washed with citric acid (10%, 10 mL X2), brine (10 mL) and dried (sodium sulfate). The yellow gum after concentration of ethyl acetate was purified by flash column chromatography on silica gel using ethyl acetate in hexane (33%-100%) followed by ethyl acetate/methanol (9/1) to give a pale yellow gum (992 mg, 16.5%). MS: 492(M-H).

### Bis-3,4-(diethylphophonyl)-β-phenylethyl amine

To the N-t-butoxycarbonyl-bis-3,4-(diethylphophonyl)-β-phenylethyl amine (0.992 g, 2.01 mmol) in dichloromethane (10 mL) was added TFA (25% in dichloromethane, 2.5 mL). After 1.5 h the solvents were removed in vacuo and the residue was diluted with saturated sodium bicarbonate and dichloromethane (5 mL and 50 mL). The aqueous layer was re extracted with dichloromethane (25 mL X 2). Combined organics were concentrated to give a pale brown gum (0.758 g, 96%) which was pure enough for the next step. MS:392 (M-H), 416 (M+23).

### Reference Example 46: Synthesis of exemplary alkylpurine compounds:

It will be appreciated that in certain exemplary embodiments, purine derivatives in which R_{C} represents an alkyl moiety can be prepared according to the general methodology described in the Scheme depicted below.

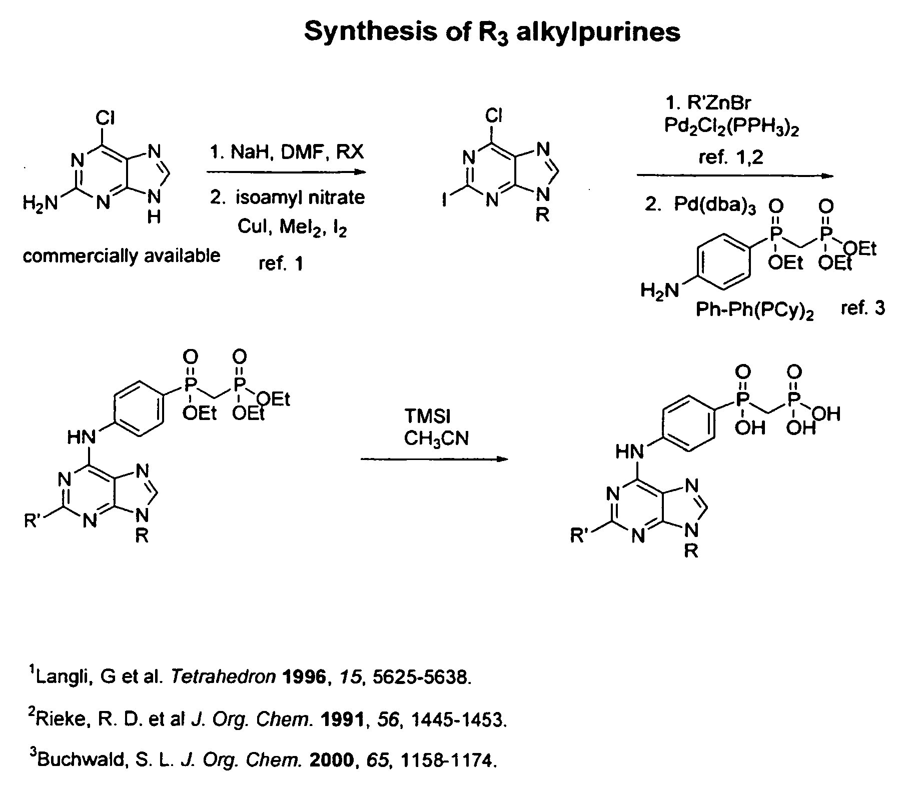

### Example 47: Compounds

Certain inventive compounds as detailed herein are shown as follows. However, it will be appreciated that the present invention is not intended to be limited to the particular compounds depicted below. It will be appreciated that a variety of compounds can be synthesized using the techniques as described herein. Certain of those compounds are depicted below; however the present invention is not intended to be limited by the descrption these compounds.

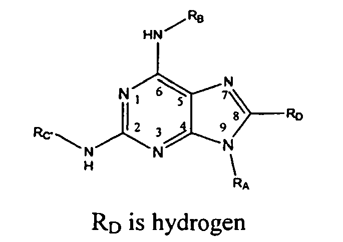

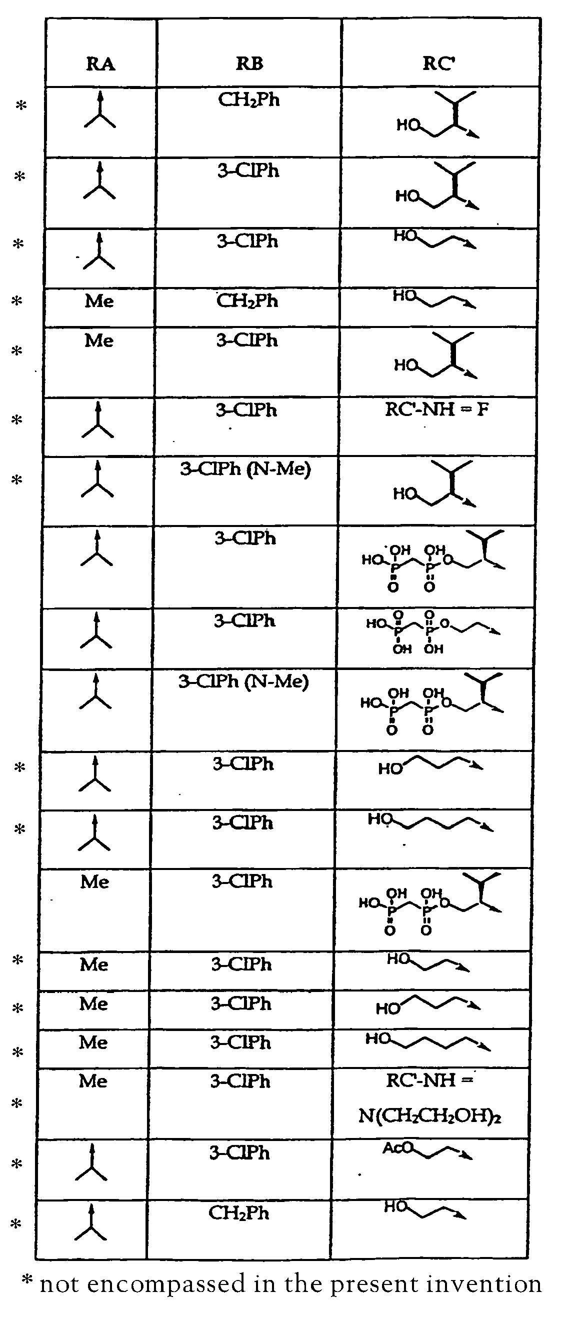

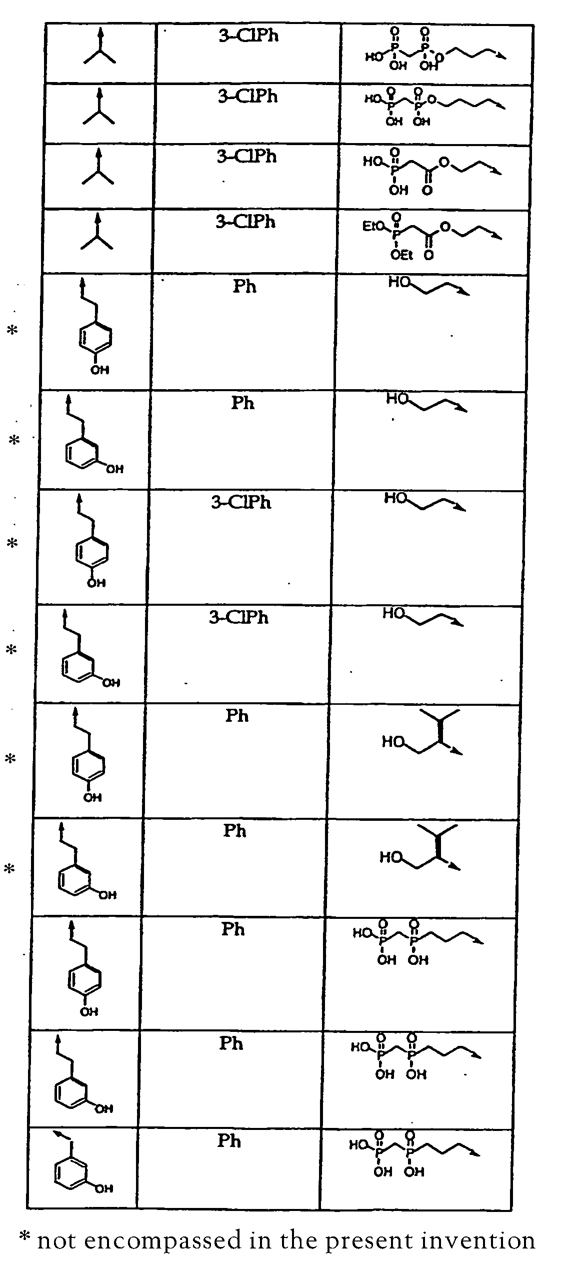

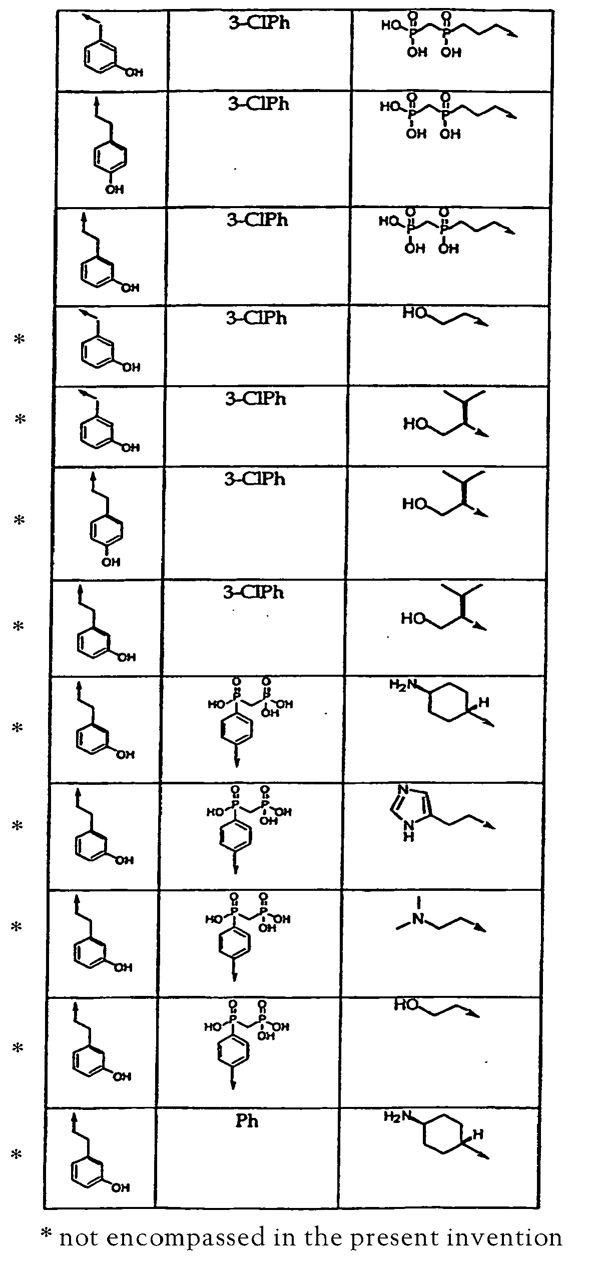

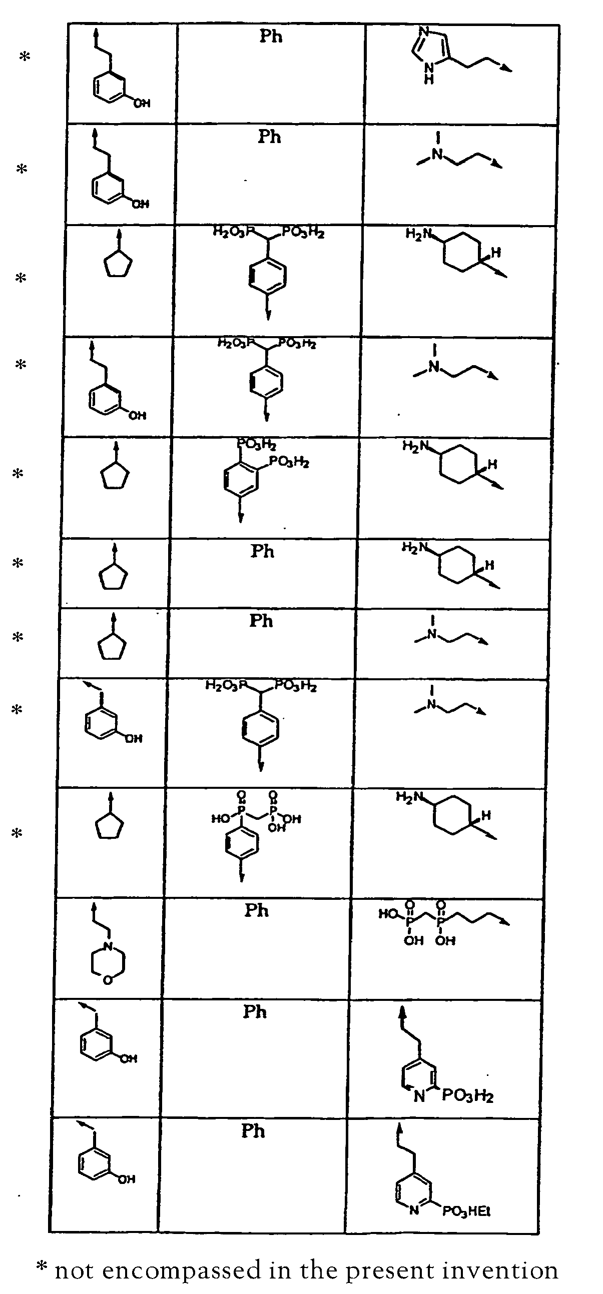

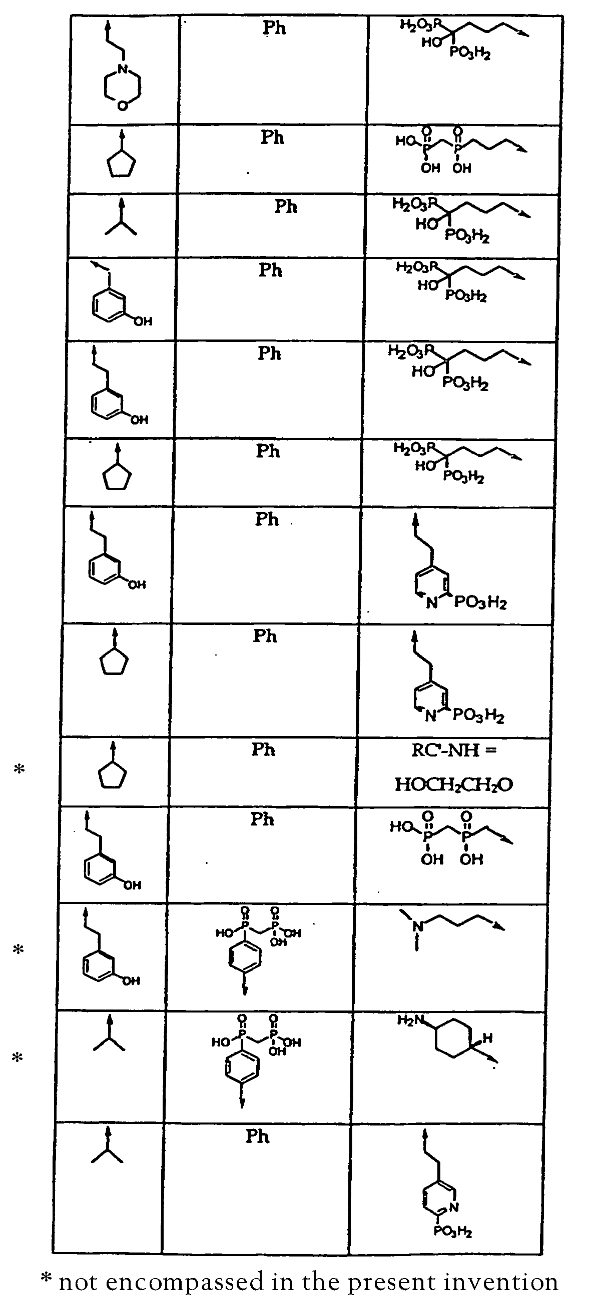

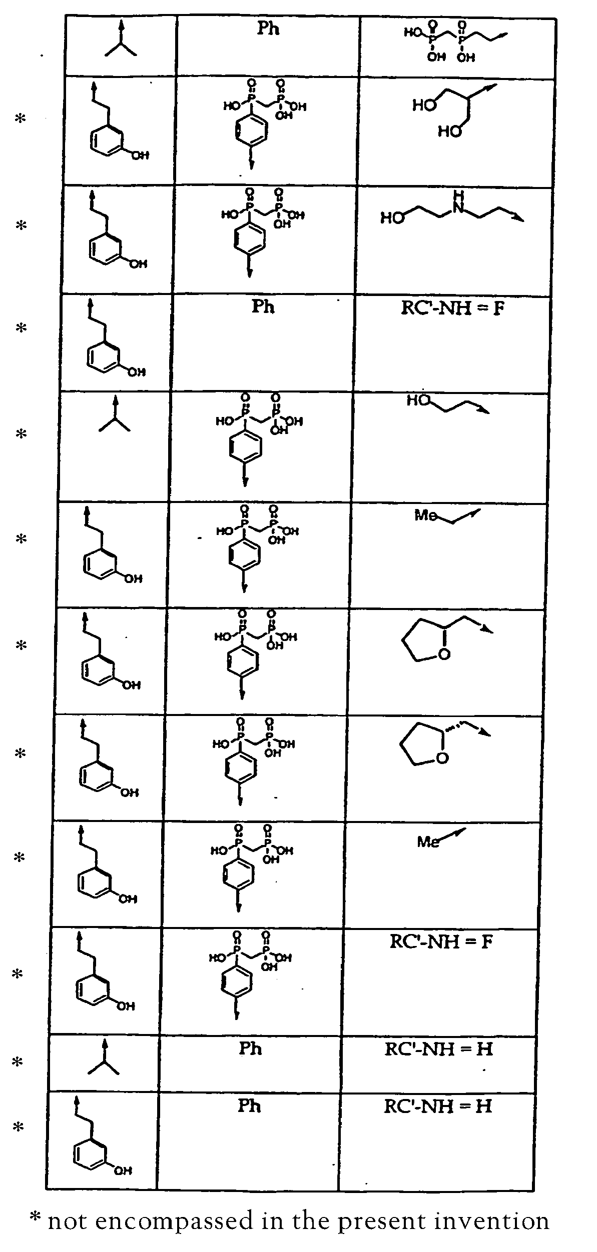

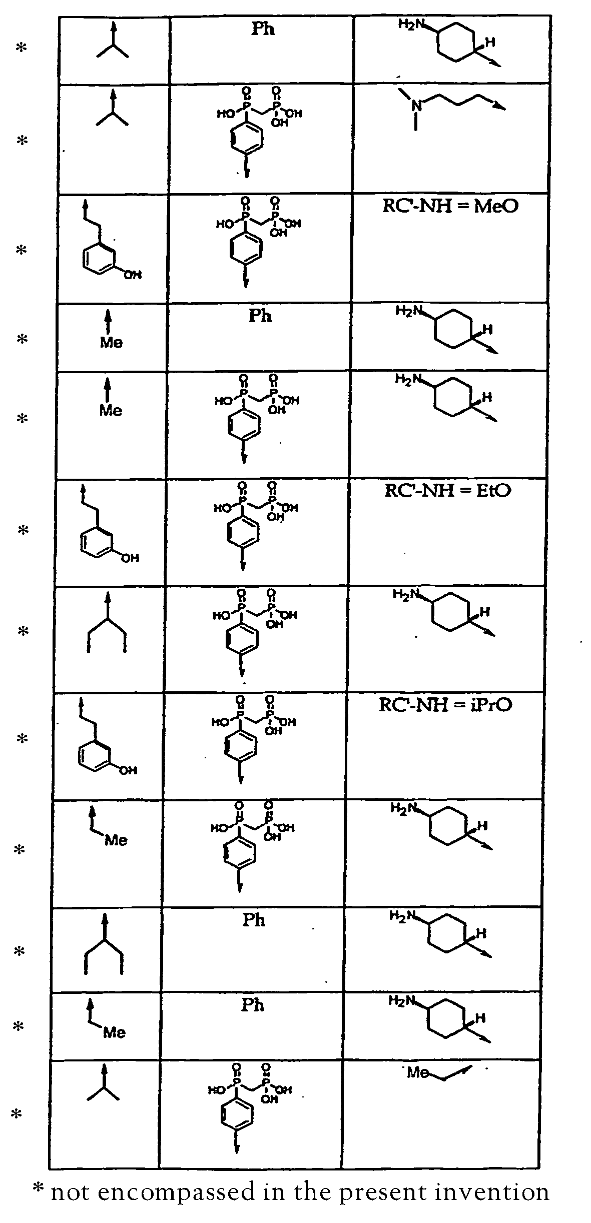

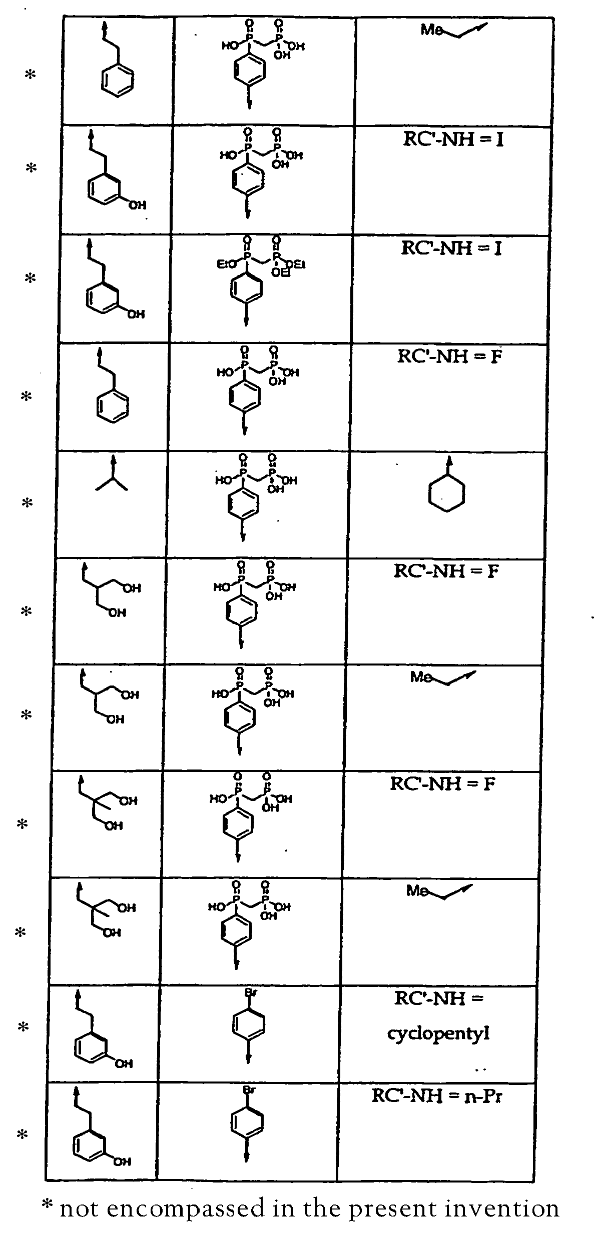

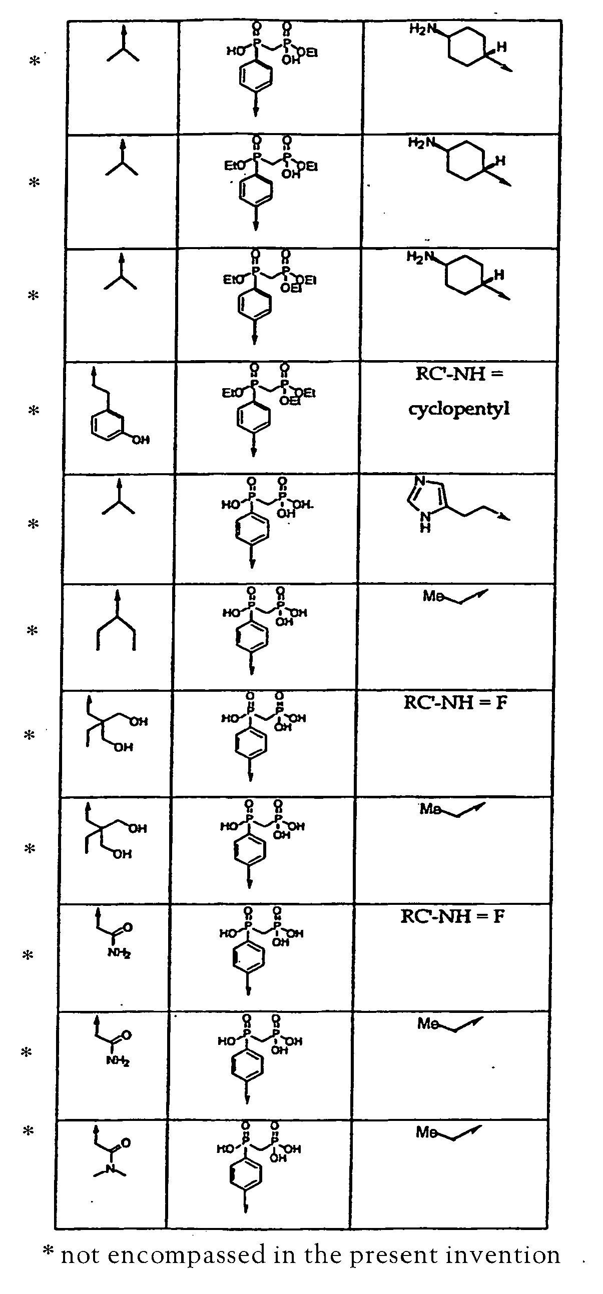

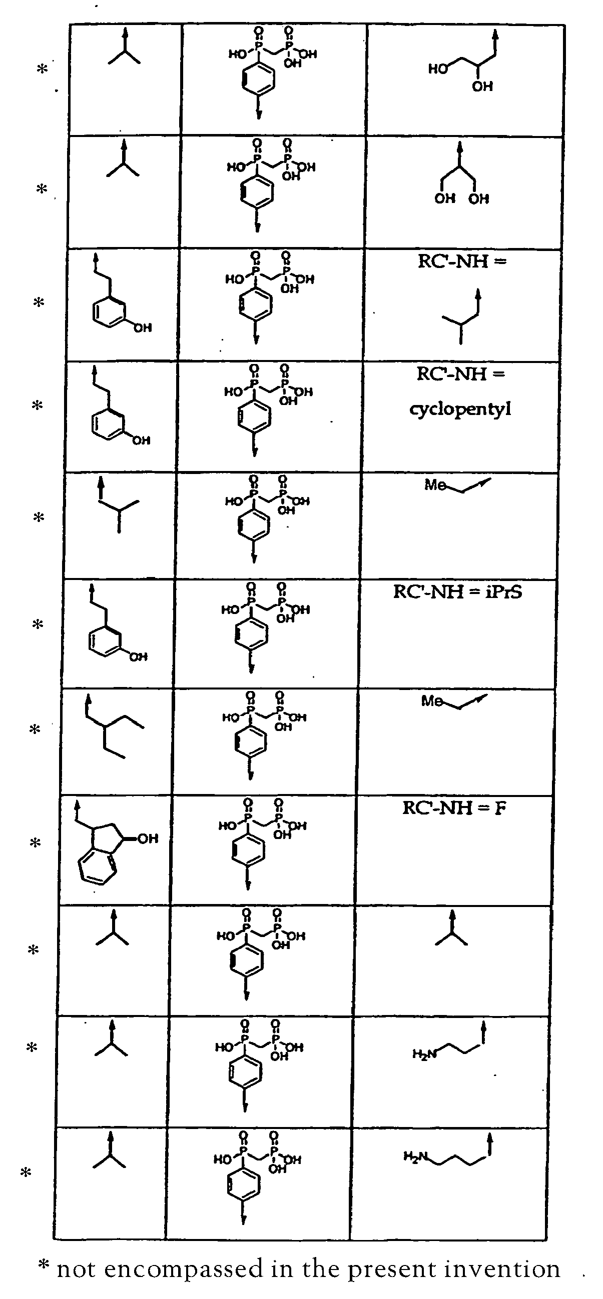

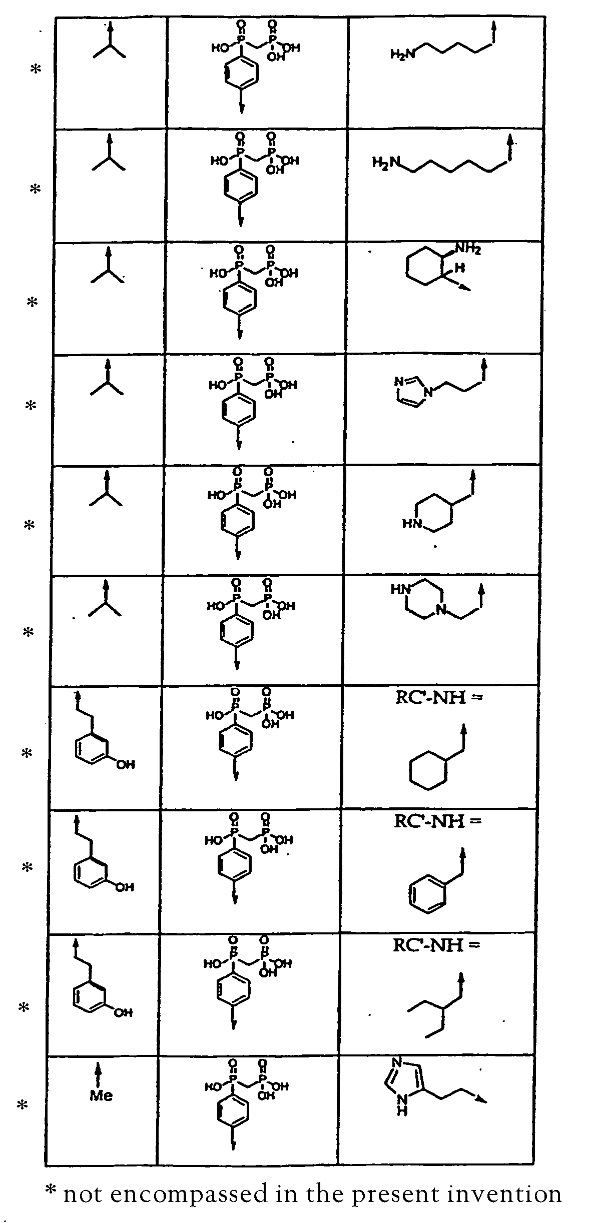

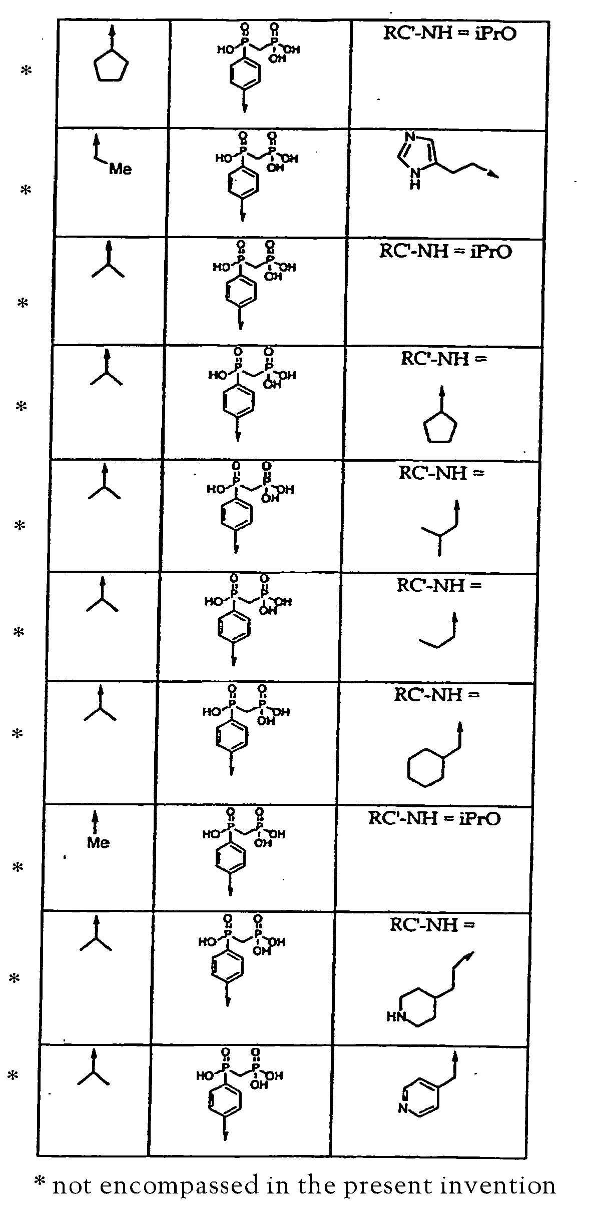

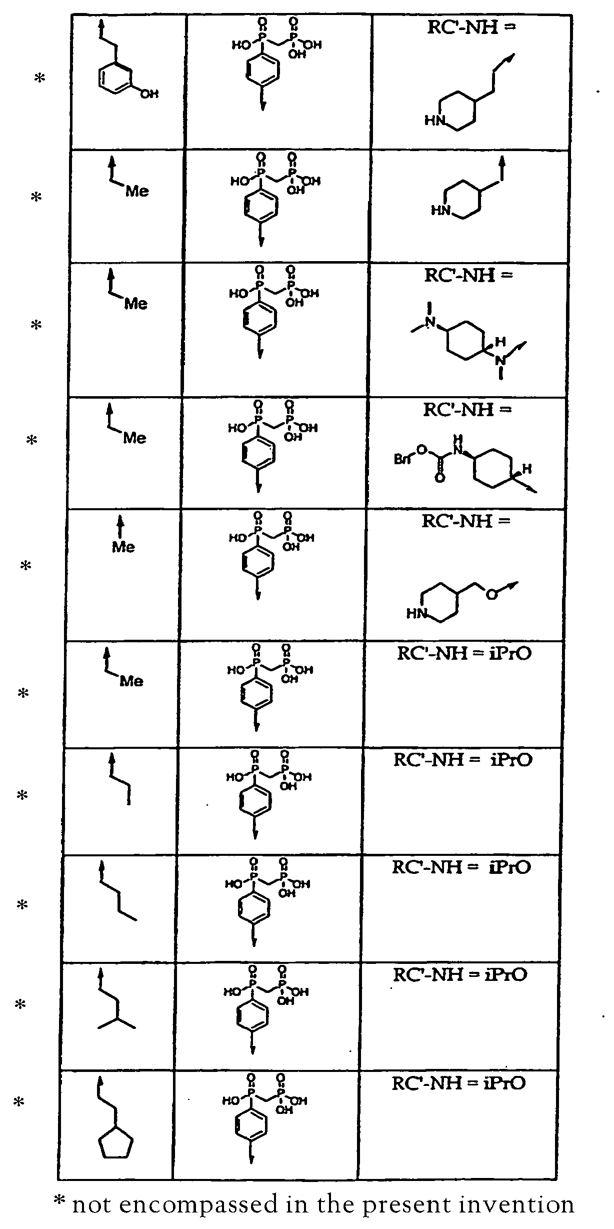

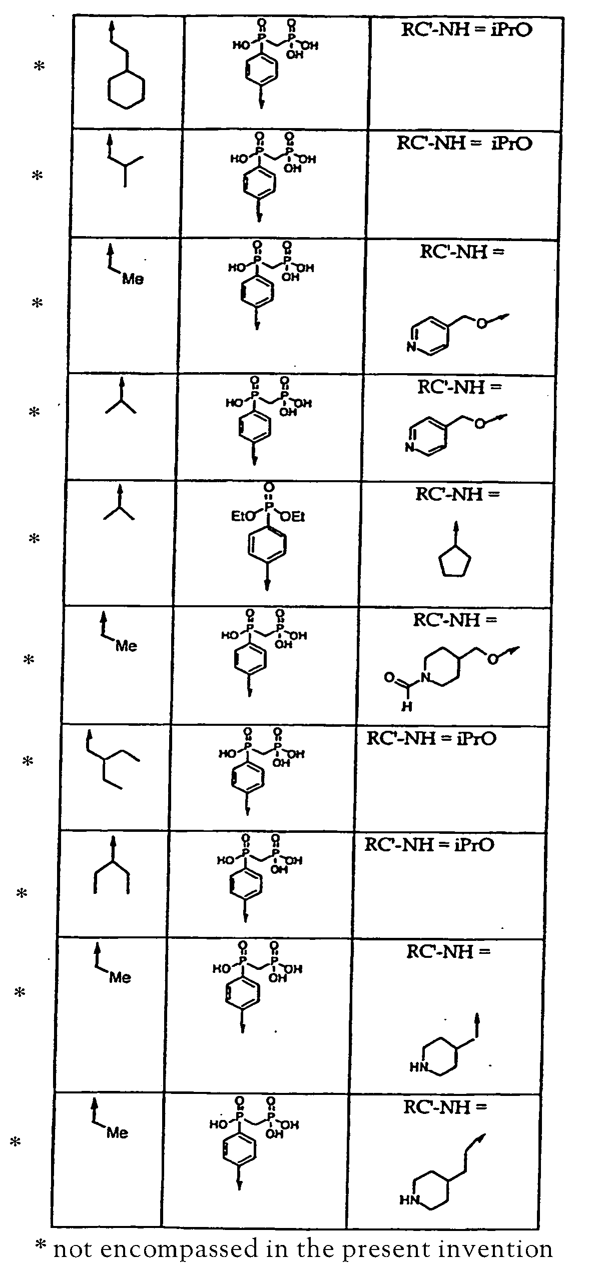

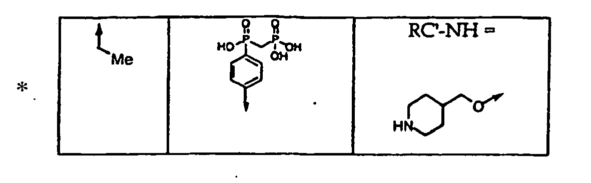

Certain other exemplary compounds as depicted below can be synthesized in a manner similar to that exemplified herein:

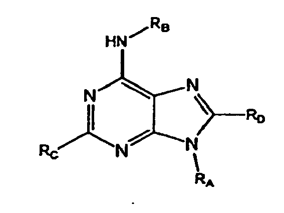

### R_{D} is hydrogen

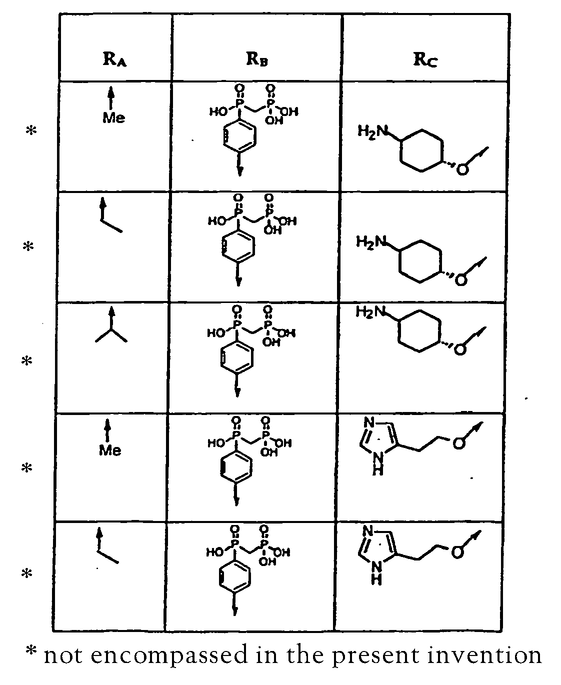

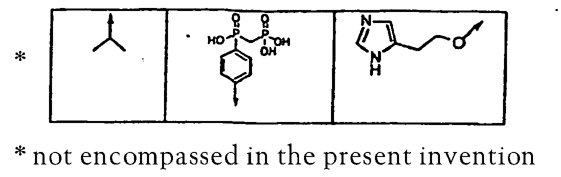

It will also be appreciated that, in addition to the compounds listed above, the present invention also encompasses those compounds in which R_{A} is a lower alkyl or is a branched aliphatic or heteroaliphatic moiety. In certain embodiments, R_{A} is methyl or ethyl, and in certain other embodiments, R_{A} is isopropyl.

### Example 48: In vitro and In vivo assays and exemplary biological data:

Compounds of the present invention may be evaluated in a variety of assays to determine or characterize their biological activities. For example, the compounds of the invention can be tested for their ability to inhibit Src kinase or other protein kinases, to bind to bone, to inhibit bone resorption or to otherwise improve the relative dynamics of bone homeostasis. The compounds can also be evaluated for their cytotoxic and growth inhibitory effects on tumor cells of interest..

### A. Anti-Resorption Cell Assay (Rabbit Osteoclast):

Femurs, tibias, and scapulas were isolated from 3-4 day old New Zealand white rabbits (Millbrook Farms, Amherst, MA). Bones were chopped and minced in a-MEM (Gibco-BRL) containing 0.55 g/L NaHCO₃, 10 mM HEPES (Gibco-BRL), 50 units/ml penicillin, and 0.05 mg/ml streptomycin, pH 7.1. Bone fragments were allowed to settle by gravitation, supernatant was collected and centrifuged at 400 RPM (Beckman GS-6KR) for two minutes, and the cell pellet was resuspended in the same medium supplemented with 10% HIFBS (Hyclone). For prebinding experiments, 0.75 ml of cell suspension was added to wells containing sperm whale dentine discs preincubated for 2 hours with 0.75 ml culture medium containing a 2X concentration of test compound. Alternatively, 0.75 ml of cell suspension was added to each well containing dentine slices preincubated with 0.75 ml culture medium alone and test compound was added after the adhesion phase. Sperm whale dentine was cut as 1 mm x 6 mm circular discs. The adhesion phase was carried out for 30 minutes at 37 °C and 5% CO₂ and then the medium and non-adherent cells and debris were removed by aspiration. Fresh culture medium containing serially diluted test compounds was added and cells were incubated on dentine for 24 hours at 37 °C and 5% CO₂. After the resorption phase, dentine slices were soaked for 30 seconds in 0.5% sodium hypochlorite, wiped clean of adherent cells, and then stained for 30-45 seconds with 1% toluidine blue. Resorption was measured using reflective light microscopy and automated image analysis. The resorbed area was measured on the entire 6 mm disc. Remaining cells in the 24-well plates were stained for tartrate resistant acid phosphatase (TRAP) and also assessed visually for the presence of fibroblasts. Experiments were carried out containing triplicate samples for each concentration of compound tested with five untreated control samples per plate. IC₅₀ values were calculated based on the % resorption in the presence of compound relative to vehicle alone treated control samples. Data were calculated from at least three independent experiments each containing triplicate samples.

Generally speaking, in this assay, IC₅₀ values below about 10 µM are of particular interest, while scores below 500 nM or below are preferred, and scores below about 100 nM are particularly preferred.

Certain embodiments, in which R_{C} is a moiety -NR where R is a lower aliphatic substituted group substituted with (a) a pyridine ring bearing a -PO(OR₁)₂ substituent, or (b) a-CX[PO(YR₁)₂]₂ substituent where X is -OH, -H, or lower alkyl; R_{A} is a cyclic or acyclic aliphatic or heteroaliphatic moiety or is an aryl or alkylaryl moiety optionally substituted by one or more hydroxyl moieties; and R_{B} is a substituted or unsubstituted aryl moiety have led to IC₅₀ values in the range of 4-100 µM. In certain of those embodiments, R₁ is hydrogen and in certain other embodiments, at least one of R₁ is an alkyl moiety.

Evaluation of compounds having the structure: 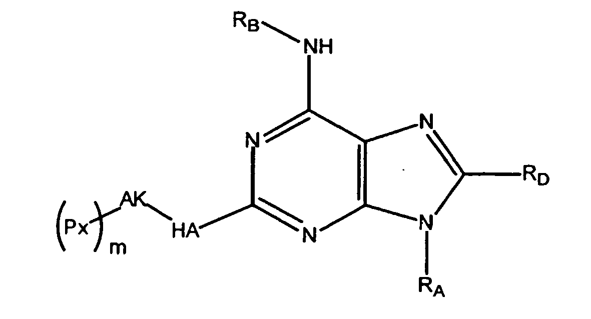 as defined herein, in which Px is a phosphorus moiety of structure i-vii as defined herein has yielded IC₅₀ values in the range of about 1-100 µM, preferably in the range of about 4-100 µM, 4-20 µM, or 0.8-4 µM. For example, compounds having the structure shown above, in which AK is lower aliphatic, and Px is formula i, R_{A} is a phenyl moiety optionally substituted with one or more hydroxyl moieties, and R_{B} is a phenyl moiety optionally substituted with, for example, a halogen, such as chlorine, have shown activities within these ranges.

Evaluation of compounds having the structure: (not encompassed in the present invention) 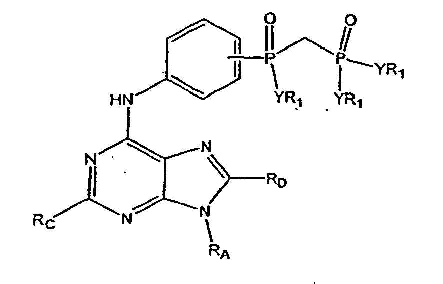 in which R_{A} is a cyclic or acyclic aliphatic or heteroaliphatic moiety or alkylaryl moiety optionally substituted with one or more hydroxyl moieties; R_{C} is an amino moiety substituted with a cyclic or acyclic aliphatic or heteroaliphatic moiety having one or more substituted or unsubstituted amino moieties, or is a hydroxyl, halogen, alkoxy or heterocycle moiety has yielded IC₅₀ values in the range of about 1-100 µM, preferably about 0.8-4.0 µM.

Other Compounds in which R_{B} is a phenyl ring bearing a p-CH [PO(YR₁)₂]₂ substituent or is a moiety of the formula: 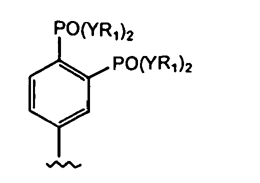 R_{A} is a cyclic or acyclic aliphatic or an alkylaryl moiety optionally substituted with one or more hydroxyl moieties; and R_{C} is an amino moiety substituted with a cyclic or acyclic aliphatic or heteroaliphatic moiety optionally substituted with one or more amino or substituted amino moieties have yielded IC₅₀ values in the range of about 1.0 to about 20 µM, preferably about 0.8 to about 4.0 µM.

### B. Kinase Assays

In addition to their ability to inhibit bone resorption, the compounds of the present invention are also able to inhibit protein kinase activity.

The following Example presents a general method for determining the effect of the inventive compounds on the phosphorylation of a kinase's target.

A purified or partially purified kinase is incubated with a peptide comprising the target sequence of the kinase under conditions suitable for the kinase to phosphorylate its target sequence of amino acids (*i.e.,* protein, peptide). The particular requirements of the kinase may be determined empirically by one of skill in the art, or the conditions that have been published for a particular kinase (for example, see Table I in Boutin "Tyrosine protein kinase assays" *J. Chromatography B* 684:179-199, 1996;) may be used. The extent of phosphorylation of the target peptide is determined in the presence and absence of the inventive compound and may be determined in the presence of varying concentrations of the inventive compound. The phosphorylation rate may be determined by any means known in the art including electrophorectic assays, chromatographic assays, phosphocellulose assays, *etc.*

In an electrophorectic assay, a radiolabled phosphate donor such as ATP or GTP is incubated with the peptide substrate in the presence of a kinase. The phosphorylated substrate versus the phosphate donor (e.g., ATP, GTP) are separated via thin-layer electrophoresis (Hunter *J. Biol. Chem.* 257:4843, 1982).

Any matrix may be used in the electrophoresis step including polyacrylamide, cellulose, *etc.* The extent of phosphorylation may then be determined by autoradiography or scintillation counting.

The labeled phosphate donor may be separated from the phosphorylated amino acid sequence by standard chromatography techniques. Any matrix may be used to effect the separation including ion exchange resins, PEI cellulose, silica gel, *etc.* Standard column chromatography methods may be used, or HPLC methods may be used for faster cleaner separations. The radio-labeled peptides are detected by scintillation counting to determine the phosphorylation rate.

Another method which is historically the most popular is the phosphocellulose paper assay, first described by Witt *et al.* (Witt *et al. Anal. Biochem.* 66:253, 1975; incorporated herein by reference). This method is well adapted to the screening of inhibitors (Traxler *et al. J. Med Chem.* 34:2328, 1991).

Immunological methods may also be used to detect the phosphorylation of a peptide or protein substrate. For example, anti-phosphotyrosine antibodies may be used in the detection or precipitation of phosphorylated amino acid sequences. The method has the advantage of not requiring the used of radio-labeled ATP.

In comparing the rates of phosphorylation in the presence and absence of the test compound, the compound should lead to at least a 25% decrease in the rate of phosphorylation, more preferably at least 50%, and most preferably at least 75%. These decreases are preferably obtained at micromolar concentrations of the compound and more preferably nanomolar concentrations (e.g., less than 100 nM).

In addition, a Quantitative Kinase Activity Assay Using a 96-Well Plate can be determined. The following Example has been adapted from the assay described by Asthagiri *et al.* (*Anal. Biochem.* 269:342-347, 1999). This assay allows high-throughput screening of a large number of potential kinase inhibitors.

The surface of a microtiter plate is coated with antibodies directed against the kinase to be studied. Reacti-Bind protein A-coated wells (Pierce, Rockford, IL) are incubated overnight at 4°C with 50 µL of 10 µg/ml antibody in blocking buffer containing 1% BSA, 50 mM Tris (pH 7.5), 150 mM NaCl, and 0.05% Triton. Wells are then washed three times with blocking buffer. A cell lysate containing the kinase to be studied is diluted in lysis buffer to a total volume of 50 µl incubated for 3 hours at 4 °C to allow the antibody to capture the kinase. To measure background, an extra well is incubated with just lysis buffer and is handled throughout the assay in the same manner as other samples. Each well is then washed twice with 200 µl wash buffer containing 50 mM Tris (pH 7.5) and 150 mM NaCl and twice more with 200 µl kinase wash buffer containing 20 mM Tris (pH 7.5), 15 mM magnesium chloride, 5 mM β-glycerolphosphate (pH 7.3), 1 mM EGTA, 0.2 mM sodium orthovanadate, and 0.2 mM DTT. The contents of the well are then resuspended in 20 µl kinase wash buffer.

To each well is then added 20 µl of 2 mg/ml substrate containing the target amino acid sequence of the kinase. To initiate the *in vitro* reaction, 20 µl kinase assay buffer containing 20 mM Tris (pH 7.5), 15 mM magnesium chloride, 5 mM β-glycerophosphate (pH 7.3), 1 mM EGTA, 0.2 mM sodium orthovanadate, 0.2 mM DTT, 0.4 µM protein kinase A inhibitor peptide (Upstate Biotech, Lake Placid, NY), 4 µM protein kinase C inhibitor peptide (Upstate Biotech), 4 µM calmidazolium (Upstate Biotech), 25 µM ATP, and 6 µCi [?-³²P]-ATP is added to two wells. To one of the wells is added the test compound at a concentration ranging from 1 mM to 1 nM. Reactions contents are maintained under agitation at 37 °C with the Jitterbug (Boekel, Feasterville, PA). After 10 minutes, the reactions are quenched with 60 µl of 75 mM phosphoric acid.

[³²P]-labeled substrate is separated from unreacted [³²P]-ATP by filtering 40 µl of the quenched reaction contents through a phosphocellulose filter using the Millipore Multiscreen system (Millipore, Bedford, MA). Each filter is washed five times with 200 µl 75 mM phosphoric acid and three times with 200 µl 70% ethanol. The filters are allowed to dry before punching out the filters into scintillation vials. ³²P amounts on the filter paper are quantified using CytoScint (ICN Biomedicals, Costa Mesa, CA) scintillation fluid and a RackBeta (Wallac, Gaithersburg, MD) scintillation counter. ³²P measurements are adjusted by subtracting the radioactivity associated with the background sample, and measurements observed in presence and absence of the test compound are compared.

If desired one may use an assay involving immunoprecipitation of the kinase. The following such assay was adapted from the method by Bondzi *et al.* (*Oncogene* 19:5030-5033, 2000).

Cells expressing the kinase of interested are washed once in PBS and lysed in buffer containing 20 mM Tris (pH 7.9), 137 mM NaCl, 5 mM EDTA, 1 mM EGTA, 10 mM NaF, 1 mM sodium pyrophosphate, 100 µM β-glycerophosphate, 10 µg/ml aprotinin, 1 mM PMSF, 10% glycerol, and 1% v/v Triton X-100. The lysate is cleared by centrifugation at 10,000 x g for 10 minutes at 4 °C. Protein concentrations are determined using the BCA method (Pierce, Rockford, IL, USA). Five hundred µg of the lysate protein is then added to 2 µg monoclonal anti-kinase antibody directed against a portion of the protein. Antibodies are prebound to 100 µl of protein A + G-agarose beads (Santa Cruz Biotechnology, Santa Cruz, CA, USA) by incubation for one hour at 4°C on a slow rotator. Increasing amounts of lysate protein (0, 50, 100, 200, 400, 800, and 1600 µg) or increasing amounts of anti-kinase antibody (0, 0.5, 1.0, 2.0, and 4.0 µg) are used in the immunoprecipitation step. The immunocomplex is washed three times in ice-cold lysis buffer, once in ice-cold washing buffer containing 10 mM HEPES (pH 7.4), 100 mM NaCl, 20 µg/ml aprotinin, and 0.5% NP-40, and once in ice-cold reaction buffer containing 20 mM Tris (pH 7.4), 20 mM NaCl, 1 mM DTT, 10 mM MgCl₂, and 1 mM MnCl₂. The kinase reaction is performed in the presence of 20 µM ATP and 500 ng of the peptide substrate in a total volume of 40 µl of reaction buffer at 30°C for 30 minutes with gentle agitation. The kinase reaction may be performed using increasing incubation intervals (0, 5, 10, 15, 20, 25, and 30 minutes), increasing amounts of the substrate (0, 100, 200, 400, and 500 ng), and increasing concentrations of the test compound (0, 1, 10, 100, 1000, 10000, and 100000 ng). The kinase reaction is terminated by the addition of 40 µl 2 x SDS sample buffer followed by boiling for 10 minutes. The samples are resolved by SDS-PAGE, transferred to Immobilon-P membrane (Millipore Corp., Bedford, MA, USA), and probed with a polyclonal phospho-substrate antibody. The blot is stripped and reprobed sequentially for kinase and substrate with anti-kinase antibody and anti-substrate antibody, respectively. Dectection is accomplished using the ECL-Plus chemiluminescent system (Amersham, Arlington Heights, IL, USA) and visualized using a Fuji cooled CCD camera and the Aida 2.0 software package (Raytest Inc., New Castle, DE, USA).

To illustrate this, a Src kinase inhibition assay was utilized as detailed below:

Compounds were tested for their ability to inhibit Src kinase using the scintillation proximity assay (SPA) technology as developed by Amersham. Reagents include: Streptavidin SPA beads from Amersham, 2-[N-morpholino]ethanesulfonic acid from Sigma, ATP from Boerhinger Mannheim, [³³P]ATP : from NEN (NEG 602H), the substrate - biotinylated peptide substrate 1 (PKS1) (cdc2 peptide) from Pierce which is prepared at 12.5 µM (5X solution) in kinase buffer, and the enzyme, human recombinant c-Src at 135 µg/ml (stock solution) which is diluted 1/40 in kinase buffer (3.38 µg/ml) before use. Buffers include: (a) Kinase buffer which contains MES 30 mM pH 6.8, MgCl₂ 10 mM, Orthovanadate 0.25 mM, PMSF 0.1 mM, and DTT 1mM; (b) ATP buffer which contains ATP 5 mM in MgCl₂ 50 mM buffer (stock solution). Note that before each use dilute in MES to 100 µM (5X solution) add 100 µCi/mL [³³P]ATP; and (c) PBS Stop buffer which contains ATP 0.1 mM, EDTA 40 mM, Triton 0.1 %. Streptavidin beads are suspended at 3.3 mg/ml in stop buffer and mixed by shaking. The Kinase reaction proceeds by stepwise addition to wells on the 96 well-plate of the following: (a) 10 µL kinase buffer + 10% DMSO or compound to be tested at different concentration in MES + 10 % DMSO, (b) 10 µL kinase buffer, (c) 10 µL substrate 12.5 µM, (d) 10 µL enzyme 3.38 µg/ml, and (e) 10 µL ATP 100 µM containing 0.2 µCi [³³P]ATP. Incubation for 2 hours at 30 degrees C is followed by addition of 150 µL Stop buffer containing 500 µg streptavidin beads. Incubation proceeds for 30 min at room temperature, followed by centrifugation for 5 min at 2000 rpm, and reading on a Wallac Microbeta Scintillation counter.

### Exemplary in vitro data for Src kinase inhibition assay:

Many compounds including several having the general structure: 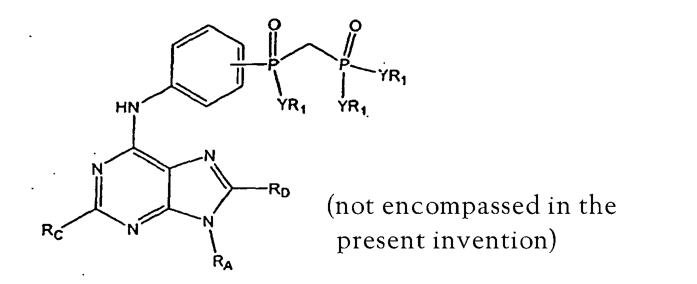 have been found to inhibit src kinase activity with an IC₅₀ in the range of about 0.001 M to about 0.1 M, preferably about 0.0007 M to about 0.1 M, about 0.007 M to about 0.005 M, and about 0.020 M to about 0.005 M.

For example, compounds in which R_{B} is a moiety having the structure: 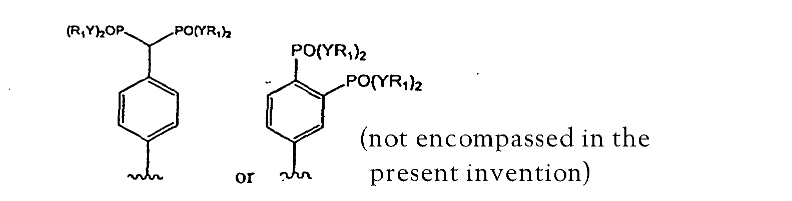 and those in which R_{C} is an amino moiety substituted with a group having the general structure: 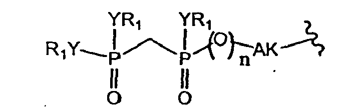 wherein Y and R₁ are as defined herein, and AK is absent or is a linear or branched aliphatic moiety, wherein n is 0 or 1, and wherein R_{A} is an linear or branched, cyclic or acyclic aliphatic moiety, or is an aryl or alkylaryl moiety optionally substituted with one or more hydroxyl moieties, and wherein R_{B} is an aryl or heteroaryl moiety optionally substituted with one or more halogen atoms, as well as those in which R_{C} is -NH- substituted with a moiety having the structure: 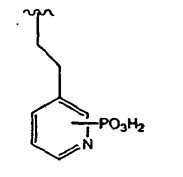 have shown activities within these ranges.

### C. Hydroxyapatite Assay:

Hydroxyapatite is the principal mineral component of bone. Hydroxyapatite adsorption chromatography is used as an assay to evaluate the bone-targeting potential of both individual bone-targeting moieties ("monomers") and of pharmaceuticals incorporating bone-targeting groups.

Method: The rentention time of a test compound is measured using a linear gradient from 10 mM sodium phosphate, 0.15 N NaCl, pH = 6.8 to 500 mM sodium phosphate, 0.15 N NaCl, pH = -6.8 on a TSK-Gel HA 1000 high pressure liquid chromatography column (7.5 mm x 75 mm). The rentention time of the compound is expressed in terms of K = (retention time-void time)/void. This K value is corrected using two reference compounds to correct from inter-column and inter-system variation to obtain a K' value.

Reference Compounds: K' values were determined for known bone targeted compounds, the bisphosphonate, alendronate and tetracycline. Alendronate gave a K' value of 3.7 and tetracycline gave a K' value of 2.0.

### D. Hypercalcemic Mouse Model for Testing In Vivo Anti Resorptive Activity

A murine hypercalcemia model for determining the efficacy of Src kinase inhibitors was developed. This model exploits the intrinsic effects of PTH (1-34) to stimulate the resorptive activity of osteoclasts *in vivo.* Briefly, compounds are each injected into mice subcutaneously, once or twice per day for five consecutive days. On the third day of test compound treatments, PTH administration begins. PTH (20 µg/kg) is given four times per day, subcutaneously, until the end of the study. Control animals receive PTH but do not receive test compounds. Blood samples are collected from the animals to obtain baseline (pre-PTH treatment), 48 hour and 72 hour (after initiation of PTH treatment) serum samples. The serum samples are analyzed for calcium concentration using the quantitative colorimetric assay reagent Arsenazo III (Sigma). Calcium serum levels for treated groups are compared to calcium serum levels of control groups and a percentage of inhibition of hypercalcemia is calculated for each time point. When a compound is effective in inhibiting the activity of osteoclasts, observed serum calcium concentrations are lower than those in animals that receive only PTH in the absence of test compound.

### Exemplary in vivo data for mouse Hyper-calcemia:

Certain compounds of this invention, when evaluated in the mouse model as described above (compounds were administered at levels of 10 mg/kg and 3 mg/kg, and serum calcium levels were checked on day 3 of the study), have shown inhibitory effects of 30-95% and 10-60% of the PTH-induced increase in serum calcium in control animals) at the higher and lower doses, respectively.

### E. Cytoxicity and Inhibition of Tumor Growth:

Certain compounds of this invention have also demonstrated cytotoxic and antitumor activity and thus may be useful in the treatment of cancer and other cell proliferative diseases. Compounds are assayed for anti-tumor activity using *in vivo* and *in vitro* assays which are well known to those skilled in the art. Generally, initial screens of compounds to identify candidates for anti-cancer drugs are performed in cellular *in vitro* assays. Compounds identified as having anti-cell proliferative activity can then be subsequently assayed in whole organisms for anti-tumor activity and toxicity. The initial screens are preferably cellular assays which can be performed rapidly and cost-effectively relative to assays that use whole organisms. For purposes of the present invention, the term "anti-proliferative compound" is used to mean compounds having the ability to impede or stop cells from progressing through the cell cycle and dividing. For purposes of the present invention, the terms "anti-tumor" and "anti-cancer" activity are used interchangeably.

Methods for determining cell proliferation are well known and can be used to identify compounds with anti-proliferative activity. In general, cell proliferation and cell viability assays are designed to provide a detectable signal when cells are metabolically active. Compounds are tested for anti-cell proliferation activity by assaying for a decrease in metabolic activity. Commonly used methods for determining cell viability depend upon, for example, membrane integrity (e.g. trypan blue exclusion) or incorporation of nucleotides during cell proliferation (e.g. BrdU or ³H-thymidine).

Preferred methods of assaying cell proliferation utilize compounds that are converted into a detectable compound during cell proliferation. Particularly preferred compounds are tetrazolium salts and include without limitation MTT (3-(4, 5-dimethylthiazol-2-yl)-2,5-diphenyltetrazolium bromide; Sigma-Aldrich, St. Louis, MO), MTS (3-(4,5-dimethylthiazol-2-yl)-5-(3-carboxymethoxyphenyl)- 2-(4-sulfophenyl)-2H-tetrazolium), XTT (2,3-bis(2-Methoxy-4-nitro-5-sulfophenyl)-2H-tetrazolium-5-carboxanilide), INT, NBT, and NTV (Bernas et al. *Biochim Biophys Acta* 1451(1):73-81, 1999). Preferred assays utilizing tetrazolium salts detect cell proliferation by detecting the product of the enzymatic conversion of the tetrazolium salts into blue formazan derivatives, which are readily detected by spectroscopic methods (Mosman. *J. Immunol. Methods.* 65:55-63, 1983).

Generally, preferred methods for assaying cell proliferation involve incubating cells in a desired growth medium with and without the compounds to be tested. Growth conditions for various prokaryotic and eukaryotic cells are well-known to those of ordinary skill in the art (Ausubel et al. Current Protocols in Molecular Biology. Wiley and Sons. 1999; Bonifacino et al. Current Protocols in Cell Biology. Wiley and Sons. 1999 both incorporated herein by reference). To detect cell proliferation, the tetrazolium salts are added to the incubated cultured cells to allow enzymatic conversion to the detectable product by active cells. Cells are processed, and the optical density of the cells is determined to measure the amount of formazan derivatives. Furthermore, commercially available kits, including reagents and protocols, are availabe for examples, from Promega Corporation (Madison, WI), Sigma-Aldrich (St. Louis, MO), and Trevigen (Gaithersburg, MD).

Any cultured cell line may be used to screen compounds for antiproliferative activity. In certain embodiments of the invention cell lines utilized include, but are not limited to, Exemplary cell lines utilized for the determination of the ability of inventive compounds to inhibit cellular proliferation include, but are not limited to COLO 205 (colon cancer), DLD-1 (colon cancer), HCT-15 (colon cancer), HT29 (colon cancer), HEP G2 (Hepatoma), K-562 (Leukemia), A549 (Lung), NCI -H249 (Lung), MCF7 (Mammary), MDA-MB-231 (Mammary), SAOS-2 (Osteosarcoma), OVCAR-3 (Ovarian), PANC-1 (Pancreas), DU-145 (Prostate), PC-3 (Prostate), ACHN (Renal), CAKI-1 (Renal), MG-63 (Sarcoma).

Preferably, the cell line is a mammalian, but is not limited to mammalian cells since lower order eukaryotic cells such as yeast may also be used to screen compounds. Preferred mammalian cell lines are derived from humans, rats, mice, rabbits, monkeys, hamsters, and guinea pigs since cells lines from these organisms are well-studied and characterized. However, the present invention does not limit the use of mammalians cells lines to only the ones listed.

Suitable mammalian cell lines are often derived from tumors. For example, the following tumor cell-types may be sources of cells for culturing cells: melanoma, myeloid leukemia, carcinomas of the lung, breast, ovaries, colon, kidney, prostate, pancreas and testes), cardiomyocytes, endothelial cells, epithelial cells, lymphocytes (T-cell and B cell), mast cells, eosinophils, vascular intimal cells, hepatocytes, leukocytes including mononuclear leukocytes, stem cells such as haemopoetic, neural, skin, lung, kidney, liver and myocyte stem cells (for use in screening for differentiation and de-differentiation factors), osteoclasts, chondrocytes and other connective tissue cells, keratinocytes, melanocytes, liver cells, kidney cells, and adipocytes. Non-limiting examples of mammalian cells lines that have been widely used by researchers include HeLa, NIH/3T3, HT1080, CHO, COS-1, 293T, WI-38, and CV-1/EBNA-1.

Other *in vitro* cellular assays may be used which rely upon a reporter gene to detect metabolically active cells. Non-limiting examples of reporter gene expression systems include green fluorescent protein (GFP), and luciferase. As an example of the use of GFP to screen for potential antitumor drugs, Sandman et al. (Chem Biol. 6:541-51; incorporated herein by reference) used HeLa cells containing an inducible variant of GFP to detect compounds that inhibited expression of the GFP, and thus inhibited cell proliferation.

Compounds identified by *in vitro* cellular assays as having anti-cell proliferation activity are then tested for anti-tumor activity in whole organisms. Preferably, the organisms are mammalian. Well-characterized mammalians systems for studying cancer include rodents such as rats and mice. Typically, a tumor of interest is transplanted into a mouse having a reduced ability to mount an immune response to the tumor to reduce the likelihood of rejection. Such mice include for example, nude mice (athymic) and SCID (severe combined immunodeficiency) mice. Other transgenic mice such as oncogene containing mice may be used in the present assays (see for example USP 4,736,866 and USP 5,175,383). For a review and discussion on the use of rodent models for antitumor drug testing see Kerbel (*Cancer Metastasis Rev.* 17:301-304, 1998-99).

In general, the tumors of interest are implanted in a test organism preferably subcutaneously. The organism containing the tumor is treated with doses of candidate anti-tumor compounds. The size of the tumor is periodically measured to determine the effects of the test compound on the tumor. Some tumor types are implanted at sites other than subcutaneous sites (e.g., at intrapertoneal sites) and survival is the measured endpoint. Parameters to be assayed with routine screening include different tumor models, various tumor and drug routes, and doses amounts and schedule. For a review of the use of mice in detecting antitumor compounds see Corbett *et al*. (Invest New Drugs. 15:207-218, 1997; incorporated herein by reference)

### Example 49: Pro-Drugs

As described previously, the compounds of the present invention may be provided as pro-drugs. To give but one example, bone targeting moieities of the following formula: 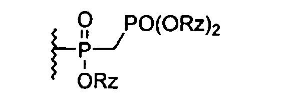 may be protected using the following R_{Z} groups:

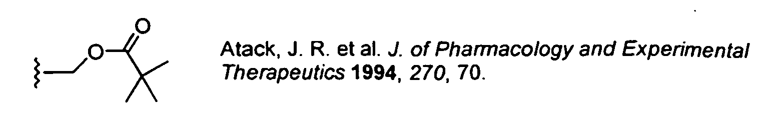

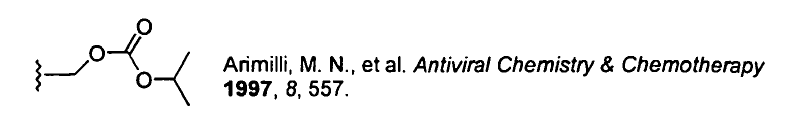

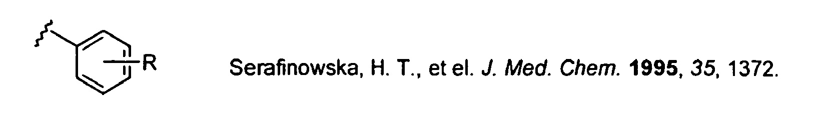

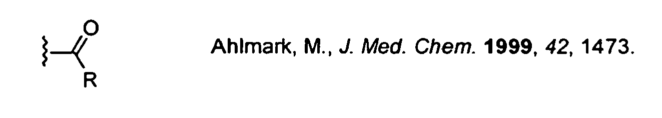

Alternatively, the bone targeting moiety may be provided as a pro-drug with the formula: 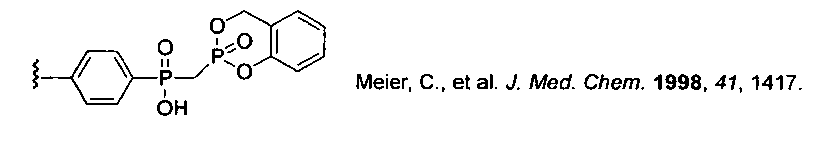 For a review of pro-drugs such as these, please see Krise, J. P., Stella, V. J. *Advanced Drug Delivery Reviews* **1996**, 19:287; incorporated herein by reference.

## Claims

1. A compound having the structure: 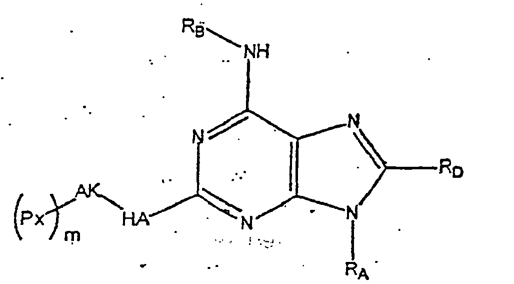 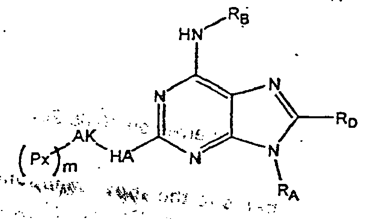 wherein R_{A} is hydrogen, an aliphatic, heteroaliphatic, aryl, heteroaryl, alkylaryl, or alkylheteroaryl moiety; R_{B} is an aliphatic, heteroaliphatic, aryl, heteroaryl, alkylaryl, or alkylheteroaryl moiety; and R_{D} is hydrogen, halogen, an aliphatic, heteroaliphatic, aryl, heteroaryl, alkylaryl, or alkylheteroaryl moiety, or ZR_{E2} wherein Z is -O-, -S-, or NR_{F}, wherein R_{E} is hydrogen, or an aliphatic, heteroaliphatic, aryl, heteroaryl, alkylaryl, or alkylheteroaryl moiety, and R_{F} is an aliphatic, heteroaliphatic, aryl, heteroaryl, alkylaryl, or alkylheteroaryl moiety, wherein in each of the foregoing groups each aliphatic, heteroaliphatic, alkylaryl, or alkylheteroaryl moiety may be branched or unbranched, cyclic or acyclic and substituted or unsubstituted, and each aryl and heteroaryl moiety may be substituted or unsubstituted;
wherein AK is a linear or branched, cyclic or acyclic, substituted or unsubstituted aliphatic or heteoraliphatic moiety; and wherein HA is absent, -O-, -S- or NH-;
wherein Pₓ is a phosphorus containing moiety having the structure -P(X)YR_{G}YR_{H}, wherein X is independently an alkyl moiety, =O or =S; R_{G} and R_{H}, for each occurrence, are independently hydrogen, or substituted or unsubstituted aliphatic, heteroaliphatic, aryl, heteroaryl, alkylaryl, or alkylheteroaryl, and each occurrence of Y is independently a covalent bond, -O-, -S- or N(R_{J})₂, wherein R_{J}, for each occurrence, is independently hydrogen, aliphatic, heteroaliphatic, aryl, heteroaryl, alkylaryl, or alkylheteroaryl;
or is a phosphorus moiety having any one of structures i-viii: 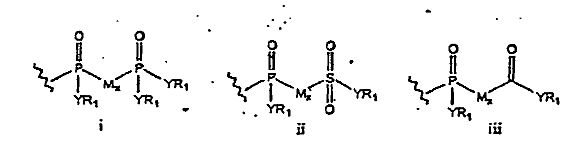 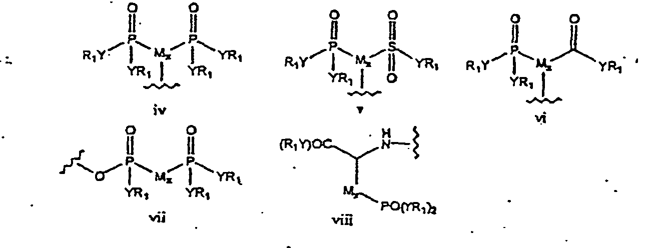 wherein each occurrence of M is independently CH₂, CHV, CHOH; or CV₂; each occurrence of Y is independently a covalent bond, -O-, -S- or N(R_{J})₂, wherein R_{J}, for each occurrence, is independently hydrogen, alipliatic, heteroaliphatic, aryl, heteroaryl, alkylaryl, or alkylheteroaryl; wherein V is a halogen; each occurrence of x is independently 1-6, and in certain embodiments is 1 or 2; and each occurrence of R₁ is independently hydrogen, aliphatic, heteroaliphatic, aryl, heteroaryl, alkylaryl, alkylheteroaryl, a prodrug or pharmaceutically acceptable derivative; and
wherein m is 1-3.

2. The compound of claim 1, wherein Px is formula i-vii, m is 1, and one or more occurrences of Y is O.

3. The compound of claim 1, wherein one or more occurrences of Y is a covalent bond.

4. The compound of claim 1, wherein HA is -O- or-S-.

5. The compound of claim 1, wherein HA is -NH-.

6. The compound of claim 1, wherein HA is absent.

7. The compound of claim 1, wherein Px is -P(X)YR_{G}YR_{H}, one or more occurrences of Y is O, X is O, R_{G} and R_{H} are each hydrogen or aliphatic, and m is 2 or 3.

8. The compound of claim 1, wherein one or more occurrences of R₁ is hydrogen.

9. The compound of claim, 1, wherein one or more of R_{G}, R_{H}, R₁ are each independently alkyl, heteroalkyl, aryl, heteroaryl, alkylaryl, or alkylheteroaryl.

10. The compound of claim, 1, wherein R_{D} is hydrogen.

11. The compound of claim, 1, wherein R_{B} is an aryl, heteroaryl, alkylaryl, or alkylheteroaryl optionally substituted with one or more halogen groups.

12. The compound of claim 11, wherein the halogen is chlorine.

13. The compound of claim 11, wherein R_{B} is phenyl.

14. The compound of Claim 1, wherein R_{A} is an aryl, heteroaryl, alkylaryl, or alkylheteroaryl moiety optionally substituted with one or more hydroxyl moieties.

15. The compound of claim 1, wherein R_{A} is a substituted or unsubstituted branched or unbranched, cyclic or acyclic aliphatic or heteroaliphatic moiety optionally substituted by one of more hydroxyl moieties.

16. A pharmaceutical composition comprising the compound of any of claims 1- 15, and a pharmaceutically acceptable carrier or excipient.

17. Use of a compound of any one of claims 1 - 15 for the preparation of a pharmaceutical composition for treating a bone-related disease.

18. Use of a compound of any one of claims 1 - 15 for the preparation of a pharmaceutical composition for treating cancer.

## Patentansprüche

1. Verbindung mit der Struktur 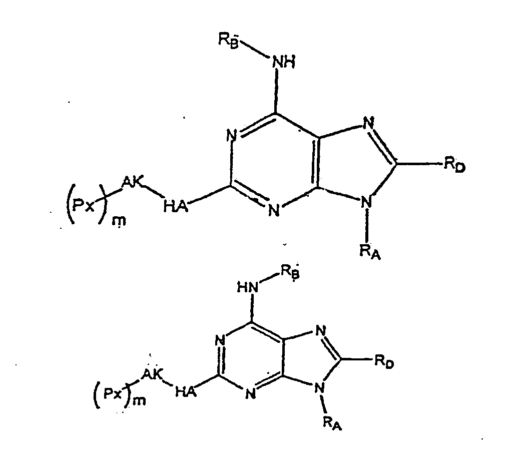 wobei R_{A} ein Wasserstoffatom, eine aliphatische Einheit, eine heteroaliphatische Einheit, eine Aryl-, Heteroaryl-, Alkylaryl- oder Alkylheteroaryleinheit darstellt; R_{B} eine aliphatische Einheit, eine heteroaliphatische Einheit, eine Aryl-, Heteroaryl-, Alkylaryl- oder Alkylheteroaryleinheit darstellt; und R_{D} ein Wasserstoffatom, ein Halogenatom, eine aliphatische Einheit, eine heteroaliphatische Einheit, eine Aryl-, Heteroaryl-, Alkylaryl- oder Alkylheteroaryleinheit oder ZR_{E2} darstellt, wobei Z gleich -O-, -S- oder NR_{F} ist, wobei R_{E} ein Wasserstoffatom oder eine aliphatische Einheit, eine heteroaliphatische Einheit, eine Aryl-, Heteroaryl-, Alkylaryl- oder Alkylheteroaryleinheit darstellt, und R_{F} eine aliphatische Einheit, eine heteroaliphatische Einheit, eine Aryl-, Heteroaryl-, Alkylaryl- oder Alkylheteroaryleinheit darstellt, wobei in jedem der vorstehenden Reste jede aliphatische, heteroaliphatische, Alkylaryl- oder Alkylheteroaryleinheit verzweigt oder unverzweigt, cyclisch oder acyclisch und substituiert oder unsubstituiert sein kann und jede Aryl- und Heteroaryleinheit substituiert oder unsubstituiert sein kann;
wobei AK eine geradkettige oder verzweigte, cyclische oder acyclische, substituierte oder unsubstituierte, aliphatische oder heteroaliphatische Einheit ist; und wobei HA nicht vorhanden, -O-, -S- oder -NH- ist;
wobei Pₓ eine Phosphor enthaltende Einheit mit der Struktur -P(X)YR_{G}YR_{H} ist, wobei X unabhängig eine Alkyleinheit, =O oder =S darstellt; R_{G} und R_{H} bei jedem Vorkommen unabhängig ein Wasserstoffatom oder einen substituierten oder unsubstituierten aliphatischen, heteroaliphatischen, Aryl-, Heteroaryl-, Alkylaryl- oder Alkylheteroarylrest darstellen, und Y bei jedem Vorkommen unabhängig eine kovalente Bindung, -O-, -S- oder N(R_{J})₂ ist, wobei R_{J} bei jedem Vorkommen unabhängig ein Wasserstoffatom, einen aliphatischen Rest, einen heteroaliphatischen Rest, einen Aryl-, Heteroaryl-, Alkylaryl- oder Alkylheteroarylrest darstellt;
oder eine Phosphoreinheit mit jeder beliebigen der Strukturen i-viii ist: 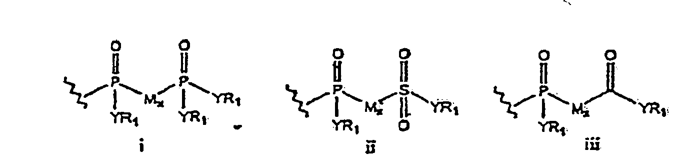 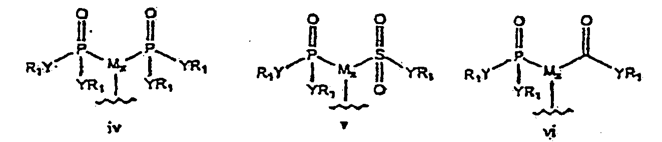 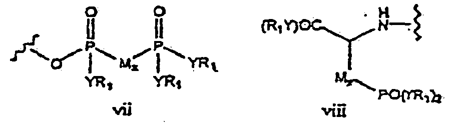 wobei M bei jedem Vorkommen unabhängig CH₂, CHV, CHOH oder CV₂ darstellt; Y bei jedem Vorkommen unabhängig eine kovalente Bindung, -O-, -S- oder N(R_{J})₂ ist, wobei R_{J} bei jedem Vorkommen unabhängig ein Wasserstoffatom, einen aliphatischen, einen heteroaliphatischen Rest, einen Aryl-, Heteroaryl-, Alkylaryl- oder Alkylheteroarylrest darstellt; wobei V ein Halogenatom ist; x bei jedem Vorkommen unabhängig 1 bis 6 bedeutet, und in bestimmen Ausführungsformen 1 oder 2 ist; und R₁ bei jedem Vorkommen unabhängig ein Wasserstoffatom, einen aliphatischen Rest, einen heteroaliphatischen Rest, einen Aryl-, Heteroaryl-, Alkylaryl-, Alkylheteroarylrest, ein Prodrug oder ein pharmazeutisch verträgliches Derivat darstellt; und
wobei m 1 bis 3 ist.

2. Verbindung gemäß Anspruch 1, wobei Px die Formel i-vii darstellt, m 1 ist und Y bei einem oder mehreren Vorkommen O ist.

3. Verbindung gemäß Anspruch 1, wobei Y bei einem oder mehreren Vorkommen eine kovalente Bindung darstellt.

4. Verbindung gemäß Anspruch 1, wobei HA -O- oder -S- darstellt.

5. Verbindung gemäß Anspruch 1, wobei HA -NH- darstellt.

6. Verbindung gemäß Anspruch 1, wobei HA nicht vorhanden ist.

7. Verbindung gemäß Anspruch 1, wobei Px -P(X)YR_{G}YR_{H} ist, Y bei einem oder mehreren Vorkommen O darstellt, X O ist, R_{G} und R_{H} jeweils ein Wasserstoffatom oder einen aliphatischen Rest darstellen und m 2 oder 3 ist.

8. Verbindung gemäß Anspruch 1, wobei R₁ bei einem oder mehreren Vorkommen ein Wasserstoffatom ist.

9. Verbindung gemäß Anspruch 1, wobei einer oder mehrere der Reste R_{G}, R_{H}, R₁ jeweils unabhängig einen Alkyl-, Heteroalkyl-, Aryl-, Heteroaryl-, Alkylaryl- oder Alkylheteroarylrest darstellen.

10. Verbindung gemäß Anspruch 1, wobei R_{D} ein Wasserstoffatom darstellt.

11. Verbindung gemäß Anspruch 1, wobei R_{B} ein gegebenenfalls mit einem oder mehreren Halogenresten substituierter Aryl-, Heteroaryl-, Alkylaryl- oder Alkylheteroarylrest ist.

12. Verbindung gemäß Anspruch 11, wobei das Halogenatom ein Chloratom ist.

13. Verbindung gemäß Anspruch 11, wobei R_{B} Phenyl ist.

14. Verbindung gemäß Anspruch 1, wobei R_{A} eine gegebenenfalls mit einer oder mehreren Hydroxyleinheiten substituierte Aryl-, Heteroaryl-, Alkylaryl- oder Alkylheteroaryleinheit ist.

15. Verbindung gemäß Anspruch 1, wobei R_{A} eine substituierte oder unsubstituierte, verzweigte oder unverzweigte, cyclische oder acylische, aliphatische oder heteroaliphatische Einheit ist, welche gegebenenfalls mit einer oder mehreren Hydroxyleinheiten substituiert ist.

16. Arzneimittel, umfassend die Verbindung gemäß einem der Ansprüche 1 bis 15 und einen pharmazeutisch verträglichen Träger oder Exzipienten.

17. Verwendung einer Verbindung gemäß einem der Ansprüche 1 bis 15 für die Herstellung eines Arzneimittels zur Behandlung von einer mit Knochen in Beziehung stehenden Erkrankung.

18. Verwendung einer Verbindung gemäß einem der Ansprüche 1 bis 15 für die Herstellung eines Arzneimittels zur Behandlung von Krebs.

## Revendications

1. Composé présentant la structure 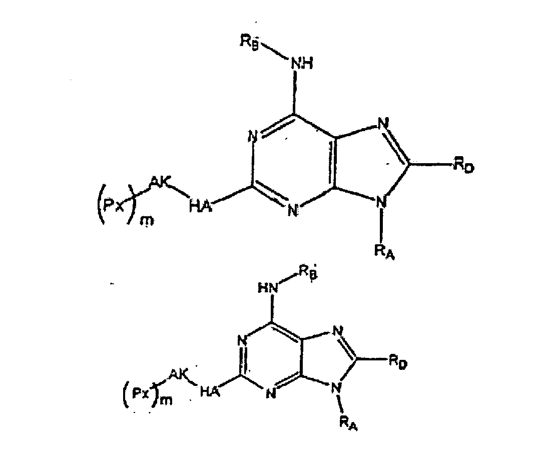 dans laquelle R_{A} est hydrogène, un groupe aliphatique, hétéroaliphatique, aryle, hétéroaryle, alkylaryle, ou alkylhétéroaryle ; R_{B} est un groupe aliphatique, hétéroaliphatique, aryle, hétéroaryle, alkylaryle, ou alkylhétéroaryle ; et R_{D} est hydrogène, halogène, un groupe aliphatique, hétéroaliphatique, aryle, hétéroaryle, alkylaryle, ou alkylhétéroaryle, ou ZR_{E2}, dans lequel Z est -O-, -S-, ou NR_{F}, dans lequel R_{E} est hydrogène, ou un groupe aliphatique, hétéroaliphatique, aryle, hétéroaryle, alkylaryle, ou alkylhétéroaryle et R_{F} est un groupe aliphatique, hétéroaliphatique, aryle, hétéroaryle, alkylaryle, ou alkylhétéroaryle, dans laquelle dans chacun des groupes précédents chaque groupe aliphatique, hétéroaliphatique, alkylaryle, ou alkylhétéroaryle peut être ramifié ou non ramifié, cyclique ou non cyclique et substitué ou non substitué, et chaque groupe aryle et hétéroaryle peut être substitué ou non substitué;
dans laquelle AK est un groupe aliphatique ou hétéroaliphatique linéaire ou ramifié, cyclique ou non cyclique, substitué ou non substitué ; et dans laquelle HA est absent, -O-, -S- ou -NH-;
dans laquelle P_{X} est un groupe contenant un phosphore ayant la structure -P(X)YR_{G}YR_{H}, dans laquelle X est indépendamment un groupe alkyle, =O ou =S ; R_{G} et R_{H}, pour chaque occurrence, sont indépendamment hydrogène ou un aliphatique, hétéroaliphatique, aryle, hétéroaryle, alkylaryle ou alkylhétéroaryle substitué ou non substitué, et chaque occurrence de Y est indépendamment une liaison covalente, -O-, -S- ou N(R_{J})₂, dans laquelle R_{J}, pour chaque occurrence, est indépendamment hydrogène, un aliphatique, hétéroaliphatique, aryle, hétéroaryle, alkylaryle, ou alkylhétéroaryle; ou est un groupe phosphore ayant l'une des structures i-viii: 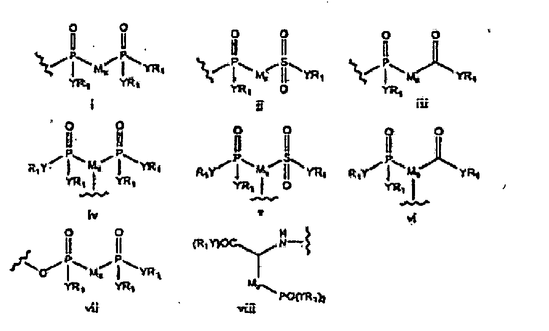 dans lesquelles chaque occurrence de M est indépendamment CH₂, CHV, CHOH, ou CV₂; chaque occurrence de Y est indépendamment une liaison covalente, -O-, -S- ou N(R_{J})₂, dans laquelle R_{J}, pour chaque occurrence, est indépendamment hydrogène, un aliphatique, hétéroaliphatique, aryle, hétéroaryle, alkylaryle, ou alkylhétéroaryle ; dans laquelle V est un halogène ; chaque occurrence de x est indépendamment 1-6, et dans certains modes de réalisation, est 1 ou 2; et chaque occurrence de R₁ est indépendamment hydrogène, un aliphatique, hétéroaliphatique, aryle, hétéroaryle, alkylaryle, ou alkylhétéroaryle, une prodrogue ou un dérivé acceptable sur le plan pharmaceutique; et
dans laquelle m est 1-3.

2. Composé selon la revendication 1, dans lequel Px est la formule i-vii, m est 1, et une ou plusieurs occurrences de Y est O.

3. Composé selon la revendication 1, dans lequel une ou plusieurs occurrences de Y est une liaison covalente.

4. Composé selon la revendication 1, dans lequel HA est -O- ou -S-.

5. Composé selon la revendication 1, dans lequel HA est -NH-.

6. Composé selon la revendication 1, dans lequel HA est absent.

7. Composé selon la revendication 1, dans lequel Px est -P(X)YR_{G}YR_{H}, une ou plusieurs occurrences de Y est O, X est O, R_{G} et R_{H} sont chacun hydrogène ou aliphatique, et m est 2 ou 3.

8. Composé selon la revendication 1, dans lequel une ou plusieurs occurrences de R₁ est hydrogène.

9. Composé selon la revendication 1, dans lequel un ou plusieurs de R_{G}, R_{H}, R_{J} sont chacun indépendamment alkyle, hétéroalkyle, aryle, hétéroaryle, alkylaryle, ou alkylhétéroaryle.

10. Composé selon la revendication 1, dans lequel R_{D} est hydrogène.

11. Composé selon la revendication 1, dans lequel R_{B} est un aryle, hétéroaryle, alkylaryle, ou alkylhétéroaryle éventuellement substitué par un ou plusieurs groupes halogènes.

12. Composé selon la revendication 11, dans lequel l'halogène est le chlore.

13. Composé selon la revendication 11, dans lequel R_{B} est phényle.

14. Composé selon la revendication 1, dans lequel R_{A} est un groupe aryle, hétéroaryle, alkylaryle, ou alkylhérétoaryle éventuellement substitué par un ou plusieurs groupes hydroxyle.

15. Composé selon la revendication 1, dans lequel R_{A} est un groupe aliphatique ou hétéroaliphatique, substitué ou non substitué, ramifié ou non ramifié, cyclique ou acyclique éventuellement substitué par un ou plusieurs groupes hydroxyle.

16. Composition pharmaceutique comprenant le composé de l'une des revendications 1-15, et un support ou excipient acceptable sur le plan pharmaceutique.

17. Utilisation d'un composé selon l'une quelconque des revendications 1-15 pour la préparation d'une composition pharmaceutique pour traiter une maladie liée à l'os.

18. Utilisation d'un composé selon l'une quelconque des revendications 1-15 pour la préparation d'une composition pharmaceutique pour traiter le cancer.
